# EUROPEAN PATENT APPLICATION

(11) **EP 2 428 231 A2**
(43) Date of publication of application: **14.03.2012**
(21) Application number: 11005294.1
(22) Date of filing: 10.12.2009
(51) Int. Cl.: A61K 51/08, C07D 213/16, A61K 49/12, A61K 49/14

(54) **Biomolecule complexes as contrast agents in Positron Emission Tomography (PET) based methods for the assessment of organ function**

(30) Priority: 10.12.2008 DK 200801767; 10.12.2008 US 121297 P
(62) Divisional of application: 09795743.5
(71) Applicant: Bergen Teknologioverforing AS, 5006 Bergen (NO)
(72) Inventor: Tenstad, Olav, 5009 Bergen (NO)
(74) Representative: Kröncke, Rolf

(57) **Abstract**

The present invention relates to complexes comprising one or more markers and one or more biomolecules for use as a contrast agent in positron emission tomography based imaging. The complexes according to the present invention preferably accumulate in a target compartment, such as the kidney. The present invention further relates to methods for generating the complexes and methods for using the complexes such as for evaluation of different aspects of kidney functionality e.g. for calculation of total and/or regional glomerular filtration rate in the kidney without the need for sampling blood or urine.

## Description

All patent and non-patent references cited in this application are hereby incorporated by reference in their entirety.

### Field of invention

The present invention relates to complexes comprising one or more marker(s) and one or more biomolecules. The complexes according to the present invention are designed to be used as contrast agents in imaging methods such as positron emission tomography (PET).

This invention also relates to compositions, such as pharmaceutical compositions, comprising one or more complexes according to the invention, a kit-of-parts comprising the complexes, as well as methods for making and using the complexes and the compositions according to the invention.

### Background

The main functions of the kidney are to regulate the amount of salt and water in the body and to remove waste products from plasma by excreting them into the urine. To accomplish this, 120 ml of plasma is normally filtered each minute in the glomeruli of the kidney.

A large number of kidney diseases, such as IgA nephritis, glomerulonephritis, chronic pyelonephritis and urinary retention, can lead to renal failure and subsequent decreased glomerular filtration rate (GFR). The decreased GFR may be associated with one or all of the following: proteinuria, inflammation, connective tissue formation and loss of functioning nephrons.

The decreased GFR leads in its turn to accumulation of waste products in the body, electrolyte and endocrine disturbances and hypertension. Hypertension will further damage the kidney and the outcome is a progressive deleterious process, which ultimately affects all organ systems and increases mortality.

If GFR falls below 60 ml/min, relative mortality is markedly increased, partly because decreased GFR is an independent risk factor for cardiovascular diseases [Manjunath et al (2003)ab]. It is estimated that as much as 5% of the world wide population or about 300 million people may be at risk, i.e. have a GFR lower than 60 ml/min. It follows that correct and timely measurements of kidney function in risk groups, e.g. patients with diabetes mellitus, hypertension, familial renal failure, nephrotoxic medication and proteinuria would represent a major socio-economic, medical and scientific gain.

The most commonly employed method to assess kidney function is a blood test for levels of creatinine. However, since creatinine levels in an individual depend not only kidney function, but also vary with age, sex, and race, creatinine levels are at best an approximation of the GFR in an individual.

Another method for measurement of GFR typically involves administering a substance which is freely filtered in the kidney to a subject and subsequently measuring the level of the substance in a recovered sample of body fluid (e.g. urine). Inulin has been used in this context as a marker of GFR. Measuring urinary clearance of inulin is laborious and time-consuming, and does not give information on individual kidney function.

One development along this principle is disclosed in US 7,048,907 (Groman). In '907, a contrast agent is administered to a subject, and samples of body fluid (e.g. urine) are subsequently recovered from the subject. The release of this contrast agent to the body fluid is measured by neutron activation of the contrast agent in the recovered sample. This method shares the disadvantages of those cited for urinary clearance on inulin in being cumbersome, invasive and time-consuming.

Urography (visualising the urinary tract using a series of X-ray images) and scintigraphy (intravenous injection of radioactive substance and subsequent imaging of emitted gamma rays) are two further methods useful for visualizing kidney function.

These methods both expose the subject to ionizing radiation. Furthermore, these methods do not allow calculation of regional glomerular filtration rate.

Positron emission tomography (PET) is a nuclear medicine imaging technique, which produces a three-dimensional image or picture of functional processes in the body. PET technology can be used to trace the biological pathway of any compound in living humans, provided it can be radiolabeled with a PET isotope.

One example of an organ in which PET imaging is used for functional imaging is the kidney. Functional PET imaging of the kidney relies at present on low molecular weight contrast agents. These agents are freely filtered in the kidneys and thus serve as markers of glomerular filtration rate. Examples of such markers are inulin and Gadolinium diethyllene triamine pentaacetic acid (Gd-DTPA). These contrast agents have a short half-life, and provide no information on the source of proteinuria [Choyke et al (2006)].

Macromolecular contrast agents for the evaluation of specific renal parenchymal diseases in functional PET imaging of the kidney are known in the art. Macromolecular contrast agents were originally developed in order to increase the half-life of the contrast agents used, as this was desirable when performing high-resolution angiography [Choyke et al (2006)].

Ultrasmall particles of iron oxide (USPIO) are not filtered in the kidney, and such particles are therefore not normally observed when assessing kidney function. However, since monocytes and macrophages take up USPIO, and these subsequently localise at sites of inflammation, such sites of inflammation in the kidney may be visualised. This method would provide information as to where the kidney is damaged, but only if this damage to the kidney was associated with inflammation. However, no information would be available as to the reduction in GFR or the sources of proteinuria not involving inflammation [Choyke et al (2006)].

Dendrimers are organic molecules that are polymerised to form nanoparticles of precise sizes. Different sized dendrimers have different properties, in particular in reference to filtration/retention in the kidney. Kobayashi et al. 2005 discloses the use of Gd-labelled dendrimer nanoparticles for the injection into haematological malignancies to perform dynamic micro-magnetic resonance lymphangiography (micro-MRL).

Gd-MS-325 is a Gadolinium chelate which is injected intravenously. Once injected it binds to circulating albumin. After some time, the Gadolinium salt dissociates from the albumin and is filtered out with the urine [Choyke et al (2006)].

The use of iodinated Aprotinin for measurement of uptake in rat renal cortex is known [See references Tenstad et al,1994ab,1996; Wang et al 1995,1996,1997ab; Treeck et al 1997, 2002; Roald et al 2000, 2004ab, Baran et al 2003ab, 2006].

Surprisingly, the present inventor has found that by combining the administration of biomolecule complexes and PET imaging methods, an improved detection of biological pathways can be obtained. Consequently, this combination can be used to determine organ function, such as for example kidney function.

### Summary of invention

The present invention relates to biomolecule complexes, to be used as contrast agents, comprising one or more biomolecules linked to one or more markers and which are designed for use as a contrast agent in imaging methods such as positron emission tomography.

The complexes according to the invention are preferably differentially accumulated in a target compartment. The contrast agents are thus useful in imaging techniques for visualising target compartments in an individual.

The methods of making the complexes make it possible to tailor complexes for use in visualising specific target compartments.

Megalin is an endocytic receptor expressed on the luminal surface of the renal proximal tubules. By labeling megalin ligands (such as aprotinin, lysozyme, chymotrypsinogen, albumin, Cystatin C, Cytochrome C, Ribonuclease and the like) with a PET isotope, the transport of filtered protein ligands from the tubular lumen into tubular cells and subsequent intracellular accumulation can be visualized by PET imaging.

If the targeting biomolecule or protein (e.g. aprotinin and lysozyme) is freely filtered in the glomeruli, the rate of accumulation of said targeting protein or biomolecule in the kidney reflects the GFR. Thus, a decline in the rate of biomolecule accumulation implies a decrease in the GFR. Freely filtered proteins are thus suitable for estimating GFR.

Use of the complexes according to the present invention allows for the calculation of total and/or regional glomerular filtration rate in the kidney without the need for sampling blood or urine.

If the targeting protein is larger (i.e. albumin), it is normally not filtered in the glomeruli and accumulation of the targeting protein in the kidney is low. However, local or regional glomeruli damage can in some instances lead to the filtration of larger proteins in the glomeruli and cause proteinuria. Proteinuria caused by local glomeruli damage can be visualized as hot spots while a more homogenous accumulation of a larger targeting protein would reflect a generalized proteinuria. Thus, larger proteins that are not normally filtered in the healthy kidney are thus suitable for evaluating proteinuria.

Also, tubular uptake of proteins is believed to depend on net molecular charge. In general, cationic proteins are usually more avidly reabsorbed than anionic proteins with net negative molecular charge.

Thus, targeting proteins with different molecular size and net molecular charge can visualize different aspects of renal functions like for example GFR, proteinuria and tubular function.

### Brief description of the drawings

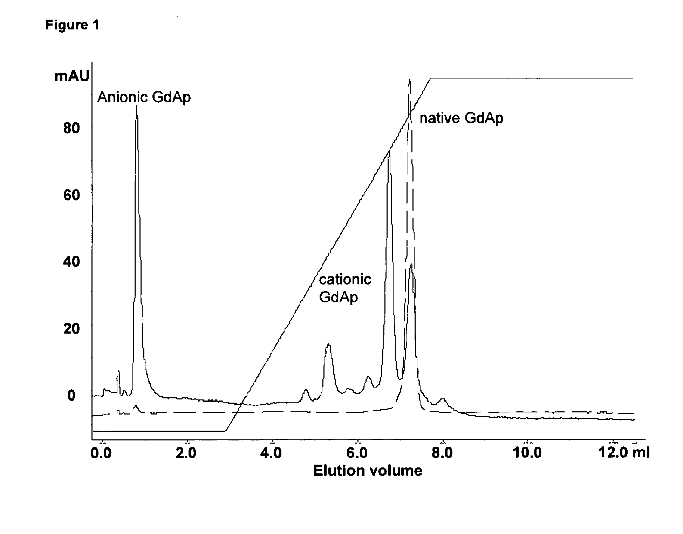

Figure 1 illustrates that Gd-DTPA-aprotinin yields several subpopulations of probes with decreasing pI corresponding to the amount of incorporated Gd-DTPA. The peak to the right shows a fraction that is left un-incorporated (co-elutes with native aprotinin) while the peak to the left represent the fraction which is most heavily incorporated with Gd-DTPA. Anionic Gd-DTPA-aprotinin is partially reabsorbed by the proximal tubular cells and is partially excreted into the urine. Cationic Gd-DTPA-aprotinin is filtered in the glomeruli and quantitatively accumulated in the proximal tubular cells in the same manner as native aprotinin. Gd-DTPA-aprotinin complexes can therefore be used as a PET imaging contrast agent for evaluation of single kidney function/glomerular filtration rate. RESOURCE® S 1 ml cation exhange chromatography. Buffer A: 10 mM Phosphate, pH 6.5, Buffer B: A+0.5M NaCl. Gradient: 0-100% B in 5 column volumes (5ml) at 1 ml/min.

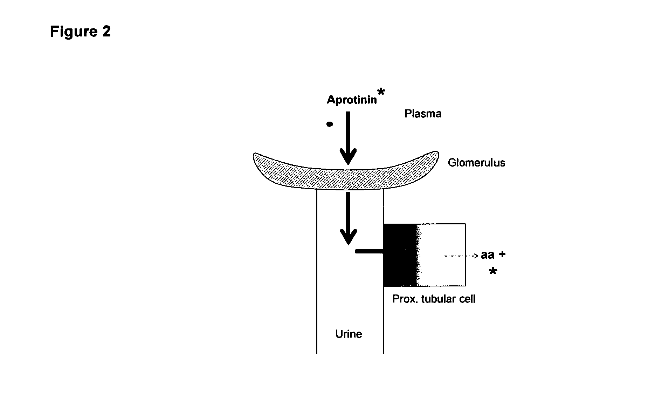

Figure 2 illustrates that aprotinin, a 6.5 kDa polypeptide, is freely filtered in the glomeruli and quantitatively taken up into proximal tubular cells, close to its parent glomerulus by adsorptive endocytosis. Filtered aprotinin is then digested in the lysosomes and breakdown products can be detected in plasma 20 minutes after i.v. injection of labelled aprotinin (aprotinin*). By recording the uptake of aprotinin* in different layers of the renal cortex, detailed and accurate information of single kidney function can be obtained.

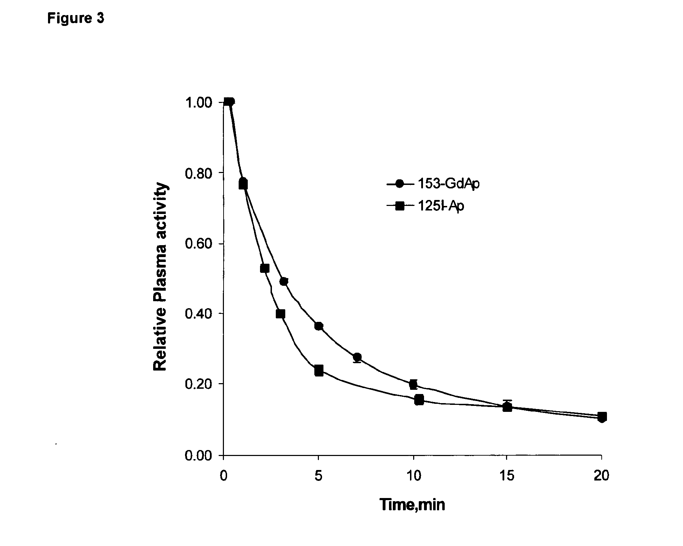

Figure 3. Left panel: Plasma concentration relative to initial activity ± 1 SEM after i.v. injection of ¹²⁵I-aprotinin and ¹⁵³Gd-aprotinin.

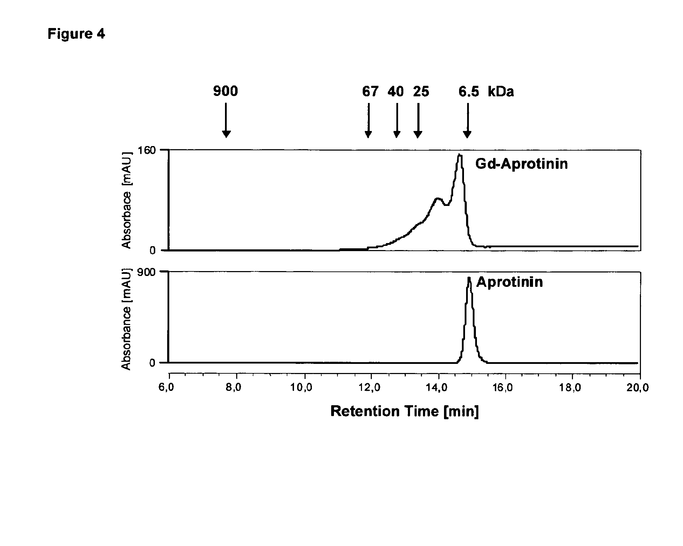

Figure 4. Size exclusion chromatogram of aprotinin (reference molecule) and Gd-aprotinin using a SW3000XL HPLC column from TosoHaas. The elution volume of molecular weight standards (IgM (900 kDa), BSA (67 kDa), ovalbumin (40 kDa), chymotrypsinogen A (25 kDa) and aprotinin (6.5 kDa)) is indicated by arrows.

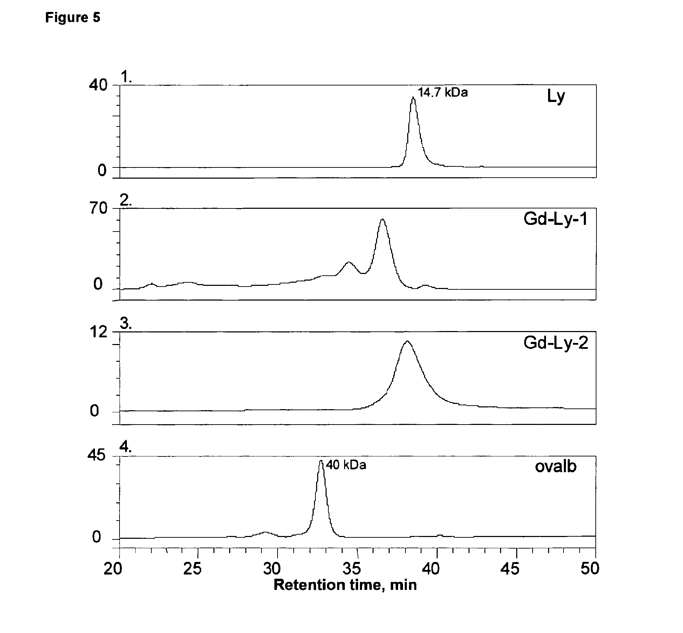

Figure 5. Size exclusion high performance chromatography (Toso Haas super SW3000, 4.6mm diam x 60cm height eluted with 0.1 mol/L phosphate buffer + 0.1mol/L Na2SO4, pH 6.4, at 0.2 ml/min) of 1) Native Lysozyme (Ly), 2) Gd-DTPA-Lysozyme using standard protocol (Gd-Ly-1), 3) Gd-DTPA-Lysozyme using ¼ amount DTPA (Gd-Ly-2) and 4) Native ovalbumin (ovalb).

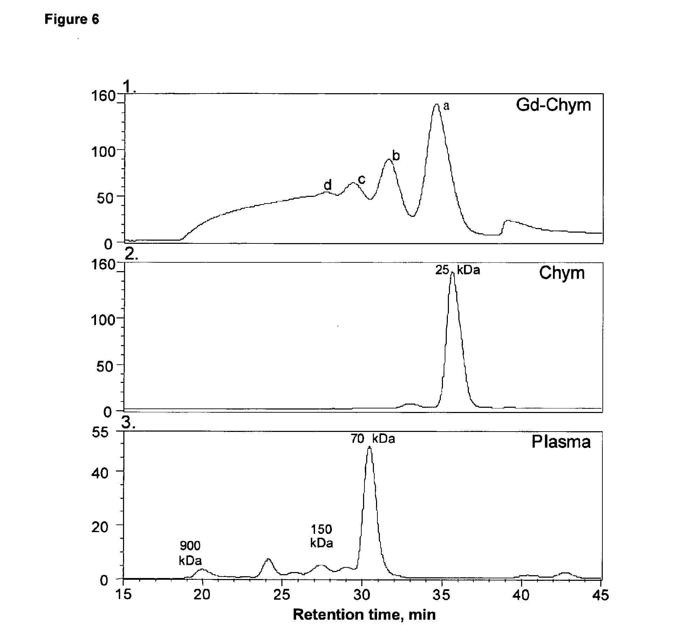

Figure 6 illustrates size exclusion high performance chromatography (Toso Haas super SW3000, 4.6mm diam x 60cm height eluted with 0.1 mol/L phosphate buffer + 0.1mol/L Na2SO4, pH 6.4, at 0.2 ml/min) of 1. Gd-DTPA-Chymotrypsinogen A (Gd-Chym) forming discrete complexes (a-d) with molecular weight ranging from 30-150 kDa and a continuum of larger complexes ranging from about 200 kDa to more than 900 kDa., 2. Native Chymotrypsinogen A (Chym) and 3. Plasma. Molecular weight of plasma albumin, plasma IgG, plasma IgM and native chymotrypsinogen A indicated.

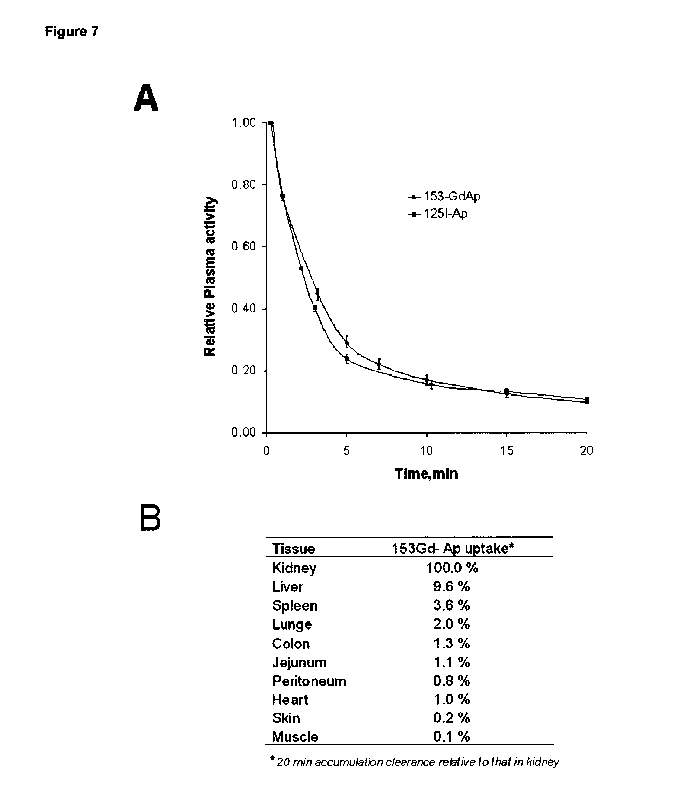

Figure 7: A: Plasma concentration relative to initial activity ± 1 SEM after i.v. injection of ¹²⁵I-aprotinin and cationic ¹⁵³Gd-DTPA-aprotinin in 3 anaesthetized rats. B: Relative accumulation of cationic ¹⁵³Gd-DTPA-aprotinin in various organs 20 minutes after i.v. injection in an anaesthetized rat.

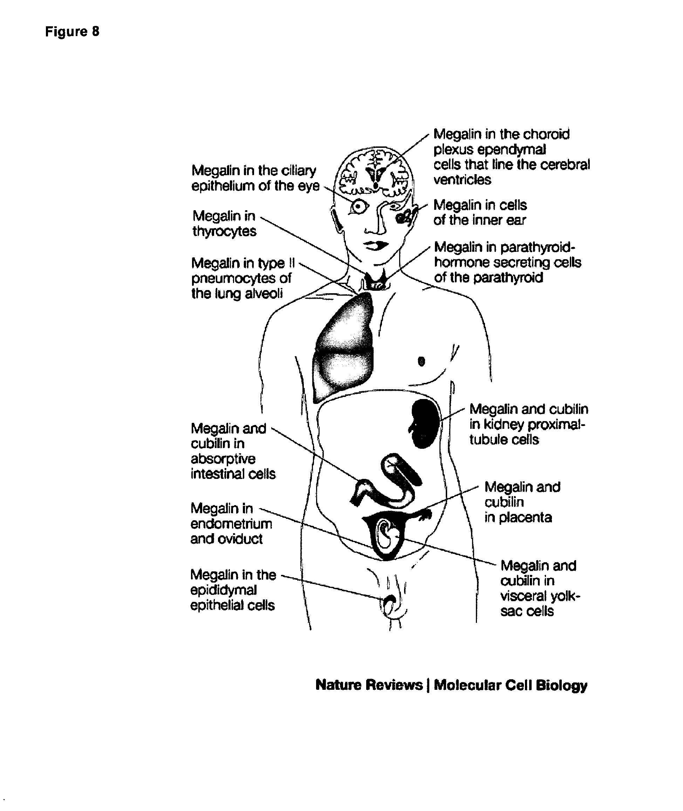

Figure 8: Organs comprising Megalin and Cubilin (Christensen & Birn 2002).

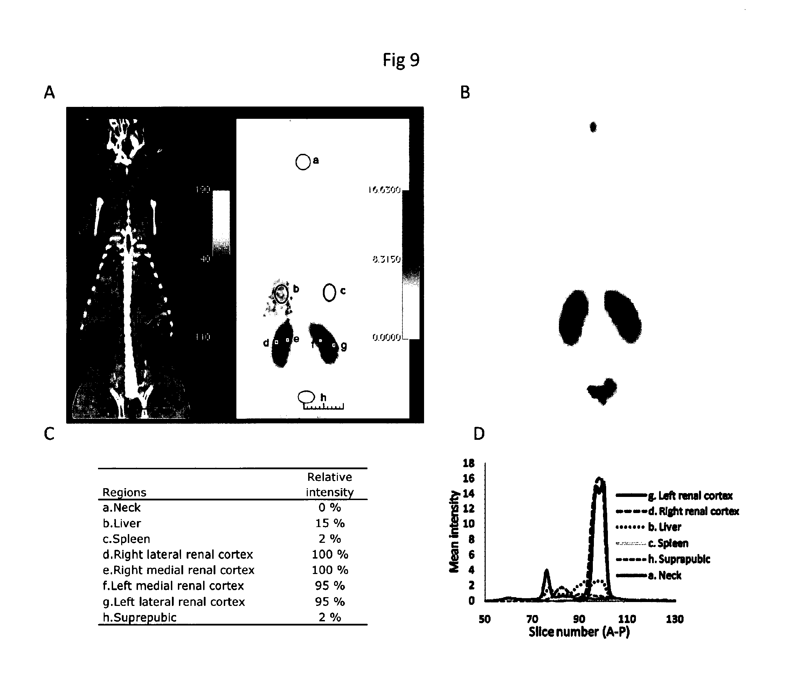

Figure 9: Panel A. Fused PET-CT slice (coronal, 6 bed, 165 cm) and PET (slice 99 of 168 anterior to posterior, 4.07 mm slice thickness) showing accumulation of 124I-Aprotinin in renal cortex 60 minutes after intravenous injection of 133 Mbq 124I-Aprotinin into a 26 kg anesthetized pig (pig 1) Ruler length = 10 cm. Panel B. 3D reconstruction of 168 PET slices showing the whole body volume. Note that the pixel intensities in the kidneys, thyroid and the bladder are saturated in order to visualize diffuse uptake throughout the liver. Panel C shows 124I-intensities in the different regions indicated in PET slice no 99 (Panel A), the average 124I-intensities through the whole stack being displayed in panel D.

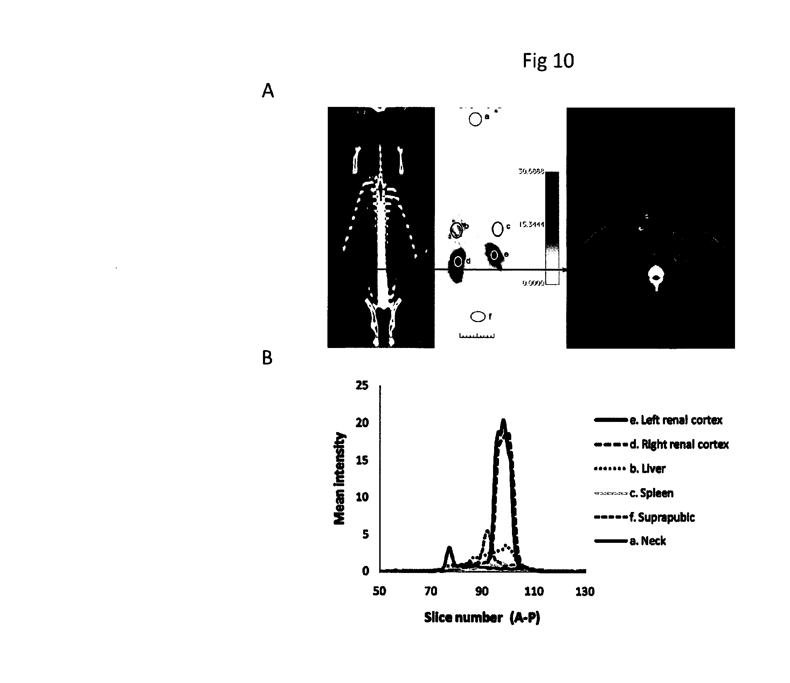

Figure 10: Panel A. Fused coronal PET-CT (left) and PET (middle) slices showing accumulation of 124I-Aprotinin in renal cortex 60 minutes after intravenous injection of 47 Mbq 124I-Aprotinin into a 27 kg anesthetized pig (pig 2). The lower polar branch of the left renal artery was ligated prior to the tracer injection resulting in cessation of glomerular filtration in the lower part of the left kidney. Ruler length = 10 cm. A transverse PET-CT slice (5 mm thick) at the level of injury is shown to the right. Panel B. Average 124I-intensity in the regions of interest indicated on the coronal PET-slice (Panel A) as a function of anterior to posterior position (A-P). Slice thickness = 4.07 mm.
Fiurge 11: Fused coronal PET-CT slice (left panel) showing accumulation of 124I-Aprotinin in renal cortex 60 minutes after intravenous injection of 30 Mbq 124I-Aprotinin and CT contrast (Omnipaque, 60ml) into a 27 kg anesthetized pig (pig 3). The lower polar branch of the left renal artery was ligated prior to the tracer injection resulting in cessation of glomerular filtration in the lower part of the left kidney. Ruler length = 10 cm. The transverse PET-CT slice (5 mm thick) at the level of the ligated artery (some artifacts radiate from the metal clip) shows intact glomerular filtration in the dorsal part of the cortex, loss of glomerular filtration in the ventral part and CT-contrast accumulating in urinary spaces. The non fused PET-slice (5 mm thick) is also shown.

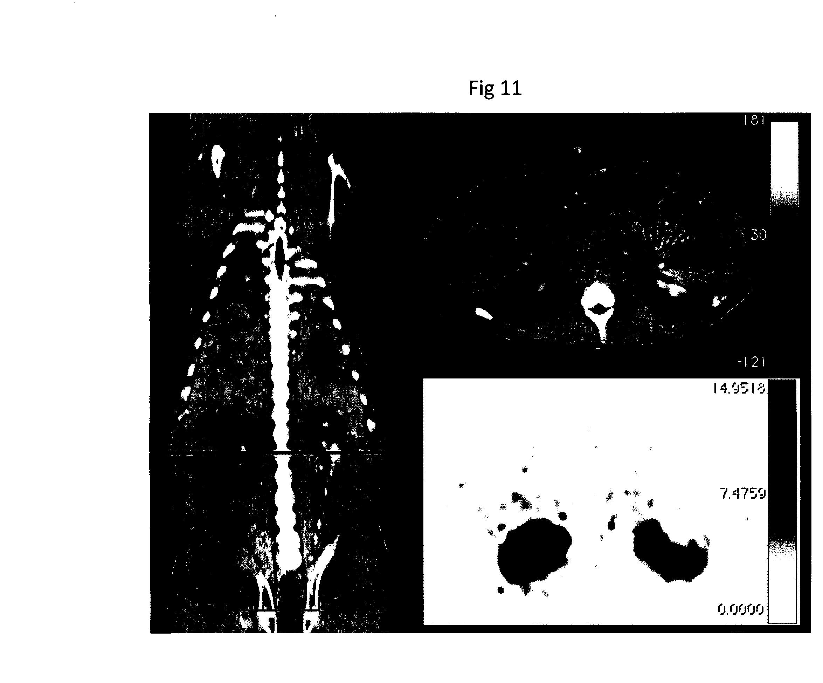

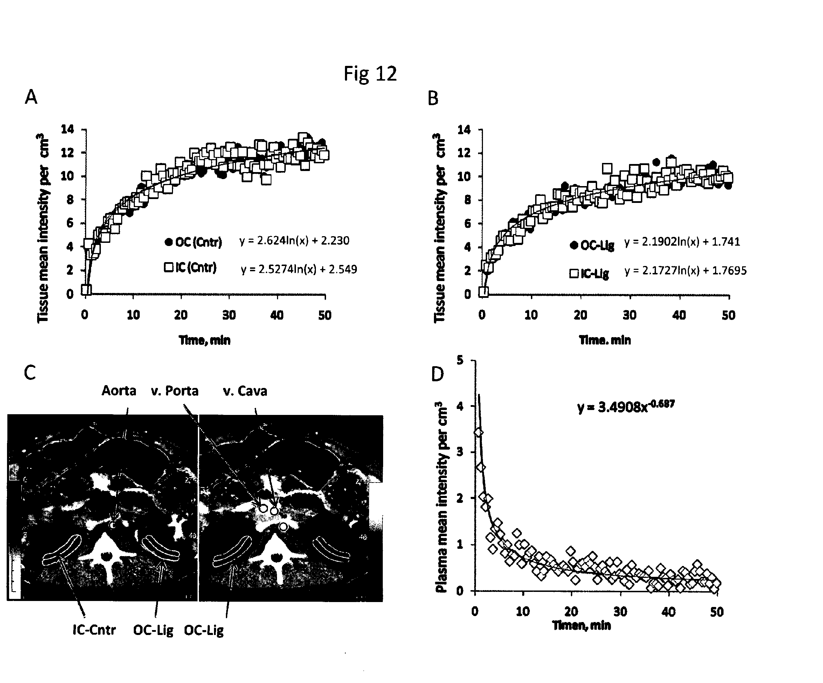

Figure 12: Data from the same pig as in Fig. 11 showing accumulation of 124I-Aprotinin in outer (OC-Cntr) and inner (IC-Cntr) renal cortex of the right normal kidney (Panel A) and in corresponding zones (OC-Lig and IC-Lig) of the left kidney following polar branch artery ligation (Panel B) at the level of injury as shown in panel C. The 124I-aprotinin activity in plasma (Panel D) was detected as the average intensity of several PET-sections through the portal vein and the caval vein (Panel C, left and right picture 0,5 and 50 minutes after tracer injection respectively). 124I-Aprotinin activity (y) showed an exponential decay in plasma and a logarithmic increase in kidney cortex with time (x).

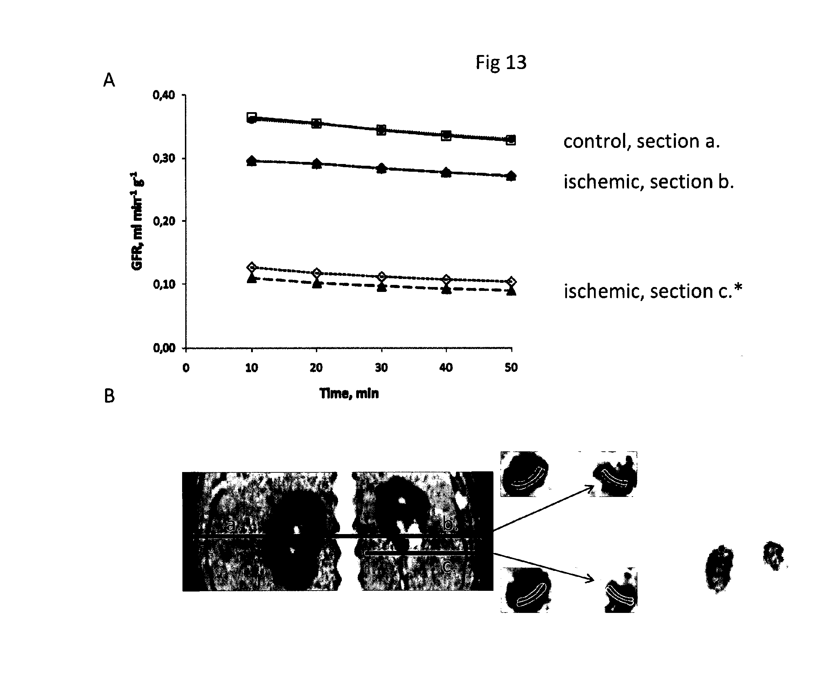

Figure 13: Data from the same animal as in Fig 11 and 12. Panel A. Glomerular filtration rate (GFR) as a function of time in outer cortex (filled circle) and inner cortex (open square) in the normal right kidney (control, transverse section a.) and in outer cortex (filled triangles) and inner cortex (open diamonds) from two different ischemic zones (b. and c.) of the ligated left kidney as shown in Panel B (coronal PET-CT to the left; transverse PET section, middle picture showing the sampling zones. Whole body PET 3D reconstruction to the right). Regional GFR was calculated as the mean tissue intensity per unit volume divided by time integrated mean plasma intensity per unit volume and expressed as ml per g cortex assuming a tissue density of 1 g/cm3 and a plasma density of 1ml/cm3. * P< 0.05 (mean glomerular filtration rate in inner cortex significantly greater than that of outer cortex in zone c.)

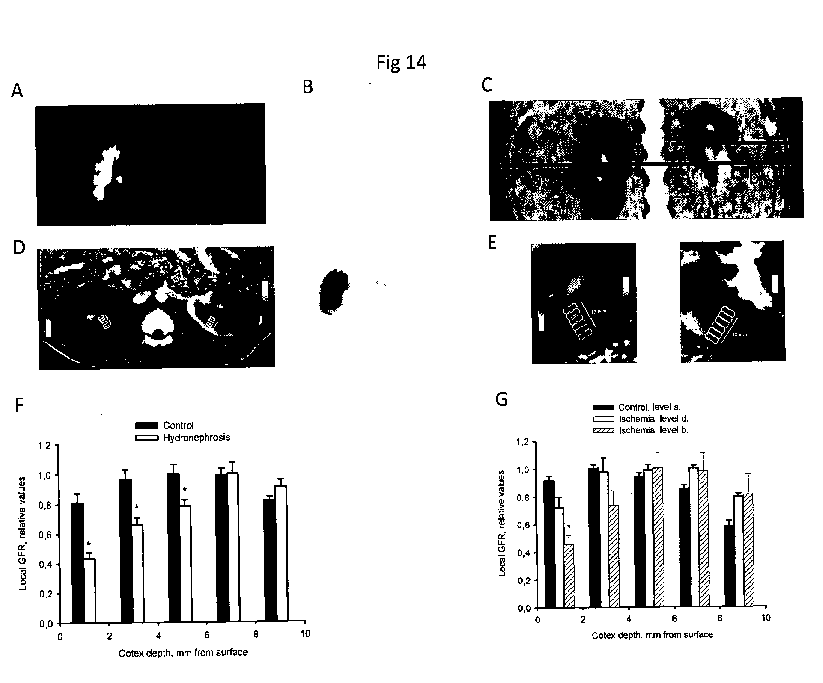

Figure 14: Coronal CT (Panel A), 3D PET-reconstruction (Panel B) and a transverse PET-CT slice (Panel D) of an anaesthetized pig (pig 4) 2 hours after ligation of the left urether. Dynamic PET-recordings (list mode) were performed from the time of injection of 18 Mbq 124I-aprotinin until the time of killing the pig 20 minutes after injection. CT-contrast was infused prior to 124I-aprotinin showing filling of urinary spaces in the right control kidney (Panel A) whereas no contrast is excreted from the left obstructed kidney. Panel F shows glomerular filtration rate (local GFR) in subpopulations of nephrons sampled from different cortex depths (Panel D), average values from three levels as indicated in Panel A (right control kidney, zone 2-4 and left hydronephrotic kidney, zone 1-3). Local GFR was calculated as average intensity of the sampling volume minus average background intensity in volumes from the erector spinae muscle group divided by time integrated plasma activity. There was a significant redistribution of local GFR to deeper cortex in the hydronephrotic left kidney (white bars) compared to the corresponding right control kidney (black bars). Panel C, E and G are from the same experiment as in Figs 11-13 showing a coronal PET-CT slice (Panel C), Sampling of local GFR (Panel E) and a significant redistribution of local GFR in the ischemic zone b (panel G).

### Definitions

Unless specifically indicated otherwise, all technical and scientific terms used herein have the same meaning as commonly understood by those of ordinary skill in the art to which this invention belongs. For purposes of the present invention, the following terms are defined.

**DTPA** is Diethylene triamine pentaacetic acid.

**USPIO** is ultra-small particles of iron oxide.

**DOTA** is 1,4,7,10-tetraazacyclododecane-1,4,7,10-tetraacetic acid. DOTA is a 12 member tetraaza macrocycle, essentially a Cyclen skeleton that has been modified with acetate side arms to form a polyamino carboxylic acid.

**NOTA** is 1, 4, 7-triazacyclononane-N, N, N-triacetic acid.

The term **"aprotinin"** as used herein refers to a single-chain polypeptide which can be obtained at least from lung tissue of bovine species. As used herein, the term relates to a polypeptide of from 50 to 70 amino acids, such as about 58 amino acids. It is an inhibitor of certain proteolytic enzymes.

The term **"lysozyme"** as used herein refers to an enzyme of from 10 to 20 kDa, such as about 14.4 kilodalton enzyme, belonging to class EC 3.2.1.17 and consisting of a single polypeptide chain of about 120 to 140 amino acids, such as about 129 amino acids. The enzyme has four disulphide bridges.

The term **"chymotrypsinogen A"** as used herein refers to a precursor of the digestive enzyme chymotrypsin, which precursor has a molecular weight of from 20,000 kDa to 30,000 kDa, such as about 25,000 kDa, and an isoelectric point of from 8.7 to 9.5, such as a pI of about 9.1.

The term **"Ovalbumin"** refers to the major constituent of egg white. The ovalbumin protein of chickens is made up of about 385 amino acids and has a relative molecular mass of about 45 kD. Ovalbumin from sources others than chickens are also envisaged.

The term **"Cystatin C"** refers to the cysteine protease inhibitor. The Cystatin C protein of chickens is made up of about 120 amino acids and has a relative molecular mass of about 13 kD. Cystatin C from sources others than chickens are also envisaged.

The term **"Cytochrome** C" refers to the heme protein. The Cytochrome C protein of chickens is made up of about 100 amino acids and has a relative molecular mass of about 12 kD. Cytochrome C from sources others than chickens are also envisaged.

The term **"Ribonuclease"** refers to any of the RNases from the group consisting of RNase A, RNase H, RNase I, RNase III, RNase L, RNase P, RNase PhyM, RNase T1, RNase T2, RNase U2, RNase V1, RNase V, PNPase, RNase PH, RNase II. Rnase R, RNase D, RNase T, Oligoribonuclease, Exoribonuclease I and Exoribonuclease. Ribonuclease from sources others than chickens are also envisaged.

The term **"biomolecule"** as used herein refers to a natural compound or a synthetic, biocompatible compound, such as a polypeptide or any other chemical compound that originally occurs in living organisms, irrespective of the means by which the biomolecule is produced, and including all variants of said chemical compound. For example, aprotinin, aprotinin fragments, a chimera of aprotinin and a second protein, and glycosylated aprotinin are all biomolecules within the meaning used herein.

The term **"linker"** as used herein refers to a residue or chemical bond separating at least two species, such as a biomolecule and a marker. The species may be retained at an essentially fixed distance, or the linker may be flexible and allow the species some freedom of movement in relation to each other. The link can be a covalent bond or a non-covalent bond.

The term **"marker"** as used herein refers to a compound capable of undergoing positron emission radioactive decay, such as a compound comprising or consisting of one or more PET isotopes.

The term **"complex"** or **"biomolecule complex"** herein is used to refer to both the form B-X-M and (B-X-M)ⁿ, where n is a positive integer. B is a biomolecule, X is an optional linker and M is a marker comprising at least one PET isotope. The word "tracer" is used interchangeably with the word complex throughout the application.

The term **"supracomplex"** is used to denote (B-X-M)ⁿ, where n is a positive integer.

The term **"contrast agent"** is used herein to refer to a compound or complex capable of improving the visibility of body structures in imaging techniques, such as PET, Magnetic resonance imaging, or radiograph based methods such as Computed tomography -based methods. The term as used herein is synonymous with "labelling agent". The complexes of the invention to be used as contrast agents can have the formula B-X-M, or be linked together to form supracomplexes (B-X-M)ⁿ, where n is a positive integer. The term contrast agent comprises both forms. The term contrast agent herein is used interchangeably with the term complex and biomolecule complex.

The term **"macromolecular contrast agents"** as used herein refers to compounds with a molecular weight of preferably more than 1 kDa and preferably less than 1500 kDa.

The term **"engineered"** is used herein to refer to molecules which are synthesised or are in any way modified in order to derivatise a natural molecule.

A **"positron"** or antielectron is the antiparticle or the antimatter counterpart of the electron. The positron has an electric charge of +1, a spin of 1/2, and the same mass as an electron. When a low-energy positron collides with a low-energy electron, annihilation occurs, resulting in the production of two or more gamma ray photons. Positrons may be generated by positron emission radioactive decay (through weak interactions), or by pair production from a sufficiently energetic photon.

**"Positron emission tomography"** (PET) is a nuclear medicine imaging technique which produces a three-dimensional image or picture of functional processes in the body. The PET scanner detects pairs of gamma rays emitted indirectly by a positron-emitting radionuclide (tracer), which is introduced into the body on a biologically active molecule. Images of tracer concentration in 3-dimensional space within the body are then reconstructed by computer analysis.

The term **"positron emission tomography (PET) imaging"** is used herein to refer to the use of PET to capture images, particularly of a living body. It is to be understood that the term includes both dynamic and still images received as a result of the technique.

The term **"magnetic resonance imaging"** abbreviated MRI is used herein to refer to the use of magnetic resonance to capture images, particularly of a living body. It is to be understood that the term includes both dynamic and still images received as a result of the technique.

The term **"computed tomography"** abbreviated CT is used herein to refer to a medical imaging method employing tomography where digital geometry processing is used to generate a three-dimensional image of the internals of an object from a large series of two-dimensional X-ray images taken around a single axis of rotation. The term as used herein is non-exclusive, and includes CT-based methods and combination methods, such as PET/CT.

The term **"target compartment"** as used herein may refer to any location in an individual. Examples of target compartments may be:
- an organ e.g. the heart,
- a tissue (e.g. the cortex of the kidney),
- a compartment (e.g. the lumen of an artery)

The term **"individual"** as used herein refers to any body, living or dead, of any species.

The term **"normal"** as used herein refers to an individual assessed to be normal by general standards.

The term **"differential accumulation"** is used herein to describe the situation where there is a difference in the amount of complex in the target compartment relative to the surroundings. E.g., an accumulation of the complex in kidney cortex, or in the case of leaky vasculature, where in a normal situation the complex would be retained, the loss of complex is indicative of an abnormal situation.

The term **"radioactive"** is used herein to describe a substance which gives off, or is capable of giving off, radiant energy in the form of particles (alpha or beta radiation) or rays (gamma radiation) by the spontaneous disintegration of the nuclei of atoms.

The term **"Radioisotope"** or **"radionuclide"** refers to an atom with an unstable nucleus, capable of undergoing radioactive decay by emission of gamma ray(s) and/or subatomic particles. These particles constitute ionizing radiation. Radionuclides may occur naturally, but can also be artificially produced. Radioisotopes may decay into different atoms or a different state of the same atom.

The term **"Glomerular filtration rate"** (GFR) describes the flow rate of filtered fluid through the kidney, more specifically the rate at which plasma including freely filterable solutes is filtered through the glomeruli of an individual.

The term **"proteinuria"** means the presence of an excess of serum proteins in the urine.

**"Megalin"** refers to a multiligand binding receptor found in the plasma membrane of many absorptive epithelial cells, for example the proximal tubuli of the kidney. Megalin can be complexed with Cubulin. Ligands for Megalin and/or the Megalin-Cubulin complex include aprotinin, chymtropsinogen A, lysozyme, ovalbumin, cystatins, ribonucleases and cytochrome C.

The term **"Ligand"** refers to an ion, a molecule, or a molecular group that interacts with another chemical entity to form a larger complex, wherein said interaction can be covalent or non-covalent.

The term **"contrast agent"** refers to a composition comprising one or more complexes suitable for use in PET based methods for the visualization of one or more target compartments.

### Detailed description of the invention

### Methods of making complexes

Complexes according to the present invention may be illustrated by the general formula B-X-M, where B is indicative of one or more biomolecules, X is an optional linker moiety, for example a chelator, and M is a marker moiety capable of undergoing positron emission radioactive decay. M may for example be a paramagnetic moiety, a radioactive label or a fluorescent label.

The complexes of the invention are synthesised by a novel and inventive method comprising the steps of:
a) Providing the biomolecule based on the desired target compartment,
b) Providing the marker moiety,
c) Linking the biomolecule to the marker moiety optionally via a linker, and
d) Selecting the complex based on one or more of the following: isoelectric point (pI), size and/or specificity for one or more ligands.

In one embodiment of the present invention the complexes are generated by the above method further comprising the steps of:
a) dissolving an amount of the biomolecule in a suitable solvent thereby obtaining a solution of the biomolecule,
b) dissolving an amount of the linker in a suitable solvent thereby obtaining a solution of the linker,
c) combining the solutions obtained in a) and b) and optionally adjusting the pH of the mixture of solutions,

### The biomolecule moiety

The biomolecule moiety of the complex may be selected in order to achieve a differential accumulation of the complex in or near the target compartment of interest. The differential accumulation may be an increased amount of complex present in or near the target compartment as compared to the normal case. Differential accumulation may also be a diminished amount of complex in or near the target organ relative to the normal case.

In one embodiment, the differential accumulation is a result of passive forces. Normally, large anionic complexes are retained in the lumen of vasculature. In the case of leaky vasculature, such complexes are leaked to the surrounding tissue. Thus, visualising the leakage of these complexes is indicative of a particular situation in that vasculature; e.g that the vessels are newly formed, or indicative of some pathology resulting in leaky vasculature.

In other embodiments, the differential accumulation is achieved by selecting a biomolecule which specifically interacts with one or more components of the target compartment, resulting either in the biomolecule being retained from said target compartment or eliminated from said target compartment.

Further embodiments include biomolecules engineered so as to confer to the complex the ability to accumulate in a particular target compartment, or to enhance an integral ability of the biomolecule to do so.

The one or more biomolecules of the invention are preferably selected from nucleic acids, polypeptides (hereunder glycosylated and otherwise post-translationally modified polypeptides), polysaccharides and/or lipids and/or mixtures thereof.

In one embodiment the complex of the present invention comprises a single biomolecule.

In other embodiments the complex of the present invention comprises two or more biomolecules, such as three, for example 4, such as 5 biomolecules which can be the same or different.

In one embodiment the biomolecule is a nucleic acid with a length of from 1 nucleic acid to 5,000 nucleic acids, such as from 1 nucleic acid to 5 nucleic acids, for example from 5 to 10 nucleic acids, such as from 10 nucleic acid to 15 nucleic acids, for example from 15 to 20 nucleic acids, such as from 20 nucleic acid to 25 nucleic acids, for example from 25 to 30 nucleic acids, such as from 30 nucleic acid to 35 nucleic acids, for example from 35 to 40 nucleic acids, such as from 40 nucleic acid to 45 nucleic acids, for example from 45 to 50 nucleic acids, such as from 50 nucleic acid to 75 nucleic acids, for example from 75 to 100 nucleic acids, such as from 100 nucleic acid to 150 nucleic acids, for example from 150 to 200 nucleic acids, such as from 200 nucleic acid to 250 nucleic acids, for example from 250 to 300 nucleic acids, such as from 300 nucleic acid to 350 nucleic acids, for example from 350 to 400 nucleic acids, such as from 400 nucleic acid to 450 nucleic acids, for example from 450 to 500 nucleic acids, such as from 500 nucleic acid to 600 nucleic acids, for example from 600 to 700 nucleic acids, such as from 700 nucleic acid to 800 nucleic acids, for example from 800 to 900 nucleic acids, such as from 900 nucleic acid to 1000 nucleic acids, for example from 1000 to 1500 nucleic acids, such as from 1500 nucleic acid to 2000 nucleic acids, for example from 2000 to 2500 nucleic acids, such as from 2500 nucleic acid to 3000 nucleic acids, for example from 3000 to 3500 nucleic acids, such as from 3500 nucleic acid to 4000 nucleic acids, for example from 4000 to 4500 nucleic acids, such as from 4500 to 5000 nucleic acids.

In one embodiment the biomolecule is not a nucleic acid.

In one embodiment the biomolecule is a polypeptide with a length of from 1 amino acid to 2000 amino acids, such as from 1 to 5 amino acids, for example from 5 to 10 amino acids, such as from 10 to 15 amino acids, for example from 15 to 20 amino acids, such as from 20 to 25 amino acids, for example from 25 to 30 amino acids, such as from 30 to 35 amino acids, for example from 35 to 40 amino acids, such as from 40 to 45 amino acids, for example from 45 to 50 amino acids, such as from 50 to 75 amino acids, for example from 75 to 100 amino acids, such as from 100 to 150 amino acids, for example from 150 to 200 amino acids, such as from 200 to 250 amino acids, for example from 250 to 300 amino acids, such as from 300 to 400 amino acids, for example from 400 to 500 amino acids, such as from 500 to 600 amino acids, for example from 600 to 700 amino acids, such as from 700 to 800 amino acids, for example from 800 to 900 amino acids, such as from 900 to 1000 amino acids, for example from 1000 to 1200 amino acids, such as from 1200 to 1400 amino acids, for example from 1400 to 1600 amino acids, such as from 1600 to 1800 amino acids, for example from 1800 to 2000 amino acids.

In one embodiment the biomolecule is not a polypeptide.

In one embodiment the biomolecule is a polysaccharide.

In one embodiment the one or more polysaccharides comprises one or more branched polymers. The present invention relates to homopolysaccharides as well as heteropolysaccharides and mixtures thereof. The polysaccharides may comprise glucose and/or mannose and/or galactose and/or fructose and/or maltose and/or sucrose and/or lactose and/or cellulose.

Polysaccharides have a general formula of Cₙ(H₂O)ₙ₋₁ where n is usually a large number between 200 and 2500. Considering that the repeating units in the polymer backbone are often six-carbon monosaccharides, the general formula can also be represented as (C₆H)₁₀O₅)ₙ where n={40...3000}.

In one embodiment the biomolecule is a polysaccharide with the formula of Cₙ(H₂O)ₙ₋₁ where n is number between 200 and 2500, such as from 200 to 300, for example from 300 to 400, such as from 400 to 500, for example from 500 to 600, such as from 600 to 700, for example from 700 to 800, such as from 800 to 900, for example from 900 to 1000, such as from 1000 to 1100, for example from 1100 to 1200, such as from 1200 to 1300, for example from 1300 to 1400, such as from 1400 to 1500, for example from 1500 to 1600, such as from 1600 to 1700, for example from 1700 to 1800, such as from 1800 to 1900, for example from 1900 to 2000, such as from 2000 to 2100, for example from 2100 to 2200, such as from 2200 to 2300, for example from 2300 to 2400, such as from 2400 to 2500.

In another embodiment the biomolecule is a polysaccharide with the formula (C₆H₁₀O₅)ₙ where n from 40 to 3000, such as from 40 to 100, for example from 100 to 200, such as from 200 to 300, for example from 300 to 400, such as from 400 to 500, for example from 500 to 600, such as from 600 to 700, for example from 700 to 800, such as from 800 to 1000, for example from 1000 to 1200, such as from 1200 to 1400, for example from 1400 to 1600, such as from 1600 to 1800, for example from 1800 to 2000, such as from 2000 to 2200, for example from 2200 to 2400, such as from 2400 to 2600, for example from 2600 to 2800, such as from 2800 to 3000.

In one embodiment the biomolecule is not a polysaccharide.

In one embodiment the biomolecule is a lipid. The lipid can in one embodiment be selected from the group consisting of any fat-soluble (lipophilic), naturally-occurring molecule, such as fats, oils, sterols, fat-soluble vitamins (such as vitamins A, D, E and K), phospholipids, monoglycerides, diglycerides, triglycerides, fatty acid, fatty acids derivates, sterol-containing metabolites such as cholesterol.

The Lipids may be broadly defined as hydrophobic or amphiphilic small molecules that originate entirely or in part from two distinct types of biochemical subunits or "building blocks": ketoacyl and isoprene groups. Using this approach, lipids may be divided into eight categories: fatty acyls, glycerolipids, glycerophospholipids, sphingolipids, saccharolipids and polyketides (derived from condensation of ketoacyl subunits); and sterol lipids and prenol lipids (derived from condensation of isoprene subunits).

Fatty acyls (including fatty acids) are a diverse group of molecules synthesized by chain-elongation of an acetyl-CoA primer with malonyl-CoA or methylmalonyl-CoA groups. The carbon chain may be saturated or unsaturated, and may be attached to functional groups containing oxygen, halogens, nitrogen and sulfur. Examples of biologically interesting fatty acyls are the eicosanoids which are in turn derived from arachidonic acid which include prostaglandins, leukotrienes, and thromboxanes. Other major lipid classes in the fatty acyl category are the fatty esters and fatty amides. Fatty esters include important biochemical intermediates such as wax esters, fatty acyl thioester coenzyme A derivatives, fatty acyl thioester ACP derivatives and fatty acyl carnitines. The fatty amides include N-acyl ethanolamines such as anandamide.

Glycerolipids are composed mainly of mono-, di- and tri-substituted glycerols, the most well-known being the fatty acid esters of glycerol (triacylglycerols), also known as triglycerides. Additional subclasses are represented by glycosylglycerols, which are characterized by the presence of one or more sugar residues attached to glycerol via a glycosidic linkage. Examples of structures in this category are the digalactosyldiacyl-glycerols and seminolipid.

Glycerophospholipids are also referred to as phospholipids. Glycerophospholipids may be subdivided into distinct classes, based on the nature of the polar headgroup at the *sn-*3 position of the glycerol backbone in eukaryotes and eubacteria or the *sn-*1 position in the case of archaebacteria. Examples of glycerophospholipids found in biological membranes are phosphatidylcholine (also known as PC or GPCho, and lecithin), phosphatidylethanolamine (PE or GPEtn) and phosphatidylserine (PS or GPSer). Some glycerophospholipids in eukaryotic cells, such as phosphatidylinositols and phosphatidic acids are either precursors of, or are themselves, membrane-derived second messengers. Typically one or both of these hydroxyl groups are acylated with long-chain fatty acids, but there are also alkyl-linked and 1Z-alkenyl-linked (plasmalogen) glycerophospholipids, as well as dialkylether variants in prokaryotes.

Sphingolipids are a complex family of compounds that share a common structural feature, a sphingoid base backbone that is synthesized *de novo* from serine and a long-chain fatty acyl CoA, then converted into ceramides, phosphosphingolipids, glycosphingolipids and other species. The major sphingoid base of mammals is commonly referred to as sphingosine. Ceramides (N-acyl-sphingoid bases) are a major subclass of sphingoid base derivatives with an amide-linked fatty acid. The fatty acids are typically saturated or mono-unsaturated with chain lengths from 14 to 26 carbon atoms. The major phosphosphingolipids of mammals are sphingomyelins (ceramide phosphocholines), whereas insects contain mainly ceramide phosphoethanolamines and fungi have phytoceramidephosphoinositols and mannose containing headgroups. The Glycosphingolipids are a diverse family of molecules composed of one or more sugar residues linked via a glycosidic bond to the sphingoid base. Examples of these are the simple and complex glycosphingolipids such as cerebrosides and gangliosides.

Sterol lipids, such as cholesterol and its derivatives are an important component of membrane lipids, along with the glycerophospholipids and sphingomyelins. The steroids, which also contain the same fused four-ring core structure, have different biological roles as hormones and signaling molecules. The C18 steroids include the estrogen family whereas the C19 steroids comprise the androgens such as testosterone and androsterone. The C21 subclass includes the progestogens as well as the glucocorticoids and mineralocorticoids. The secosteroids, comprising various forms of vitamin D, are characterized by cleavage of the B ring of the core structure. Other examples of sterols are the bile acids and their conjugates.

Prenol lipids are synthesized from the 5-carbon precursors isopentenyl diphosphate and dimethylallyl diphosphate that are produced mainly via the mevalonic acid (MVA) pathway. The simple isoprenoids (linear alcohols, diphosphates, etc.) are formed by the successive addition of C5 units, and are classified according to number of these terpene units. Structures containing greater than 40 carbons are known as polyterpenes. Carotenoids are important simple isoprenoids that function as anti-oxidants and as precursors of vitamin A. Another biologically important class of molecules is exemplified by the quinones and hydroquinones, which contain an isoprenoid tail attached to a quinonoid core of non-isoprenoid origin. Vitamin E and vitamin K, as well as the ubiquinones, are examples of this class. Bacteria synthesize polyprenols (called bactoprenols) in which the terminal isoprenoid unit attached to oxygen remains unsaturated, whereas in animal polyprenols (dolichols) the terminal isoprenoid is reduced.

Saccharolipids describe compounds in which fatty acids are linked directly to a sugar backbone, forming structures that are compatible with membrane bilayers. In the saccharolipids, a sugar substitutes for the glycerol backbone that is present in glycerolipids and glycerophospholipids. The most familiar saccharolipids are the acylated glucosamine precursors of the Lipid A component of the lipopolysaccharides in Gram-negative bacteria. Typical lipid A molecules are disaccharides of glucosamine, which are derivatized with as many as seven fatty-acyl chains. The minimal lipopolysaccharide required for growth in *E. coli* is Kdo₂-Lipid A, a hexa-acylated disaccharide of glucosamine that is glycosylated with two 3-deoxy-D-manno-octulosonic acid (Kdo) residues.

Polyketides are synthesized by polymerization of acetyl and propionyl subunits by classic enzymes as well as iterative and multimodular enzymes that share mechanistic features with the fatty acid synthases. They comprise a very large number of secondary metabolites and natural products from animal, plant, bacterial, fungal and marine sources, and have great structural diversity. Many polyketides are cyclic molecules whose backbones are often further modified by glycosylation, methylation, hydroxylation, oxidation, and/or other processes. Many commonly used anti-microbial, anti-parasitic, and anti-cancer agents are polyketides or polyketide derivatives, such as erythromycins, tetracylines, avermectins, and antitumor epothilones.

The biomolecule of the present invention can be, but is not limited to, the lipids mentioned above.

In one embodiment the biomolecule is not a lipid.

When the complex of the present invention comprises more than one biomolecule, the more than one biomolecules can in one embodiment be any combination of the biomolecules mentioned herein.

In one embodiment, the biomolecule moiety of the contrast agent is a fragment of a biomolecule. Such fragments may be generated by any means, including hydrolysis, enzyme digestion and recombinant methods.

The one or more biomolecules of the invention, or the fragments thereof, may furthermore be modified by any means. One examples of modification is post-translational modification such as glycosylation of e.g. polypeptides.

Illustrative, non-limiting examples of biomolecules according to the present invention include globular proteins of from 1 kDa to preferably less than 1500 kDa, such as polypeptides comprising or consisting of aprotinin, chymotrypsinogen A, lysozyme, ovalbumin, cystatins, ribonucleases and cytochrome C, as well as fragments and/or variants thereof.

The invention in yet another embodiment comprises complexes wherein the one or more biomolecules are genetically engineered. Said engineering can include recombinant production of the biomolecule or fragment or variant of the biomolecule.

Further embodiments include biomolecules engineered so as to confer to the complex the ability to accumulate in a particular target compartment, or to enhance an integral ability of a biomolecule to do so. An example of such engineering includes introducing a binding site for Megalin and/or Cubilin to a biomolecule. A further example of modification is to engineer the biomolecule so as to change the electric charge of the biomolecule.

In one embodiment the biomolecule moiety is selected from the group comprising proteins capable of specific interaction with the Megalin/cubulin complex. Biomolecules capable of specific interaction with the Megalin/cubulin complex include but are not limited to aprotinin, chymotrypsinogen A, lysozyme, ovalbumin, cystatins, ribonucleases and cytochrome C, as well as fragments and/or variants thereof.

The Megalin/cubulin complex is for example present in the proximal tubuli of the kidney. Complexes comprising such proteins, or fragments thereof, and which are thus retained in the kidney, are useful for use in visualising the kidney and processes in the kidney.

Megalin and Cubulin are also located in other organs/tissues besides the kidneys (see figure 8 - from Christensen & Birn 2002). The present invention also relates to complexes comprising one or more biomolecules with a natural ability to accumulate in, or alternatively engineered to, accumulate in organs/tissues with Megalin and/or Cubulin expression.

In one embodiment of the invention, the one or more biomolecules of the invention comprise or consist of an aprotinin polypeptide or a fragment and/or variant thereof.

In one embodiment the one or more biomolecules of the invention do not comprise or consist of an aprotinin polypeptide or a fragment and/or variant thereof.

In a further embodiment of the invention, the one or more biomolecules of the invention comprise or consist of a chymotrypsinogen A polypeptide or a fragment and/or variant thereof.

In one embodiment the one or more biomolecules of the invention do not comprise or consist of a chymotrypsinogen A polypeptide or a fragment and/or variant thereof.

In another embodiment of the invention, the one or more biomolecules of the invention comprise or consist of a lysozyme polypeptide or a fragment and/or variant thereof.

In one embodiment the one or more biomolecules of the invention do not comprise or consist of a lysozyme polypeptide or a fragment and/or variant thereof.

In a further embodiment of the invention, the one or more biomolecules of the invention comprise or consist of an ovalbumin polypeptide or a fragment and/or variant thereof.

In one embodiment the one or more biomolecules of the invention do not comprise or consist of an ovalbumin polypeptide or a fragment and/or variant thereof.

In a further embodiment of the invention, the one or more biomolecules of the invention comprise or consist of a Cystatin C polypeptide or a fragment and/or variant thereof.

In one embodiment the one or more biomolecules of the invention do not comprise or consist of a Cystatin C polypeptide or a fragment and/or variant thereof.

In a further embodiment of the invention, the one or more biomolecules of the invention comprise or consist of a Cytochrome C polypeptide or a fragment and/or variant thereof.

In one embodiment the one or more biomolecules of the invention do not comprise or consist of a Cytochrome C polypeptide or a fragment and/or variant thereof.

In a further embodiment of the invention, the one or more biomolecules of the invention comprise or consist of a Ribonuclease polypeptide or a fragment and/or variant thereof.

In one embodiment the one or more biomolecules of the invention do not comprise or consist of a Ribonuclease polypeptide or a fragment and/or variant thereof.

In one embodiment the biomolecule moiety of the contrast agent comprises one or more fragments of a biomolecule, such as a polypeptide, for example a globular polypeptide, such as one or more fragments of aprotinin, chymtropsinogen A, lysozyme, ovalbumin, Cystatin C, Cytochrome C or Ribonuclease.

In one embodiment the one or more biomolecules of the invention comprise or consist of Angiotensin or a fragment and/or variant thereof.

The size of the biomolecule can influence the accumulation in the target compartment. The biomolecule of the invention preferably has a molecular weight of less than 1500 kDa.

In one embodiment of the present invention the molecular weight (MW) of the biomolecule moiety of the complex is in the range of from 1 kDa to 50 kDa, for example of from 1 kDa to 45 kDa, such as from 1 kDa to 40 kDa, for example of from 1 kDa to 35 kDa, such as from 1 kDa to 30 kDa, for example of from 1 kDa to 25 kDa, such as from 1 kDa to 24 kDa, for example of from 1 kDa to 23 kDa, such as from 1 kDa to 22 kDa, for example of from 1 kDa to 21 kDa, such as from 1 kDa to 20 kDa, for example of from 1 kDa to 19 kDa, such as from 1 kDa to 18 kDa, for example of from 1 kDa to 17 kDa, such as from 1 kDa to 16 kDa, for example of from 1 kDa to 15 kDa, such as from 1 kDa to 14 kDa, for example of from 1 kDa to 13 kDa, such as from 1 kDa to 12 kDa, for example of from 1 kDa to 11 kDa, such as from 1 kDa to 10 kDa, for example of from 1 kDa to 9 kDa, such as from 1 kDa to 8 kDa, for example of from 1 kDa to 7 kDa, such as from 1 kDa to 6 kDa, for example of from 1 kDa to 5kDa, for example of from 2 kDa to 45 kDa, such as from 2 kDa to 40 kDa, for example of from 2 kDa to 35 kDa, such as from 2 kDa to 30 kDa, for example of from 2 kDa to 25 kDa, such as from 2 kDa to 24 kDa, for example of from 2 kDa to 23 kDa, such as from 2 kDa to 22 kDa, for example of from 2 kDa to 21 kDa, such as from 2 kDa to 20 kDa, for example of from 2 kDa to 19 kDa, such as from 2 kDa to 18 kDa, for example of from 2 kDa to 17 kDa, such as from 2 kDa to 16 kDa, for example of from 2 kDa to 15 kDa, such as from 2 kDa to 14 kDa, for example of from 2 kDa to 13 kDa, such as from 2 kDa to 12 kDa, for example of from 2 kDa to 11 kDa, such as from 2 kDa to 10 kDa, for example of from 2 kDa to 9 kDa, such as from 2 kDa to 8 kDa, for example of from 2 kDa to 7 kDa, such as from 2 kDa to 6 kDa, for example of from 2 kDa to 5kDa, for example of from 5 kDa to 45 kDa, such as from 5 kDa to 40 kDa, for example of from 5 kDa to 35 kDa, such as from 5 kDa to 30 kDa, for example of from 5 kDa to 25 kDa, such as from 5 kDa to 24 kDa, for example of from 5 kDa to 23 kDa, such as from 5 kDa to 22 kDa, for example of from 5 kDa to 21 kDa, such as from 5 kDa to 20 kDa, for example of from 5 kDa to 19 kDa, such as from 5 kDa to 18 kDa, for example of from 5 kDa to 17 kDa, such as from 5 kDa to 16 kDa, for example of from 5 kDa to 15 kDa, such as from 5 kDa to 14 kDa, for example of from 5 kDa to 13 kDa, such as from 5 kDa to 12 kDa, for example of from 5 kDa to 11 kDa, such as from 5 kDa to 10 kDa, for example of from 5 kDa to 9 kDa, such as from 5 kDa to 8 kDa, for example of from 5 kDa to 7 kDa, such as from 5 kDa to 6 kDa, for example of from 10 kDa to 45 kDa, such as from 10 kDa to 40 kDa, for example of from 10 kDa to 35 kDa, such as from 10 kDa to 30 kDa, for example of from 10 kDa to 25 kDa, such as from 10 kDa to 24 kDa, for example of from 10 kDa to 23 kDa, such as from 10 kDa to 22 kDa, for example of from 10 kDa to 21 kDa, such as from 10 kDa to 20 kDa, for example of from 10 kDa to 19 kDa, such as from 10 kDa to 18 kDa, for example of from 10 kDa to 17 kDa, such as from 10 kDa to 16 kDa, for example of from 10 kDa to 15 kDa, such as from 10 kDa to 14 kDa, for example of from 10 kDa to 13 kDa, such as from 10 kDa to 12 kDa, for example of from 15 kDa to 45 kDa, such as from 15 kDa to 40 kDa, for example of from 15 kDa to 35 kDa, such as from 15 kDa to 30 kDa, for example of from 15 kDa to 25 kDa, such as from 15 kDa to 24 kDa, for example of from 15 kDa to 23 kDa, such as from 15 kDa to 22 kDa, for example of from 15 kDa to 21 kDa, such as from 15 kDa to 20 kDa, for example of from 15 kDa to 19 kDa, such as from 15 kDa to 18 kDa, for example of from 15 kDa to 17 kDa.

In one embodiment the biomolecule has a molecular weight in the range of from 1 kDa to 50 kDa, such as from 1 kDa to 2 kDa, for example from 2 kDa to 3 kDa, such as from 3 kDa to 4 kDa, for example from 4 kDa to 5 kDa, such as from 5 kDa to 6 kDa, for example from 6 kDa to 7 kDa, such as from 7 kDa to 8 kDa, for example from 8 kDa to 9 kDa, such as from 9 kDa to 10 kDa, for example from 10 kDa to 11 kDa, such as from 11 kDa to 12 kDa, for example from 12 kDa to 13 kDa, such as from 13 kDa to 14 kDa, for example from 14 kDa to 15 kDa, such as from 15 kDa to 16 kDa, for example from 16 kDa to 17 kDa, such as from 17 kDa to 18 kDa, for example from 18 kDa to 19 kDa, such as from 19 kDa to 20 kDa, for example from 20 kDa to 21 kDa, such as from 21 kDa to 22 kDa, for example from 22 kDa to 23 kDa, such as from 23 kDa to 24 kDa, for example from 24 kDa to 25 kDa, such as from 25 kDa to 26 kDa, for example from 26 kDa to 27 kDa, such as from 27 kDa to 28 kDa, for example from 28 kDa to 29 kDa, such as from 29 kDa to 30 kDa, for example from 30 kDa to 31 kDa, such as from 31 kDa to 32 kDa, for example from 32 kDa to 33 kDa, such as from 33 kDa to 34 kDa, for example from 34 kDa to 35 kDa, such as from 35 kDa to 36 kDa, for example from 36 kDa to 37 kDa, such as from 37 kDa to 38 kDa, for example from 38 kDa to 39 kDa, such as from 39 kDa to 40 kDa, for example from 40 kDa to 41 kDa, such as from 41 kDa to 42 kDa, for example from 42 kDa to 43 kDa, such as from 43 kDa to 44 kDa, for example from 44 kDa to 45 kDa, such as from 45 kDa to 46 kDa, for example from 46 kDa to 47 kDa, such as from 47 kDa to 48 kDa, for example from 48 kDa to 49 kDa, such as from 49 kDa to 50 kDa.

The one or more biomolecules of the present invention is not limited by the pI. The one or more biomolecules of the invention preferably have an isoelectric point in the range of from 4.5 to 11.5, such as from 4.5 to 11, for example of from 4.5 to 10.5, such as from 4.5 to 10, for example of from 4.5 to 9.5, such as from 4.5 to 9, for example of from 4.5 to 8.5, such as from 4.5 to 8, for example of from 4.5 to 7.5, such as from 4.5 to 7, for example of from 4.5 to 6.5, such as from 4.5 to 6, for example of from 4.5 to 5.5, such as from 4.5 to 5; for example of 5 to 11.5, such as from 5 to 11, for example of from 5 to 10.5, such as from 5 to 10, for example of from 5 to 9.5, such as from 5 to 9, for example of from 5 to 8.5, such as from 5 to 8, for example of from 5 to 7.5, such as from 5 to 7, for example of from 5 to 6.5, such as from 5 to 6, for example of from 5 to 5.5; for example of 5.5 to 11.5, such as from 5.5 to 11, for example of from 5.5 to 10.5, such as from 5.5 to 10, for example of from 5.5 to 9.5, such as from 5.5 to 9, for example of from 5.5 to 8.5, such as from 5.5 to 8, for example of from 5.5 to 7.5, such as from 5.5 to 7, for example of from 5.5 to 6.5, such as from 5.5 to 6; 6 to 11.5, such as from 6 to 11, for example of from 6 to 10.5, such as from 6 to 10, for example of from 6 to 9.5, such as from 6 to 9, for example of from 6 to 8.5, such as from 6 to 8, for example of from 6 to 7.5, such as from 6 to 7, for example of from 6 to 6.5; 6.5 to 11.5, such as from 6.5 to 11, for example of from 6.5 to 10.5, such as from 6.5 to 10, for example of from 6.5 to 9.5, such as from 6.5 to 9, for example of from 6.5 to 8.5, such as from 6.5 to 8, for example of from 6.5 to 7.5, such as from 6.5 to 7; for example of 7 to 11.5, such as from 7 to 11, for example of from 7 to 10.5, such as from 7 to 10, for example of from 7 to 9.5, such as from 7 to 9, for example of from 7 to 8.5, such as from 7 to 8, for example of from 7 to 7.5; for example of 7.5 to 11.5, such as from 7.5 to 11, for example of from 7.5 to 10.5, such as from 7.5 to 10, for example of from 7.5 to 9.5, such as from 7.5 to 9, for example of from 7.5 to 8.5, such as from 7.5 to 8; 8 to 11.5, such as from 8 to 11, for example of from 8 to 10.5, such as from 8 to 10, for example of from 8 to 9.5, such as from 8 to 9, for example of from 8 to 8.5; 8.5 to 11.5, such as from 8.5 to 11, for example of from 8.5 to 10.5, such as from 8.5 to 10, for example of from 8.5 to 9.5, such as from 8.5 to 9; for example of 9 to 11.5, such as from 9 to 11, for example of from 9 to 10.5, such as from 9 to 10, for example of from 9 to 9.5; for example of 9.5 to 11.5, such as from 9.5 to 11, for example of from 9.5 to 10.5, such as from 9.5 to 10; for example of 10 to 11.5, such as from 10 to 11, for example of from 10 to 10.5; for example of 10.5 to 11.5, such as from 10.5 to 11, for example from 11 to 11.5.

In one embodiment the biomolecule of the present invention has an isolelectric point in the range of from 4.5 to 11.5, such as from 4.5 to 4.6, for example from 4.6 to 4.8, such as from 4.8 to 5.0, for example from 5.0 to 5.2, such as from 5.2 to 5.4, for example from 5.4 to 5.6, such as from 5.6 to 5.8, for example from 5.8 to 6.0, such as from 6.0 to 6.2, for example from 6.2 to 6.4, such as from 6.4 to 6.6, for example from 6.6 to 6.8, such as from 6.8 to 7.0, for example from 7.0 to 7.2, such as from 7.2 to 7.4, for example from 7.4 to 7.6, such as from 7.6 to 7.8, for example from 7.8 to 8.0, such as from 8.0 to 8.2, for example from 8.2 to 8.4, such as from 8.4 to 8.6, for example from 8.6 to 8.8, such as from 8.8 to 9.0, for example from 9.0 to 9.2, such as from 9.2 to 9.4, for example from 9.4 to 9.6, such as from 9.6 to 9.8, for example from 9.8 to 10.0, such as from 10.0 to 10.2, for example from 10.2 to 10.4, such as from 10.4 to 10.6, for example from 10.6 to 10.8, such as from 10.8 to 11.0, for example from 11.0 to 11.2, such as from 11.2 to 11.4, for example from 11.4 to 11.5.

The complexes of the present invention per se, and uses of the complexes, are further described and exemplified below.

### The marker moiety

The complex of the present invention comprises one or more markers, such as one, for example two, such as three, for example four, such as five markers, which can be the same or different.

The marker moiety of the complex according to the present invention can be any marker capable of providing a signal that can be detected by PET imaging methods, said signal being positron emission as a result of radioactive decay of a radionuclide. The marker moiety of the complex is preferably selected from the group consisting of radioactive markers which are tolerable to a living individual.

The radioactive marker according to the present invention may be, for example, radioactive Technetium or a marker labelled with radioactive Technetium, a radioactive iodinated species, or a substance comprising radioactive isotopes of Carbon.

Further examples of suitable radionuclides or PET isotopes which can be used as markers according to the present invention are presented in the below table. Included in the table is the half life (T_{½}) of the PET isotopes and how to produce the radionuclides.

| **PET isotope** | **T_{1/2}** | **Production** |
|---|---|---|
| ¹⁸F | 109min | ¹⁸O(p,n)¹⁸F |
| ⁶⁴Cu | 12,7h | ⁶⁴Ni(p,n)⁶⁴Cu |
| ⁶⁸Ga | 68min | ⁶⁸Ge/⁶⁸Ga-generator |
| ¹²⁰I | 81min | ¹²⁰Te(p,n)¹²⁰I |
| ¹²⁴I | 4,2d | ¹²⁴Te(p,n)¹²⁴I |

In one embodiment of the present invention the complex comprises or consists of a biomolecule directly labelled with one or more PET isotopes. In this particular embodiment the marker moiety of the complex is the one or more PET isotopes.

In other embodiments of the present invention the complex comprises or consists of a biomolecule in complex with one or more other molecules labelled with one or more PET isotopes. In this case, the PET isotope-labelled molecule(s) can be construed as the marker moiety of the complex.

In addition to providing a signal detectable by PET imaging in the form of positron emission, the one or more markers of the complex of the present invention may be fluorescent, paramagnetic or radioactive.

In one embodiment of the present invention, the marker is selected from the group containing paramagnetic markers.

A paramagnetic marker is any chemical compound which provides a paramagnetic signal useful in imaging methods, such as MRI. Inclusion of one or more paramagnetic markers in the complex of the present invention makes the complexes suitable for use as dual contrast agents for PET-MRI imaging. In one embodiment the present invention relates to a dual contrast agent for use as a contrast agent in PET-MRI.

The paramagnetic markers of the present invention may for example be one or more of the paramagnetic isotopes of Aluminium, Barium, Calcium, Cesium, Dysprosium, Gadolinium, Iron oxide, Lithium, Magnesium, Manganese, Oxygen, Platinum, Sodium, Strontium, Tungsten, Technetium and Uranium or any variant thereof and/or mixtures thereof.

When using gadolinium, DOTA is a preferred chelating agent over DTPA and EDTA, and gadolinium is preferably used in low doses.

In one embodiment, the marker is not a paramagnetic molecule.

In one embodiment the marker is not gadolinium.

In one embodiment the marker molecule is a fluorescent marker molecule. The fluorescent marker molecule can be selected from the group consisting of 5-(and 6)-carboxyfluorescein, 5- or 6-carboxyfluorescein, 6-(fluorescein)-5-(and 6)-carboxamido hexanoic acid, fluorescein isothiocyanate (FITC), rhodamine, tetramethylrhodamine, optionally substituted coumarin including AMCA, PerCP, phycobiliproteins including R-phycoerythrin (RPE) and allophycoerythrin (APC), Texas Red, Princeston Red, Green fluorescent protein (GFP) and analogues thereof, and conjugates of R-phycoerythrin or allophycoerythrin or Texas Red, and inorganic fluorescent labels based on semiconductor nanocrystals (like quantum dot and Qdot™ nanocrystals), and time-resolved fluorescent labels based on lanthanides like Eu3+ and Sm3+, Fluor dyes, Pacific Blue™, Pacific Orange™, Cascade Yellow™, AlexaFluor®(AF), AF405, AF488,AF500, AF514, AF532, AF546, AF555, AF568, AF594, AF610, AF633, AF635, AF647, AF680, AF700, AF710, AF750, AF800, Quantum Dot based dyes, QDot® Nanocrystals (Invitrogen, MolecularProbs), Qdot®525, Qdot®565, Qdot®585, Qdot®605, Qdot®655, Qdot®705, Qdot®800, DyLight™ Dyes (Pierce) (DL), DL549, DL649, DL680, DL800, Fluorescein (Flu) or any derivate thereof, Cy-Dyes, Cy2, Cy3, Cy3.5, Cy5, Cy5.5, Cy7, Fluorescent Proteins, RPE, PerCp, APC, Green fluorescent proteins; GFP and GFP derivated mutant proteins; BFP,CFP, YFP, DsRed, T1, Dimer2, mRFP1,MBanana, mOrange, dTomato, tdTomato, mTangerine, mStrawberry, mCherry, Tandem dyes, RPE-Cy5, RPE-Cy5.5, RPE-Cy7, RPE-AlexaFluor® tandem conjugates; RPE-Alexa610, RPE-TxRed, APC-Aleca600, APC-Alexa610, APC-Alexa750, APC-Cy5, APC-Cy5.5, Ionophors; ion chelating fluorescent props

The tables below list further examples of fluorescent marker molecules:

| Fluorophor / Fluorochrome | Excitation | Emission |
|---|---|---|
| | nm | nm |
| 2-(4'-maleimidylanilino)naphthalene-6- sulfonic acid, sodium salt | 322 | 417 |
| 5-((((2-iodoacetyl)amino)ethyl)amino) naphthalene-1-sulfonic acid | 336 | 490 |
| Pyrene-1-butanoic acid | 340 | 376 |
| AlexaFluor 350 (7-amino-6-sulfonic acid-4-methyl coumarin-3-acetic acid) | 346 | 442 |
| AMCA (7-amino-4-methyl coumarin-3-acetic acid) | 353 | 442 |
| 7-hydroxy-4-methyl coumarin-3-acetic acid | 360 | 455 |
| Marina Blue (6,8-difluoro-7-hydroxy-4-methyl coumarin-3-acetic acid) | 362 | 459 |
| 7-dimethylamino-coumarin-4-acetic acid | 370 | 459 |
| Fluorescamin-N-butyl amine adduct | 380 | 464 |
| 7-hydroxy-coumarine-3-carboxylic acid | 386 | 448 |
| CascadeBlue (pyrene-trisulphonic acid acetyl azide) | 396 | 410 |
| Pacific Blue (6,8 difluoro-7-hydroxy coumarin-3-carboxylic acid) | 416 | 451 |
| 7-diethylamino-coumarin-3-carboxylic acid | 420 | 468 |
| N-(((4-azidobenzoyl)amino)ethyl)- 4-amino-3,6-disulfo-1,8-naphthalimide, dipotassium salt | 426 | 534 |
| Alexa Fluor 430 | 434 | 539 |
| 3-perylenedodecanoic acid | 440 | 448 |
| 8-hydroxypyrene-1,3,6-trisulfonic acid, trisodium salt | 454 | 511 |
| 12-(N-(7-nitrobenz-2-oxa-1,3- diazol-4-yl)amino)dodecanoic acid | 467 | 536 |
| N,N'-dimethyl-N- (iodoacetyl)-N'-(7-nitrobenz-2- oxa-1,3-diazol-4-yl)ethylenediamine | 478 | 541 |
| Oregon Green 488 (difluoro carboxy fluorescein) | 488 | 518 |
| 5-iodoacetamidofluorescein | 492 | 515 |
| propidium iodide-DNA adduct | 493 | 636 |
| Carboxy fluorescein | 495 | 519 |

| Fluorochrome family | Example fluorochrome |
|---|---|
| AlexaFluor®(AF) | AF®350, AF405, AF430, AF488,AF500, AF514, AF532, AF546, AF555, AF568, AF594, AF610, AF633, AF635, AF647, AF680, AF700, AF710, AF750, AF800 |
| Quantum Dot (Qdot®) based dyes | Qdot®525, Qdot®565, Qdot®585, Qdot®605, Qdot®655, Qdot®705, Qdot®800 |
| DyLight™ Dyes (DL) | DL549, DL649, DL680, DL800 |
| Small fluorescing dyes | FITC, Pacific Blue™, Pacific Orange™, Cascade Yellow™, Marina blue™, DSred, DSred-2, 7-AAD, TO- |
| | Pro-3, |
| Cy-Dyes | Cy2, Cy3, Cy3.5, Cy5, Cy5.5, Cy7 |
| Phycobili Proteins: | R-Phycoerythrin (RPE), PerCP, Allophycocyanin (APC), B-Phycoerythrin, C-Phycocyanin |
| Fluorescent Proteins | (E)GFP and GFP ((enhanced) green fluorescent protein) derived mutant proteins; BFP, CFP, YFP, DsRed, T1, Dimer2, mRFP1,MBanana, mOrange, dTomato, tdTomato, mTangerine, mStrawberry, mCherry |
| Tandem dyes with RPE | RPE-Cy5, RPE-Cy5.5, RPE-Cy7, RPE-AlexaFluor® tandem conjugates; RPE-Alexa610, RPE-TxRed |
| Tandem dyes with APC | APC-Aleca600, APC-Alexa610, APC-Alexa750, APC-Cy5, APC-Cy5.5 |
| Calcium dyes | Indo-1-Ca2+ Indo-2-Ca2+ |

In one embodiment of the present invention the marker is not a fluorescent molecule.

In one embodiment the marker molecule is a small molecule with a molecular weight less than 1000 Da.

In one embodiment the marker molecule has a molecular weight in the range of from 1 kDa to 50 kDa, such as from 1 kDa to 2 kDa, for example from 2 kDa to 3 kDa, such as from 3 kDa to 4 kDa, for example from 4 kDa to 5 kDa, such as from 5 kDa to 6 kDa, for example from 6 kDa to 7 kDa, such as from 7 kDa to 8 kDa, for example from 8 kDa to 9 kDa, such as from 9 kDa to 10 kDa, for example from 10 kDa to 11 kDa, such as from 11 kDa to 12 kDa, for example from 12 kDa to 13 kDa, such as from 13 kDa to 14 kDa, for example from 14 kDa to 15 kDa, such as from 15 kDa to 16 kDa, for example from 16 kDa to 17 kDa, such as from 17 kDa to 18 kDa, for example from 18 kDa to 19 kDa, such as from 19 kDa to 20 kDa, for example from 20 kDa to 21 kDa, such as from 21 kDa to 22 kDa, for example from 22 kDa to 23 kDa, such as from 23 kDa to 24 kDa, for example from 24 kDa to 25 kDa, such as from 25 kDa to 26 kDa, for example from 26 kDa to 27 kDa, such as from 27 kDa to 28 kDa, for example from 28 kDa to 29 kDa, such as from 29 kDa to 30 kDa, for example from 30 kDa to 31 kDa, such as from 31 kDa to 32 kDa, for example from 32 kDa to 33 kDa, such as from 33 kDa to 34 kDa, for example from 34 kDa to 35 kDa, such as from 35 kDa to 36 kDa, for example from 36 kDa to 37 kDa, such as from 37 kDa to 38 kDa, for example from 38 kDa to 39 kDa, such as from 39 kDa to 40 kDa, for example from 40 kDa to 41 kDa, such as from 41 kDa to 42 kDa, for example from 42 kDa to 43 kDa, such as from 43 kDa to 44 kDa, for example from 44 kDa to 45 kDa, such as from 45 kDa to 46 kDa, for example from 46 kDa to 47 kDa, such as from 47 kDa to 48 kDa, for example from 48 kDa to 49 kDa, such as from 49 kDa to 50 kDa.

In one embodiment the marker molecule has an isolelectric point in the range of from 4.5 to 11.5, such as from 4.5 to 4.6, for example from 4.6 to 4.8, such as from 4.8 to 5.0, for example from 5.0 to 5.2, such as from 5.2 to 5.4, for example from 5.4 to 5.6, such as from 5.6 to 5.8, for example from 5.8 to 6.0, such as from 6.0 to 6.2, for example from 6.2 to 6.4, such as from 6.4 to 6.6, for example from 6.6 to 6.8, such as from 6.8 to 7.0, for example from 7.0 to 7.2, such as from 7.2 to 7.4, for example from 7.4 to 7.6, such as from 7.6 to 7.8, for example from 7.8 to 8.0, such as from 8.0 to 8.2, for example from 8.2 to 8.4, such as from 8.4 to 8.6, for example from 8.6 to 8.8, such as from 8.8 to 9.0, for example from 9.0 to 9.2, such as from 9.2 to 9.4, for example from 9.4 to 9.6, such as from 9.6 to 9.8, for example from 9.8 to 10.0, such as from 10.0 to 10.2, for example from 10.2 to 10.4, such as from 10.4 to 10.6, for example from 10.6 to 10.8, such as from 10.8 to 11.0, for example from 11.0 to 11.2, such as from 11.2 to 11.4, for example from 11.4 to 11.5.

### Linkage

According to the present invention the biomolecule is attached to the marker moiety, optionally via a linker.

The association or linkage between the one or more biomolecules and the one or more markers of the complex of the present invention may be direct or indirect. The nature of the indirect or direct linkage may be of a single nature or comprise several species of binding, e.g. comprise both covalent binding and non-covalent binding, wherein said non-covalent binding could be ionic interactions.

In one embodiment, the biomolecule is attached directly to the marker, i.e. not via a linker.

In another embodiment the biomolecule is attached indirectly to the marker, i.e. via a linker.

In one embodiment comprising indirect linkage, the biomolecule is linked to a metal ion chelator, and the marker is chelated by said chelator.

Examples of chelators are: diethylenetriaminepentaacetic acid (DTPA), diethylenetriamine-pentamethylenephosphonic acid (DTPMP), tetraazacyclododecanetetraacetic acid (DOTA) or a derivative of DOTA, ethylenediaminetetraacetic acid (EDTA), tetraazacyclododecanetetrakis (methylene phosphonic acid) (DOTP), hydroxypropyl tetraazacylododecanetriacetic acid (HP-DO3A), diethylenetriaminetriacetic acid bismethylamide (DTPA-BMA) and MS-325.

Depending on the conditions of the coupling reaction, the single chelator can link together two or more biomolecule complexes (B-X-M) to form supracomplexes, which are designated here without prejudice as (B-X-M)ⁿ, where n= a positive integer. The term contrast agent comprises both sets of complexes. In another embodiment the biomolecule complex can comprise Bⁿ-Xⁿ-Mⁿ, wherein n is a positive integer such as 1, 2, 3, 4, 5, 6, 7, 8, 9, 10 etc.

In one embodiment of the present invention, one or more markers associated with one or more biomolecules form a complex comprising a linker moiety associating or binding the one or more markers and the one or more biomolecule(s).

The conditions of the coupling reaction can be manipulated so as to yield different subpopulations of complexes, where the size and charge (pI) will vary between the subpopulations.

The present invention also encompasses complexes comprising more than one different biomolecules and markers. These may be synthesized simultaneously in the same reaction mix, or synthesised separately and mixed subsequently at any stage.

The invention may for example comprise a chelator and a chelated globular protein. For example, the chelator may be DTPA, and the globular protein may be one of the group comprising aprotinin, lysozyme, chymotrypsinogen, ovalbumin, Cystatin C, Cytochrome C and Ribonuclease.

The chemical reaction between aprotinin and Gd-DTPA serves as one illustrative example. Suitable reaction conditions yield several subpopulations of Gd-DTPA-Aprotinin complexes with decreasing pI corresponding to the amount of incorporated Gd-DTPA. In this example, a very small fraction to the right remains unincorporated and co-elutes with native aprotinin. Anionic complexes of gdAp are also produced in this reaction and are seen in the flow-through prior to the salt gradient. These are the most heavily incorporated with Gd-DTPA of the subpopulations.

Appropriate molecules, capable of providing non covalent interactions between the marker molecule and the biomolecule, involve the following molecule pairs and molecules: streptavidin/biotin, avidin/biotin, antibody/antigen, DNA/DNA, DNA/PNA, DNA/RNA, PNA/PNA, LNA/DNA, leucine zipper e.g. Fos/Jun, IgG dimeric protein, IgM multivalent protein, acid/base coiled-coil helices, chelate/metal ion-bound chelate, streptavidin (SA) and avidin and derivatives thereof, biotin, immunoglobulins, antibodies (monoclonal, polyclonal, and recombinant), antibody fragments and derivatives thereof, leucine zipper domain of AP-1 (jun and fos), hexa-his (metal chelate moiety), hexa-hat GST (glutathione S-transferase) glutathione affinity, Calmodulin-binding peptide (CBP), Strep-tag, Cellulose Binding Domain, Maltose Binding Protein, S-Peptide Tag, Chitin Binding Tag, Immuno-reactive Epitopes, Epitope Tags, E2Tag, HA Epitope Tag, Myc Epitope, FLAG Epitope, AU1 and AU5 Epitopes, Glu-Glu Epitope, KT3 Epitope, IRS Epitope, Btag Epitope, Protein Kinase-C Epitope, VSV Epitope, lectins that mediate binding to a diversity of compounds, including carbohydrates, lipids and proteins, e.g. Con A (*Canavalia ensiformis*) or WGA (wheat germ agglutinin) and tetranectin or Protein A or G (antibody affinity). Combinations of such binding entities are also comprised.

### Labelling of proteins with positron emitting radionuclides

In one embodiment of the present invention, a biomolecule in the form of a protein or polypeptide is labelled with a PET isotope according to any methods known in the art.

In one embodiment of the present invention, a biomolecule in the form of a protein or polypeptide is labelled with a PET isotope according to any of the methods described below.

One of the most applied agents for protein labelling with ¹⁸F is the N-succimidyl 4-[¹⁸F]fluorobenzoate ([¹⁸F]SFB) synthon [Wester et al. 1996]. This is a time-consuming 3-step reaction:

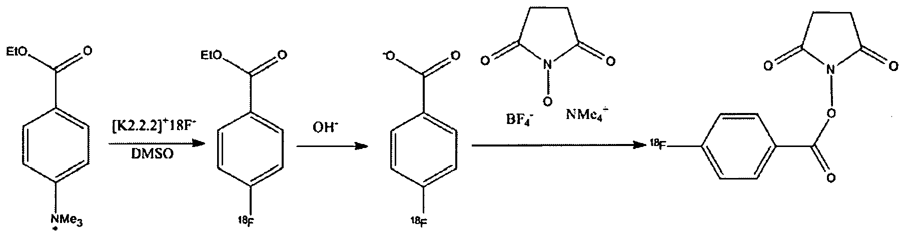

Lately a method applying hydrazinonicotinic acid (HYNIC) coupled to the N-terminus of proteins [Chang et al. 2005]. Fluoride, as [¹⁸F]-fluorobenzoic acid ([¹⁸F]FBA), was then conjugated to HYNIC via hydrazone formation:

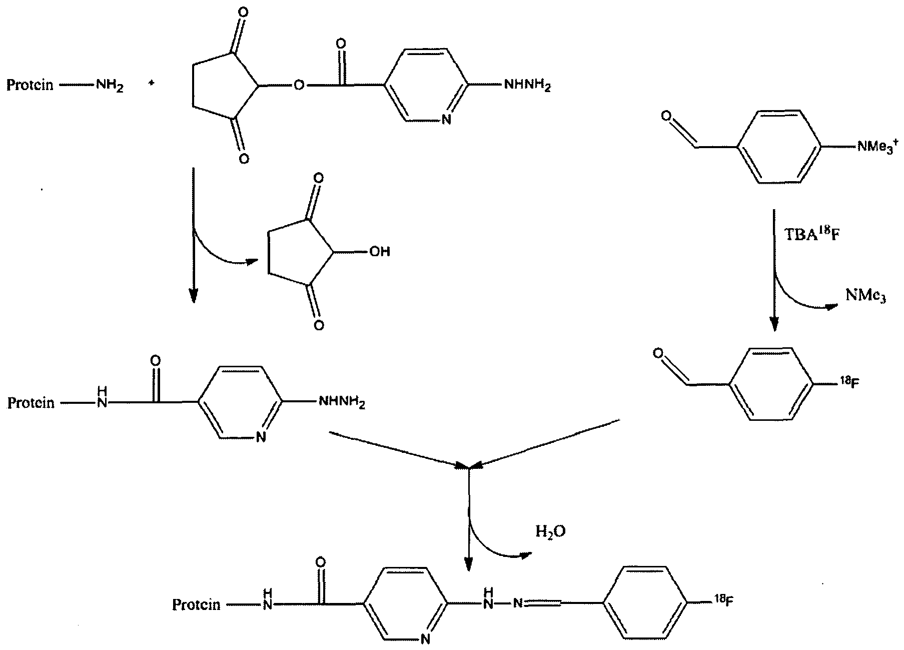

To avoid labelling at the N-terminus, or at amine sites, alternative approaches involving PEG-linkers [Gill et al. 2009, Mindt et al. 2009] attached to thiol (cysteine) and fluorinated through 1,3-cycloaddition reactions, aka. "click chemistry", exist. These are predominantly prosthetic groups containing maleimide:

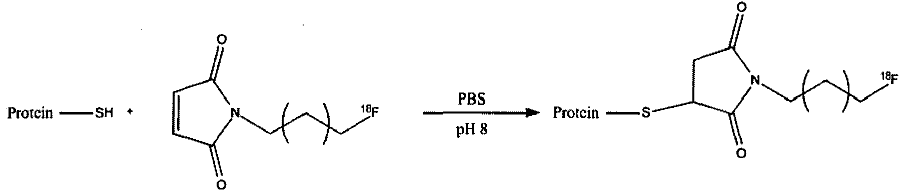

Another method is to attach a chelator, e.g. DOTA or NOTA, to the protein. This will enable the chelation of positron emitting radiometals such as ⁶⁸Ga and ⁶⁴Cu [Wängler et al. 2009]. These chelating compounds can be introduced to a protein by labelling to an prosthetic group, such as maleimide or succinimide for thiol or amine labelling, respectively. Using chelators like DOTA and other labeling methods enables the use of more pure positron-emitting PET-isotopes that may increase image resolution (less gamma coincidences). The structures of NOTA and DOTA are shown in the table below:

| NOTA | DOTA |
|---|---|
| 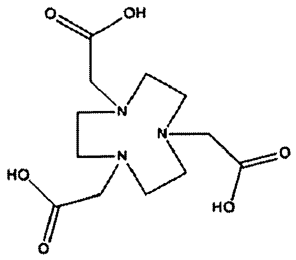 | 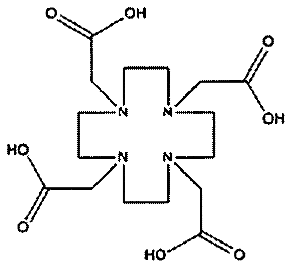 |

In one embodiment, labelling is at the amine-site such as by:
- Succinimide attached fluorobenzaldehyde, fluorobenzoate [¹⁸F]FBA or other fluorine containing prosthetic group.
- Hydrazinonicotinic acid attached to fluorobenzaldehyde through hydrazone formation.
- Silicon-fluorine acceptor (SiFA) method.

In one embodiment labelling is at the thiol-site such as by:
- Maleimide attached to fluorobenzaldehyde or other fluorine containing prosthetic group. E.g. [¹⁸F]FBEM, [¹⁸F]FBAM, [¹⁸F]FBABM, [¹⁸F]FBOM, [¹⁸F]FPyMe or [¹⁸F]FDG-MHO.

In one embodiment labelling is performed with "click chemistry" such as by:
- 1,3-cycloaddition with azide and a ¹⁸F-labelled alkyne.

In one embodiment labelling is performed with DOTA, NOTA or a similar chelator such as by:
- Attachment of DOTA, or similar polydentate chelator, to a prosthetic group. e.g. through a thiol- or amine-linker.
- Labelling DOTA with ⁶⁸Ga, ⁶⁴Cu, ⁸⁶Y, etc.

In one embodiment labelling is performed with direct halogenation:
- Labelling protein directly via lodogen-method.
- Labelling protein directly via Chloramine T-method.

### Selecting the complex

The biomolecule complexes of desired charge and size are purified and used as a contrast agent in PET based imaging methods.

Methods of separation are well-known in the art. The preferred methods of separation include separation by size, and by charge. The selection may for example be performed by one or more chromatography steps such as by gel chromatography and/or ion exchange chromatography.

### Complexes of the invention

The present invention relates to contrast agents in the form of complexes comprising one or more biomolecules linked to one or more markers, including contrast agents obtainable by the methods described above for use in PET imaging.

Complexes according to the present invention may be illustrated by the general formula B-X-M, wherein B is indicative of one or more biomolecules, X is an optional linker moiety, for example a chelator, and M is a marker moiety, wherein the marker moiety comprises at least one PET isotope.

The one or more biomolecules and the one or more markers which are part of the complexes are discussed in more detail above.

The complexes according to the invention are preferably differentially accumulated in a target compartment. The contrast agents are useful in imaging techniques for visualising target compartments in an individual.

The methods of making the complexes make it possible to tailor complexes for use in visualising specific target compartments (see above).

In one embodiment of the present invention, the complex comprises a paramagnetic marker as the one or more markers of the complex. The presence of a paramagnetic marker in the complex of the present invention makes the complex of the present invention suitable as a dual contrast agent for use in PET-MRI.

In one embodiment the present invention refers to dual contrast agents suitable for PET-MRI imaging.

In a further embodiment, the complex according to the present invention comprises aprotinin linked to a paramagnetic marker for use as a dual contrast agent in PET-MRI imaging.

Another embodiment of the present invention is directed to a complex comprising chymotrypsinogen A linked to a paramagnetic marker for use as a dual contrast agent in PET-MRI imaging.

A still further embodiment of the present invention is directed to a complex comprising lysozyme linked to a paramagnetic marker for use as a dual contrast agent in PET-MRI imaging.

In yet another embodiment the present invention is directed to a complex comprising ovalbumin linked to a paramagnetic marker for use as a dual contrast agent in PET-MRI imaging.

### Methods of using complexes

### GFR-markers

In one embodiment of the present invention the biomolecule complex is used as a marker for glomerular filtration rate, i.e. as a GFR-marker.

Polypeptides and small proteins with a molecular weight in the range of 1000 to 20.000 Da are freely filtered in the glomeruli and are therefore useful as GFR markers since the filtered load is accumulated in the renal cortex. Examples of freely filtered proteins suitable for use as GFR-markers according to the present invention include Aprotinin, Lysozyme, Chymotrypsinogen, Ovalbumin, Cystatin C, Cytochrome C and Ribonuclease.

GFR may be calculated by the following formula: GFR = Q/P,

where Q = accumulated tracer activity in kidney cortex (or region of interest) and P = time integrated tracer activity in plasma from time of injection to the time of renal scanning. The word tracer is used interchangeably with the word complex herein. The renal scanning must take place in a time window where the filtered amount of the tracer is still quantitatively retained in the proximal tubular cells, i.e. before digestion in the lysosomes is initiated. This time window ranges from about 5 minutes (cystatin C, cytochrome C) to about 30 minutes (Aprotinin, Lysozyme) depending on the protein used. For details, see: Tenstad et al. 1996.

Use of the complexes according to the present invention allows for the calculation of both total and/or regional GFR in the kidney without the need for sampling blood or urine. The total or absolute value for GFR (in ml per minute per kidney) can be thus be obtained. The present invention further allows for the calculation of the GFR in any local volumes of the renal cortex (in ml per minute per cm3 of tissue volume) down to the size limited by the resolution of the imaging modality (e.g. MRI or PET).

### Proteinuria markers

In one embodiment of the present invention the biomolecule complex is used as a marker of proteinuria.

Larger molecules ranging from 15 kDa to 100 kDa are normally not freely filtered in the glomeruli and are therefore useful as markers of proteinuria. Proteinuria is defined as excess protein in the urine and is caused by increased permeability in the glomerular filtration barrier allowing larger proteins than normal to be filtered. If the glomerular barrier is perturbated by disease, the protein permeability increases and therefore also the filtered load of the proteinuria marker-proteins. The increased load of proteinuria marker proteins in tubular fluid results in increased uptake into proximal tubular cells (via megalin/cubulin and endocytosis that can be visualized by PET provided the protein is labelled with a PET-isotope. Proteinuria is then presented as local or generalized increase in tracer accumulation in the kidney cortex and/or increased urinary excretion (accumulation of the complex in the bladder).

A suitable proteinuria marker is any complex with molecular weights ranging from about 15 kDa to 100 kDa or stokes radii ranging from 15 to 50 A (2-5 nm) providing that the filtered amount of the complex is nearly completely resorbed by the proximal tubular cells. The proteinuria is detected as increased accumulation of the complex in the kidney cortex, wherein said accumulation can be either general or local. To be suitable for use a marker of proteinuria according to the present invention, the complex should be retained in the kidney cortex for a period of at least 5 minutes following introduction of the complex, such as by i.v. injection, into the individual, such as a human being.

In a preferred embodiment the complex for use a marker of proteinuria is not toxic. This is preferably particularly for human use. In another embodiment, the complex for use a marker of proteinuria is of low toxicity.

The nature of the proteinuria may be characterized by comparing renal uptake of two or more complexes with similar hydrodynamic radius, but with different molecular charge and/or stiffness (compressibility). The complexes of the present invention can thus be used in measuring the effect of net molecular charge as well as molecular shape and compressibility on the passage of macromolecules across the glomerular capillary wall.

In a preferred embodiment, the complex used as a marker of proteinuria is a protein with a molecular weight ranging from about 15 kDa to 100 kDa or a stokes radii ranging from 15 to 50 A (2-5 nm). Examples of suitable proteins according to the present invention are proteins comprised within the group consisting of but in no way limited to Lysozyme, Chymotrypsinogen A, Ovalbumin, myoglobin, albumin, Horseradish peroxidase, transthyretin, albumin and immunoglobulin's or fragment and /or variants thereof like charge modifications.

### Markers of kidney tubular function

In one embodiment of the present invention the biomolecule complex is used as a marker of kidney tubular function.

Since tracer uptake in kidney cortex requires intact tubular function, this method can also be used to visualize tubular function. In this case any biomolecule can be used as PET-tracers for tubular function provided it can be labelled by a PET-tracer and that more than 90% of the filtered amount is normally completely reabsorbed from tubular fluid to tubular cells (glucose, amino acids, oligopeptides, polypeptides and small proteins).

Perturbation in tubular function is visualized as increased accumulation in bladder and decreased accumulation in kidney cortex compared to the normal situation e.g. a ratio between urinary contrast agent excretion rate and total renal contrast agent excretion rate > 0.1. Local defects in tubular function are presented as "cold" spots in the kidney cortex, i.e. decreased local tracer uptake due to locally impaired tubular function.

Examples of freely filtered proteins that are resorbed in the proximal tubules of the kidney and are suitable for use as markers for the evaluation of kidney tubular function according to the present are Megalin and/or Cubulin ligands such as Aprotinin, Lysozyme, Chymotrypsinogen, Ovalbumin, Cystatin C, Cytochrome C and Ribonuclease.

### The PET method

The biomolecule complexes according to the present invention are useful for both static and dynamic imaging processes. One example of a dynamic imaging process is visualising the glomerular filtration rate in the kidney. One example of static imaging is the use of the complexes in PET-scans to localise the presence of cancer tumors.

The present invention further relates to PET based methods wherein complexes according to the invention, or contrast agents comprising such complexes, are used. In particular, there is provided a PET imaging method for the measurement of the glomerular filtration rate of the kidney in an individual, the method comprising the steps of administering to an individual the complex according to the invention, capturing images of the kidney of said individual and calculating the glomerular filtration rate on the basis of the captured images.

Examples of visualisation techniques further comprise MRI and radiographic methods such as computer tomography-(CT) based imaging methods.

In one embodiment the present invention relates to a method in which the PET scans are read alongside CT or MRI scans, the combination ("co-registration") giving both anatomic and functional information (i.e., what the structure is, and what it is doing biochemically).

In one embodiment, the PET based method is PET.

In another embodiment, the PET based method is PET-CT.

Another example of a visualisation technique suitable for evaluation of target compartment function, such as evaluation and calculation of GFR is PET-MRI. When performing PET-MRI scans dual contrast agents are used. A dual contrast agent according to the present invention comprises a biomolecule suitable for visualisation of a desired target compartment, a paramagnetic marker for obtaining an MRI signal and a PET isotope for obtaining a PET signal, wherein said PET isotope can be linked to or incorporated into either the biomolecule or the paramagnetic marker.

In one embodiment the PET based method according to the present invention is PET-MRI.

The complexes of the present invention can be used for functional assessment of an organ, such as functional assessment of a kidney in an individual, such as a human being, functional assessment of a small intestine in an individual, such as a human being, and assessment of the vasculature in an individual, such as a human being.

In the example of the kidney, the accumulation in the kidney enables sensitive and high resolution scans of the kidney to be performed. This in turn renders possible quantitative assessment of the GFR. Moreover, since a proteinuric kidney displays characteristic PET scans depending on the source of proteinuria, the methods of the invention also make it possible to gather detailed information on the underlying cause of the proteinuria. Another advantage of the present invention is that information from single kidneys can be gained.

In another embodiment the invention provides a method for functional assessment of the kidney by performing sequential PET scans of the subject using complexes with different traits. Combination of data acquired from scans performed using different complexes of the invention provides detailed information on the type and scope of kidney damage/function.

The invention also relates to methods for performing PET scans. Use of more than one complex preferably gives more information than can be gained by a single scan of a single complex.

The above-cited PET based methods can be used for both diagnostic and non-diagnostic applications. Accordingly, in one aspect the present invention relates to a method for diagnosing individuals suffering from an abnormal glomerular filtration rate of the kidney.

In another embodiment the present invention relates to a non-diagnostic PET based method for the assessment and/or calculation of the glomerular filtration rate of the kidney in an individual in need thereof.

The one or more complexes of the present invention may be administered simultaneously, for example more than one complex may be present in a composition for use as a contrast agent.

In another embodiment the complexes may be administered sequentially in any order, wherein one complex is administered first and a second complex is administered after a certain time. This time interval may be from about 5 minutes and up to about 48 hours.

The biomolecule complexes of the present invention can be used for any PET-based analysis including the ones mentioned below.
- Brain Imaging: Stroke and tumor evaluation, vascular imaging, surgery planning, PET Angiography or Venography studies, PET Diffusion and Perfusion Imaging, PET brain scan, PET neuroimaging.
- Cardiac Imaging: RV dysplasia, Congenital, Tumor, Mass etc.
- Vascular Imaging: Imaging and enhancing vessels with PET Angiography.
- Spine Imaging: Cervical, thoracic, lumbar and spine imaging for congenital abnormalities, PET neuroimaging, cord compression, tumor, HNP, sciatica etc.
- Abdomen/Pelvis Imaging: cancer diagnosing/staging, PET Angiographic studies, Renal donor evaluations, Graft and Aneurysm evaluation.
- Kidney imaging.
- Orthopedic Imaging: Imaging of soft tissue, muscle ligaments, and bones.

Furthermore, the biomolecule complexes of the present invention can be used for any PET analysis areas mentioned below:
- atherosclerosis and vascular diseases, hibernating myocardium identification.
- neuropsychology and cognitive neuroscience to examine links between specific psychological processes or disorders and brain activity, psychiatry examining the state of the dopamine receptors (D1,D2, reuptake transporter), serotonin receptors (5HT1A, 5HT2A, reuptake transporter) opioid receptors (mu) and other receptors in patients compared to healthy controls in schizophrenia, substance abuse, mood disorders and other psychiatric conditions.
- Pharmacology, in pre-clinical trials.

To conduct the PET scan, a short-lived radioactive PET isotope, is introduced into the individual, e.g. by injected it into the individual (usually into blood circulation). In one embodiment the PET isotope is chemically incorporated into a biomolecule or a marker molecule linked to said biomolecule. There is a waiting period while the active biomolecule becomes concentrated in the one or more tissues of interest; then the research subject or patient is placed in the PET imaging scanner. An example of a molecule commonly used for this purpose is fluorodeoxyglucose (FDG), a sugar, for which the waiting period is typically an hour. The waiting period depends on how quickly the chosen biomolecule is distributed in the individual, e.g. how quickly it becomes localised in the desired target compartment to be visualised. During the scan a record of target compartment concentration is made as the PET isotope decays.

As the radioisotope undergoes positron emission decay (also known as positive beta decay), it emits a positron, a particle with the opposite charge of an electron. After travelling up to a few millimeters the positron encounters and annihilates with an electron, producing a pair of annihilation (gamma) photons moving in opposite directions. These are detected when they reach a scintillator material in the scanning device, creating a burst of light which is detected by photomultiplier tubes or silicon avalanche photodiodes (Si APD). The technique depends on simultaneous or coincident detection of the pair of photons; photons which do not arrive in pairs (i.e. within a timing window of few nanoseconds) are ignored.

### Localization of the positron annihilation event

The most significant fraction of electron-positron decays result in two 511 keV gamma photons being emitted at almost 180 degrees to each other; hence it is possible to localize their source along a straight line of coincidence (also called formally the line of response or LOR). In practice the LOR has a finite width as the emitted photons are not exactly 180 degrees apart. If the recovery time of detectors is in the picosecond range rather than the 10's of nanosecond range, it is possible to localize the event to a segment of a cord, whose length is determined by the detector timing resolution. As the timing resolution improves, the signal-to-noise ratio (SNR) of the image will improve, requiring fewer events to achieve the same image quality. This technology is not yet common, but it is available on some new systems.

### Image reconstruction using coincidence statistics

More commonly, a technique much like the reconstruction of computed tomography (CT) and single photon emission computed tomography (SPECT) data is used.

Using statistics collected from tens-of-thousands of coincidence events, a set of simultaneous equations for the total activity of each parcel of tissue along many LORs can be solved by a number of techniques, and thus a map of radioactivities as a function of location for parcels or bits of tissue (also called voxels), may be constructed and plotted. The resulting map shows the tissues in which the molecular probe has become concentrated, and can be interpreted by a nuclear medicine physician or radiologist in the context of the patient's diagnosis and treatment plan.

### Combination of PET with CT or MRI

PET scans are increasingly read alongside CT or magnetic resonance imaging (MRI) scans, the combination ("co-registration") giving both anatomic and functional information (i.e., what the structure is, and what it is doing biochemically). Because PET imaging is most useful in combination with anatomical imaging, such as CT, modem PET scanners are now available with integrated high-end multi-detector-row CT scanners. Because the two scans can be performed in immediate sequence during the same session, with the patient not changing position between the two types of scans, the two sets of images are more-precisely registered, so that areas of abnormality on the PET imaging can be more perfectly correlated with anatomy on the CT images. This is very useful in showing detailed views of moving organs or structures with higher anatomical variation, which is more frequent outside of the brain.

PET-MRI combination scans are also useful for combining the functional and/or metabolic information obtained from the PET scan with the high resolution anatomic information provided by MRI.

### Radioisotopes

Radionuclides used in PET scanning are typically isotopes with short half lives such as carbon-11 (~20 min), nitrogen-13 (~10 min), oxygen-15 (~2 min), and fluorine-18 (~110 min). These radionuclides are incorporated either into compounds normally used by the body such as glucose (or glucose analogues), water or ammonia, or into molecules that bind to receptors or other sites of drug action. Such labelled compounds are also known as radiotracers. It is important to recognize that PET technology can be used to trace the biologic pathway of any compound in living humans (and many other species as well), provided it can be radiolabelled with a PET isotope. Thus the specific processes that can be probed with PET are virtually limitless, and radiotracers for new target molecules and processes are being synthesized continuously; as of this writing there are already dozens in clinical use and hundreds applied in research. Due to the short half lives of most radioisotopes used in PET imaging, the radiotracers are usually produced using a cyclotron and radiochemistry laboratory in close proximity to the PET imaging facility. The half life of fluorine-18 is long enough so that fluorine-18 labelled radiotracers can be manufactured commercially at an offsite location.

### Image reconstruction

The raw data collected by a PET scanner are a list of 'coincidence events' representing near-simultaneous detection of annihilation photons by a pair of detectors. Each coincidence event represents a line in space connecting the two detectors along which the positron emission occurred.

Coincidence events can be grouped into projections images, called sinograms. The sinograms are sorted by the angle of each view and tilt, the latter in 3D case images. The sinogram images are analogous to the projections captured by computed tomography (CT) scanners, and can be reconstructed in a similar way. A normal PET data set has millions of counts for the whole acquisition, while a CT data set can reach a few billion counts, making the reconstruction of PET images more challenging that reconstruction of a CT image.

In practice, considerable pre-processing of the PET data is required - correction for random coincidences, estimation and subtraction of scattered photons, detector dead-time correction (after the detection of a photon, the detector must "cool down" again) and detector-sensitivity correction (for both inherent detector sensitivity and changes in sensitivity due to angle of incidence).

Filtered back projection (FBP) has been frequently used to reconstruct images from the projections. This algorithm has the advantage of being simple while having a low requirement for computing resources. However, shot noise in the raw data is prominent in the reconstructed images and areas of high tracer uptake tend to form streaks across the image.

Iterative expectation-maximization algorithms are now the preferred method of reconstruction. The advantage is a better noise profile and resistance to the streak artefacts common with FBP, but the disadvantage is higher computer resource requirements.

Attenuation correction: As different LORs must traverse different thicknesses of tissue, the photons are attenuated differentially. The result is that structures deep in the body are reconstructed as having falsely low tracer uptake. Contemporary scanners can estimate attenuation using integrated x-ray CT equipment, however earlier equipment offered a crude form of CT using a gamma ray (positron emitting) source and the PET detectors.

While attenuation corrected images are generally more faithful representations, the correction process is itself susceptible to significant artefacts. As a result, both corrected and uncorrected images are always reconstructed and read together.

2D/3D reconstruction: Early PET scanners had only a single ring of detectors, hence the acquisition of data and subsequent reconstruction was restricted to a single transverse plane. More modem scanners now include multiple rings, essentially forming a cylinder of detectors.

There are two approaches to reconstructing data from such a scanner: 1) treat each ring as a separate entity, so that only coincidences within a ring are detected, the image from each ring can then be reconstructed individually (2D reconstruction), or 2) allow coincidences to be detected between rings as well as within rings, then reconstruct the entire volume together (3D).

3D techniques have better sensitivity (because more coincidences are detected and used) and therefore less noise, but are more sensitive to the effects of scatter and random coincidences, as well as requiring correspondingly greater computer resources. The advent of sub-nanosecond timing resolution detectors affords better random coincidence rejection, thus favouring 3D image reconstruction.

In one embodiment, the PET data acquisition has between about 1 million to about 1 billion counts, for example about 1 million, for example about 2 millions, for example about 3 millions, for example about 4 millions, for example about 5 millions, for example about 6 millions, for example about 7 millions, for example about 8 millions, for example about 9 millions, for example about 10 millions, for example about 15 millions, for example about 20 millions, for example about 25 millions, for example about 30 millions, for example about 35 millions, for example about 40 millions, for example about 45 millions, for example about 50 millions, for example about 55 millions, for example about 60 millions, for example about 65 millions, for example about 70 millions, for example about 75 millions, for example about 80 millions, for example about 85 millions, for example about 90 millions, for example about 95 millions, for example about 100 millions, for example about 150 millions, for example about 200 millions, for example about 250 millions, for example about 300 millions, for example about 350 millions, for example about 400 millions, for example about 450 millions, for example about 500 millions, for example about 600 millions, for example about 700 millions, for example about 800 millions, for example about 900 millions, for example about 1 billions or more counts.

In another embodiment, the PET data acquisition has between 1 million to 1 billion counts, such as 1 to 2 million, for example 2 to 3 millions, for example 3 to 4 millions, for example 4 to 5 millions, for example 5 to 6 millions, for example 6 to 7 millions, for example 7 to 8 millions, for example 8 to 9 millions, for example 9 to 10 millions, for example 10 to 15 millions, for example 15 to 20 millions, for example 20 to 25 millions, for example 25 to 30 millions, for example 30 to 35 millions, for example 35 to 40 millions, for example 40 to 45 millions, for example 45 to 50 millions, for example 50 to 55 millions, for example 55 to 60 millions, for example 60 to 65 millions, for example 65 to 70 millions, for example 70 to 75 millions, for example 75 to 80 millions, for example 80 to 85 millions, for example 85 to 90 millions, for example 90 to 95 millions, for example 95 to 100 millions, for example 100 to 150 millions, for example 150 to 200 millions, for example 200 to 250 millions, for example 350 to 400 millions, for example 400 to 450 millions, for example 500 to 600 millions, for example 600 to 700 millions, for example 700 to 800 millions, for example 800 to 900 millions, for example 900 to 1 billion counts.

### Complexes for use in PET imaging of kidney

One example of uses for the complexes of the invention concerns use for visualising the glomerular filtration rate in the kidney.

Illustrative, non-limiting examples of biomolecules according to the present invention suitable for PET imaging of the kidney include globular proteins of from 1 kDa to preferably less than 500 kDa, such as polypeptides comprising or consisting of aprotinin, chymtropsinogen A, lysozyme, ovalbumin, ribonucleases and cytochrome C, cystatin C as well as fragments and/or variants thereof.

In one embodiment of the invention, the one or more biomolecules of the invention comprise or consist of a Megalin and/or Cubulin ligand or a fragment and/or variant of said ligand. Megalin and or Cubulin ligands will bind to Megalin and or Cubulin in the proximal tubules of the kidney and be internalised into proximal tubule cells.

In one embodiment of the invention, the one or more biomolecules of the invention comprise or consist of an aprotinin polypeptide.

In a further embodiment of the invention, the one or more biomolecules of the invention comprise or consist of a chymotrypsinogen A polypeptide.

In another embodiment of the invention, the one or more biomolecules of the invention comprise or consist of a lysozyme polypeptide.

In a further embodiment of the invention, the one or more biomolecules of the invention comprise or consist of an ovalbumin polypeptide.

In a further embodiment of the invention, the one or more biomolecules of the invention comprise or consist of a Cystatin C polypeptide.

In a further embodiment of the invention, the one or more biomolecules of the invention comprise or consist of a Cytochrome C polypeptide.

In a further embodiment of the invention, the one or more biomolecules of the invention comprise or consist of a Ribonuclease polypeptide.

The biomolecule moiety of the contrast agent may furthermore comprise a fragment of a biomolecule, such as a polypeptide, for example a globular polypeptide, such as one or more fragments of aprotinin, chymtropsinogen A, lysozyme, ovalbumin, Cystatin C, Cytochrome C or Ribonuclease.

The invention in yet another embodiment comprises complexes wherein the biomolecule or biomolecules are genetically engineered. Said engineering can include recombinant production of the biomolecule or fragment or variant of the biomolecule.

Further particular embodiments include biomolecules engineered so as to confer to the complex the ability to accumulate in the kidney, or to enhance an integral ability of the biomolecule to do so. An example of such engineering includes introducing a binding site for Megalin and/or Cubilin to a biomolecule. A further example of modification is to engineer the biomolecule so as to change the electric charge of the biomolecule.

In one particular embodiment, the complex comprises aprotinin as a biomolecule. Aprotinin interacts specifically with the Megalin/Cubulin complex and is specifically resorbed in the proximal tubuli. In this embodiment the complex further comprises DTPA as a linker moiety. An alternative to using DTPA is to use DOTA or NOTA as the linker moiety. The complex may further comprise a paramagnetic marker as a marker moiety thus making the complex suitable for PET-MRI combination imaging.

In one example, provided in order to illustrate and not to limit, a complex comprising a Megalin/Cubulin ligand, such as aprotinin, linked to a marker in the form of a PET isotope is administered intravenously to an individual and PET-based imaging performed. Imaging visualises kidney functionality including the glomerular filtration rate of said individual. Subsequently, a complex comprising a lysozyme derivative linked to a marker comprising a PET isotope is administered intravenously to the same individual and PET imaging performed. The images acquired in the two scans are compared. Deficient uptake of the complex comprising the lysozyme derivative (tubular injury inhibits absorption of the complex comprising the lysozyme derivative) in the absence of deficient uptake of the complex comprising aprotinin (absorbed even by injured tubules) indicates damage (e.g. after acute ischemia) locally to tubuli which has not yet resulted in decreased glomerular filtration rate.

In one embodiment of the invention, the one or more biomolecule complex(es) of the invention comprise or consist of an aprotinin polypeptide in combination with one or more PET isotopes, such as one or more of the isotopes of Aluminium, Barium, Calcium, Dysprosium, Gadolinium, Iodine, Copper, Magnesium, Manganese, Oxygen, Platinum, Sodium, Strontium, Technetium, Fluorine and Uranium or any variant thereof and/or mixtures thereof.

In a further embodiment of the invention, the one or more biomolecule complex(es) of the invention comprise or consist of a chymotrypsinogen A polypeptide in combination with one or more PET isotopes such as one or more of the isotopes of Aluminium, Barium, Calcium, Dysprosium, Gadolinium, Iodine, Copper, Magnesium, Manganese, Oxygen, Platinum, Sodium, Strontium, Technetium, Fluorine and Uranium or any variant thereof and/or mixtures thereof.

In another embodiment of the invention, the one or more biomolecule complex(es) of the invention comprise or consist of a lysozyme polypeptide in combination with one or more PET isotopes such as one or more of the isotopes of Aluminium, Barium, Calcium, Dysprosium, Gadolinium, Iodine, Copper, Magnesium, Manganese, Oxygen, Platinum, Sodium, Strontium, Technetium, Fluorine and Uranium or any variant thereof and/or mixtures thereof.

In a further embodiment of the invention, the one or more biomolecule complex(es) of the invention comprise or consist of an ovalbumin polypeptide in combination with one or more PET isotopes such as one or more of the isotopes of Aluminium, Barium, Calcium, Dysprosium, Gadolinium, Iodine, Copper, Magnesium, Manganese, Oxygen, Platinum, Sodium, Strontium, Technetium, Fluorine and Uranium or any variant thereof and/or mixtures thereof.

In a further embodiment of the invention, the one or more biomolecule complex(es) of the invention comprise or consist of an Cystatin C polypeptide in combination with one or more PET isotopes such as one or more of the isotopes of Aluminium, Barium, Calcium, Dysprosium, Gadolinium, Iodine, Copper, Magnesium, Manganese, Oxygen, Platinum, Sodium, Strontium, Technetium, Fluorine and Uranium or any variant thereof and/or mixtures thereof.

In a further embodiment of the invention, the one or more biomolecule complex(es) of the invention comprise or consist of an Cytochrome C polypeptide in combination with one or more PET isotopes such as one or more of the isotopes of Aluminium, Barium, Calcium, Dysprosium, Gadolinium, Iodine, Copper, Magnesium, Manganese, Oxygen, Platinum, Sodium, Strontium, Technetium, Fluorine and Uranium or any variant thereof and/or mixtures thereof.

In a further embodiment of the invention, the one or more biomolecule complex(es) of the invention comprise or consist of an Ribonuclease polypeptide in combination with one or more PET isotopes such as one or more of the isotopes of Aluminium, Barium, Calcium, Dysprosium, Gadolinium, Iodine, Copper, Magnesium, Manganese, Oxygen, Platinum, Sodium, Strontium, Technetium, Fluorine and Uranium or any variant thereof and/or mixtures thereof.

In a particular embodiment, the one or more biomolecule complex(es) comprise or consist of a Megalin/Cubulin ligand such as aprotinin polypeptide in combination with a PET isotope of Aluminium.

In a particular embodiment, the one or more biomolecule complex(es) comprise or consist of a Megalin/Cubulin ligand such as aprotinin polypeptide in combination with a PET isotope of Barium.

In a particular embodiment, the one or more biomolecule complex(es) comprise or consist of a Megalin/Cubulin ligand such as aprotinin polypeptide in combination with a PET isotope of Calcium

In a particular embodiment, the one or more biomolecule complex(es) comprise or consist of a Megalin/Cubulin ligand such as aprotinin polypeptide in combination with a PET isotope of Dysprosium.

In a particular embodiment, the one or more biomolecule complex(es) comprise or consist of a Megalin/Cubulin ligand such as aprotinin polypeptide in combination with a PET isotope of Gadolinium.

In a particular embodiment, the one or more biomolecule complex(es) comprise or consist of a Megalin/Cubulin ligand such as aprotinin polypeptide in combination with a PET isotope of Magnesium.

In a particular embodiment, the one or more biomolecule complex(es) comprise or consist of a Megalin/Cubulin ligand such as aprotinin polypeptide in combination with a PET isotope of Manganese.

In a particular embodiment, the one or more biomolecule complex(es) comprise or consist of a Megalin/Cubulin ligand such as aprotinin polypeptide in combination with a PET isotope of Oxygen.

In a particular embodiment, the one or more biomolecule complex(es) comprise or consist of a Megalin/Cubulin ligand such as aprotinin polypeptide in combination with a PET isotope of Platinum.

In a particular embodiment, the one or more biomolecule complex(es) comprise or consist of a Megalin/Cubulin ligand such as aprotinin polypeptide in combination with a PET isotope of Sodium.

In a particular embodiment, the one or more biomolecule complex(es) comprise or consist of a Megalin/Cubulin ligand such as aprotinin polypeptide in combination with a PET isotope of Strontium.

In a particular embodiment, the one or more biomolecule complex(es) comprise or consist of a Megalin/Cubulin ligand such as aprotinin polypeptide in combination with a PET isotope of Technetium.

In a particular embodiment, the one or more biomolecule complex(es) comprise or consist of a Megalin/Cubulin ligand such as aprotinin polypeptide in combination with a PET isotope of Uranium.

In a particular embodiment, the one or more biomolecule complex(es) comprise or consist of a Megalin/Cubulin ligand such as aprotinin polypeptide in combination with a PET isotope of fluorine.

In a particular embodiment, the one or more biomolecule complex(es) comprise or consist of a Megalin/Cubulin ligand such as aprotinin polypeptide in combination with a PET isotope of Iodine.

In a particular embodiment, the one or more biomolecule complex(es) comprise or consist of a Megalin/Cubulin ligand such as aprotinin polypeptide in combination with a PET isotope of Copper.

In a particular embodiment, the one or more biomolecule complex(es) comprise or consist of a Megalin/Cubulin ligand such as a chymotrypsinogen A polypeptide in combination with a PET isotope of Aluminium.

In a particular embodiment, the one or more biomolecule complex(es) comprise or consist of a Megalin/Cubulin ligand such as a chymotrypsinogen A polypeptide in combination with a PET isotope of Barium.

In a particular embodiment, the one or more biomolecule complex(es) comprise or consist of a Megalin/Cubulin ligand such as a chymotrypsinogen A polypeptide in combination with a PET isotope of Calcium

In a particular embodiment, the one or more biomolecule complex(es) comprise or consist of a Megalin/Cubulin ligand such as a chymotrypsinogen A polypeptide in combination with a PET isotope of Dysprosium.

In a particular embodiment, the one or more biomolecule complex(es) comprise or consist of a Megalin/Cubulin ligand such as a chymotrypsinogen A polypeptide in combination with a PET isotope of Gadolinium.

In a particular embodiment, the one or more biomolecule complex(es) comprise or consist of a Megalin/Cubulin ligand such as a chymotrypsinogen A polypeptide in combination with a PET isotope of Magnesium.

In a particular embodiment, the one or more biomolecule complex(es) comprise or consist of a Megalin/Cubulin ligand such as a chymotrypsinogen A polypeptide in combination with a PET isotope of Manganese.

In a particular embodiment, the one or more biomolecule complex(es) comprise or consist of a Megalin/Cubulin ligand such as a chymotrypsinogen A polypeptide in combination with a PET isotope of Oxygen.

In a particular embodiment, the one or more biomolecule complex(es) comprise or consist of a Megalin/Cubulin ligand such as a chymotrypsinogen A polypeptide in combination with a PET isotope of Platinum.

In a particular embodiment, the one or more biomolecule complex(es) comprise or consist of a Megalin/Cubulin ligand such as a chymotrypsinogen A polypeptide in combination with a PET isotope of Sodium.

In a particular embodiment, the one or more biomolecule complex(es) comprise or consist of a Megalin/Cubulin ligand such as a chymotrypsinogen A polypeptide in combination with a PET isotope of Strontium.

In a particular embodiment, the one or more biomolecule complex(es) comprise or consist of a Megalin/Cubulin ligand such as a chymotrypsinogen A polypeptide in combination with a PET isotope of Technetium.

In a particular embodiment, the one or more biomolecule complex(es) comprise or consist of a Megalin/Cubulin ligand such as a chymotrypsinogen A polypeptide in combination with a PET isotope of Uranium.

In a particular embodiment, the one or more biomolecule complex(es) comprise or consist of a Megalin/Cubulin ligand such as a chymotrypsinogen A polypeptide in combination with a PET isotope of fluorine.

In a particular embodiment, the one or more biomolecule complex(es) comprise or consist of a Megalin/Cubulin ligand such as a chymotrypsinogen A polypeptide in combination with a PET isotope of Iodine.

In a particular embodiment, the one or more biomolecule complex(es) comprise or consist of a Megalin/Cubulin ligand such as a chymotrypsinogen A polypeptide in combination with a PET isotope of Copper.

In a particular embodiment, the one or more biomolecule complex(es) comprise or consist of a Megalin/Cubulin ligand such as a lysozyme polypeptide in combination with a PET isotope of Aluminium.

In a particular embodiment, the one or more biomolecule complex(es) comprise or consist of a Megalin/Cubulin ligand such as a lysozyme polypeptide in combination with a PET isotope of Barium.

In a particular embodiment, the one or more biomolecule complex(es) comprise or consist of a Megalin/Cubulin ligand such as a lysozyme polypeptide in combination with a PET isotope of Calcium

In a particular embodiment, the one or more biomolecule complex(es) comprise or consist of a Megalin/Cubulin ligand such as a lysozyme polypeptide in combination with a PET isotope of Dysprosium.

In a particular embodiment, the one or more biomolecule complex(es) comprise or consist of a Megalin/Cubulin ligand such as a lysozyme polypeptide in combination with a PET isotope of Gadolinium.

In a particular embodiment, the one or more biomolecule complex(es) comprise or consist of a Megalin/Cubulin ligand such as a lysozyme polypeptide in combination with a PET isotope of Magnesium.

In a particular embodiment, the one or more biomolecule complex(es) comprise or consist of a Megalin/Cubulin ligand such as a lysozyme polypeptide in combination with a PET isotope of Manganese.

In a particular embodiment, the one or more biomolecule complex(es) comprise or consist of a Megalin/Cubulin ligand such as a lysozyme polypeptide in combination with a PET isotope of Oxygen.

In a particular embodiment, the one or more biomolecule complex(es) comprise or consist of a Megalin/Cubulin ligand such as a lysozyme polypeptide in combination with a PET isotope of Platinum.

In a particular embodiment, the one or more biomolecule complex(es) comprise or consist of a Megalin/Cubulin ligand such as a lysozyme polypeptide in combination with a PET isotope of Sodium.

In a particular embodiment, the one or more biomolecule complex(es) comprise or consist of a Megalin/Cubulin ligand such as a lysozyme polypeptide in combination with a PET isotope of Strontium.

In a particular embodiment, the one or more biomolecule complex(es) comprise or consist of a Megalin/Cubulin ligand such as a lysozyme polypeptide in combination with a PET isotope of Technetium.

In a particular embodiment, the one or more biomolecule complex(es) comprise or consist of a Megalin/Cubulin ligand such as a lysozyme polypeptide in combination with a PET isotope of Uranium.

In a particular embodiment, the one or more biomolecule complex(es) comprise or consist of a Megalin/Cubulin ligand such as a lysozyme polypeptide in combination with a PET isotope of fluorine.

In a particular embodiment, the one or more biomolecule complex(es) comprise or consist of a Megalin/Cubulin ligand such as a lysozyme polypeptide in combination with a PET isotope of Iodine.

In a particular embodiment, the one or more biomolecule complex(es) comprise or consist of a Megalin/Cubulin ligand such as a lysozyme polypeptide in combination with a PET isotope of Copper.

In a particular embodiment, the one or more biomolecule complex(es) comprise or consist of a Megalin/Cubulin ligand such as an ovalbumin polypeptide in combination with a PET isotope of Aluminium.

In a particular embodiment, the one or more biomolecule complex(es) comprise or consist of a Megalin/Cubulin ligand such as an ovalbumin polypeptide in combination with a PET isotope of Barium.

In a particular embodiment, the one or more biomolecule complex(es) comprise or consist of a Megalin/Cubulin ligand such as an ovalbumin polypeptide in combination with a PET isotope of Calcium

In a particular embodiment, the one or more biomolecule complex(es) comprise or consist of a Megalin/Cubulin ligand such as an ovalbumin polypeptide in combination with a PET isotope of Dysprosium.

In a particular embodiment, the one or more biomolecule complex(es) comprise or consist of a Megalin/Cubulin ligand such as an ovalbumin polypeptide in combination with a PET isotope of Gadolinium.

In a particular embodiment, the one or more biomolecule complex(es) comprise or consist of a Megalin/Cubulin ligand such as an ovalbumin polypeptide in combination with a PET isotope of Magnesium.

In a particular embodiment, the one or more biomolecule complex(es) comprise or consist of a Megalin/Cubulin ligand such as an ovalbumin polypeptide in combination with a PET isotope of Manganese.

In a particular embodiment, the one or more biomolecule complex(es) comprise or consist of a Megalin/Cubulin ligand such as an ovalbumin polypeptide in combination with a PET isotope of Oxygen.

In a particular embodiment, the one or more biomolecule complex(es) comprise or consist of a Megalin/Cubulin ligand such as an ovalbumin polypeptide in combination with a PET isotope of Platinum.

In a particular embodiment, the one or more biomolecule complex(es) comprise or consist of a Megalin/Cubulin ligand such as an ovalbumin polypeptide in combination with a PET isotope of Sodium.

In a particular embodiment, the one or more biomolecule complex(es) comprise or consist of a Megalin/Cubulin ligand such as an ovalbumin polypeptide in combination with a PET isotope of Strontium.

In a particular embodiment, the one or more biomolecule complex(es) comprise or consist of a Megalin/Cubulin ligand such as an ovalbumin polypeptide in combination with a PET isotope of Technetium.

In a particular embodiment, the one or more biomolecule complex(es) comprise or consist of a Megalin/Cubulin ligand such as an ovalbumin polypeptide in combination with a PET isotope of Uranium.

In a particular embodiment, the one or more biomolecule complex(es) comprise or consist of a Megalin/Cubulin ligand such as an ovalbumin polypeptide in combination with a PET isotope of fluorine.

In a particular embodiment, the one or more biomolecule complex(es) comprise or consist of a Megalin/Cubulin ligand such as an ovalbumin polypeptide in combination with a PET isotope of Iodine.

In a particular embodiment, the one or more biomolecule complex(es) comprise or consist of a Megalin/Cubulin ligand such as an ovalbumin polypeptide in combination with a PET isotope of Copper.

In a particular embodiment, the one or more biomolecule complex(es) comprise or consist of a Megalin/Cubulin ligand such as a Cystatin C polypeptide in combination with a PET isotope of Aluminium.

In a particular embodiment, the one or more biomolecule complex(es) comprise or consist of a Megalin/Cubulin ligand such as a Cystatin C polypeptide in combination with a PET isotope of Barium.

In a particular embodiment, the one or more biomolecule complex(es) comprise or consist of a Megalin/Cubulin ligand such as a Cystatin C polypeptide in combination with a PET isotope of Calcium

In a particular embodiment, the one or more biomolecule complex(es) comprise or consist of a Megalin/Cubulin ligand such as a Cystatin C polypeptide in combination with a PET isotope of Dysprosium.

In a particular embodiment, the one or more biomolecule complex(es) comprise or consist of a Megalin/Cubulin ligand such as a Cystatin C polypeptide in combination with a PET isotope of Gadolinium.

In a particular embodiment, the one or more biomolecule complex(es) comprise or consist of a Megalin/Cubulin ligand such as a Cystatin C polypeptide in combination with a PET isotope of Magnesium.

In a particular embodiment, the one or more biomolecule complex(es) comprise or consist of a Megalin/Cubulin ligand such as a Cystatin C polypeptide in combination with a PET isotope of Manganese.

In a particular embodiment, the one or more biomolecule complex(es) comprise or consist of a Megalin/Cubulin ligand such as a Cystatin C polypeptide in combination with a PET isotope of Oxygen.

In a particular embodiment, the one or more biomolecule complex(es) comprise or consist of a Megalin/Cubulin ligand such as a Cystatin C polypeptide in combination with a PET isotope of Platinum.

In a particular embodiment, the one or more biomolecule complex(es) comprise or consist of a Megalin/Cubulin ligand such as a Cystatin C polypeptide in combination with a PET isotope of Sodium.

In a particular embodiment, the one or more biomolecule complex(es) comprise or consist of a Megalin/Cubulin ligand such as a Cystatin C polypeptide in combination with a PET isotope of Strontium.

In a particular embodiment, the one or more biomolecule complex(es) comprise or consist of a Megalin/Cubulin ligand such as a Cystatin C polypeptide in combination with a PET isotope of Technetium.

In a particular embodiment, the one or more biomolecule complex(es) comprise or consist of a Megalin/Cubulin ligand such as a Cystatin C polypeptide in combination with a PET isotope of Uranium.

In a particular embodiment, the one or more biomolecule complex(es) comprise or consist of a Megalin/Cubulin ligand such as a Cystatin C polypeptide in combination with a PET isotope of fluorine.

In a particular embodiment, the one or more biomolecule complex(es) comprise or consist of a Megalin/Cubulin ligand such as a Cystatin C polypeptide in combination with a PET isotope of Iodine.

In a particular embodiment, the one or more biomolecule complex(es) comprise or consist of a Megalin/Cubulin ligand such as a Cystatin C polypeptide in combination with a PET isotope of Copper.

In a particular embodiment, the one or more biomolecule complex(es) comprise or consist of a Megalin/Cubulin ligand such as a Cytochrome C polypeptide in combination with a PET isotope of Aluminium.

In a particular embodiment, the one or more biomolecule complex(es) comprise or consist of a Megalin/Cubulin ligand such as a Cytochrome C polypeptide in combination with a PET isotope of Barium.

In a particular embodiment, the one or more biomolecule complex(es) comprise or consist of a Megalin/Cubulin ligand such as a Cytochrome C polypeptide in combination with a PET isotope of Calcium

In a particular embodiment, the one or more biomolecule complex(es) comprise or consist of a Megalin/Cubulin ligand such as a Cytochrome C polypeptide in combination with a PET isotope of Dysprosium.

In a particular embodiment, the one or more biomolecule complex(es) comprise or consist of a Megalin/Cubulin ligand such as a Cytochrome C polypeptide in combination with a PET isotope of Gadolinium.

In a particular embodiment, the one or more biomolecule complex(es) comprise or consist of a Megalin/Cubulin ligand such as a Cytochrome C polypeptide in combination with a PET isotope of Magnesium.

In a particular embodiment, the one or more biomolecule complex(es) comprise or consist of a Megalin/Cubulin ligand such as a Cytochrome C polypeptide in combination with a PET isotope of Manganese.

In a particular embodiment, the one or more biomolecule complex(es) comprise or consist of a Megalin/Cubulin ligand such as a Cytochrome C polypeptide in combination with a PET isotope of Oxygen.

In a particular embodiment, the one or more biomolecule complex(es) comprise or consist of a Megalin/Cubulin ligand such as a Cytochrome C polypeptide in combination with a PET isotope of Platinum.

In a particular embodiment, the one or more biomolecule complex(es) comprise or consist of a Megalin/Cubulin ligand such as a Cytochrome C polypeptide in combination with a PET isotope of Sodium.

In a particular embodiment, the one or more biomolecule complex(es) comprise or consist of a Megalin/Cubulin ligand such as a Cytochrome C polypeptide in combination with a PET isotope of Strontium.

In a particular embodiment, the one or more biomolecule complex(es) comprise or consist of a Megalin/Cubulin ligand such as a Cytochrome C polypeptide in combination with a PET isotope of Technetium.

In a particular embodiment, the one or more biomolecule complex(es) comprise or consist of a Megalin/Cubulin ligand such as a Cytochrome C polypeptide in combination with a PET isotope of Uranium.

In a particular embodiment, the one or more biomolecule complex(es) comprise or consist of a Megalin/Cubulin ligand such as a Cytochrome C polypeptide in combination with a PET isotope of fluorine.

In a particular embodiment, the one or more biomolecule complex(es) comprise or consist of a Megalin/Cubulin ligand such as a Cytochrome C polypeptide in combination with a PET isotope of Iodine.

In a particular embodiment, the one or more biomolecule complex(es) comprise or consist of a Megalin/Cubulin ligand such as a Cytochrome C polypeptide in combination with a PET isotope of Copper.

In a particular embodiment, the one or more biomolecule complex(es) comprise or consist of a Megalin/Cubulin ligand such as a Ribonuclease polypeptide in combination with a PET isotope of Aluminium.

In a particular embodiment, the one or more biomolecule complex(es) comprise or consist of a Megalin/Cubulin ligand such as a Ribonuclease polypeptide in combination with a PET isotope of Barium.

In a particular embodiment, the one or more biomolecule complex(es) comprise or consist of a Megalin/Cubulin ligand such as a Ribonuclease polypeptide in combination with a PET isotope of Calcium

In a particular embodiment, the one or more biomolecule complex(es) comprise or consist of a Megalin/Cubulin ligand such as a Ribonuclease polypeptide in combination with a PET isotope of Dysprosium.

In a particular embodiment, the one or more biomolecule complex(es) comprise or consist of a Megalin/Cubulin ligand such as a Ribonuclease polypeptide in combination with a PET isotope of Gadolinium.

In a particular embodiment, the one or more biomolecule complex(es) comprise or consist of a Megalin/Cubulin ligand such as a Ribonuclease polypeptide in combination with a PET isotope of Magnesium.

In a particular embodiment, the one or more biomolecule complex(es) comprise or consist of a Megalin/Cubulin ligand such as a Ribonuclease polypeptide in combination with a PET isotope of Manganese.

In a particular embodiment, the one or more biomolecule complex(es) comprise or consist of a Megalin/Cubulin ligand such as a Ribonuclease polypeptide in combination with a PET isotope of Oxygen.

In a particular embodiment, the one or more biomolecule complex(es) comprise or consist of a Megalin/Cubulin ligand such as a Ribonuclease polypeptide in combination with a PET isotope of Platinum.

In a particular embodiment, the one or more biomolecule complex(es) comprise or consist of a Megalin/Cubulin ligand such as a Ribonuclease polypeptide in combination with a PET isotope of Sodium.

In a particular embodiment, the one or more biomolecule complex(es) comprise or consist of a Megalin/Cubulin ligand such as a Ribonuclease polypeptide in combination with a PET isotope of Strontium.

In a particular embodiment, the one or more biomolecule complex(es) comprise or consist of a Megalin/Cubulin ligand such as a Ribonuclease polypeptide in combination with a PET isotope of Technetium.

In a particular embodiment, the one or more biomolecule complex(es) comprise or consist of a Megalin/Cubulin ligand such as a Ribonuclease polypeptide in combination with a PET isotope of Uranium.

In a particular embodiment, the one or more biomolecule complex(es) comprise or consist of a Megalin/Cubulin ligand such as a Ribonuclease polypeptide in combination with a PET isotope of fluorine.

In a particular embodiment, the one or more biomolecule complex(es) comprise or consist of a Megalin/Cubulin ligand such as a Ribonuclease polypeptide in combination with a PET isotope of Iodine.

In a particular embodiment, the one or more biomolecule complex(es) comprise or consist of a Megalin/Cubulin ligand such as a Ribonuclease polypeptide in combination with a PET isotope of Copper.

### Administration

The mode of administration of the complexes or pharmaceutical compositions comprising the complexes of the invention may be enteral or parenteral, for example orally, sublingually, nasally, by inhalation, injection, intravenously, subcutaneously, rectally, vaginally, cutaneously or transdermally. In preferred embodiments the complexes are administered perorally or intravenously. Compositions comprising or consisting of the complexes may also be administered by enema.

In a preferred embodiment the complexes or pharmaceutical compositions comprising the complexes of the invention are administered intravenously. Examples of intravenous administration include, but are not limited to, injection or via gravity drip. The administration may for example be a bolus injection. Another example of mode of administration may be a short infusion, for example for about 1 minute, such as for 2 to 3 minutes, for example for about 4 minutes, such as for about 5 minutes, for example for about 6 minutes, such as for about 7 minutes, for example for about 8 minutes, such as for about 9 minutes, for example for about 10 minutes, such as for about 12 minutes, for example for about 15 minutes, such as for about 16 minutes, for example for about 17 minutes, such as for about 18 minutes, for example for about 19 minutes, such as for about 20 minutes

In another embodiment of the present invention the complexes and compositions according to the invention are administered perorally. The dosage form can be a solution, a tonic, a tablet, a capsule, a lozenge, a chewable tablet, and the like.

The dosage of the complexes comprise a safe and sufficient amount of the complexes, which is preferably in the range of from about 0.01 mg to about 200 mg per dosage. The dosage may be for example from 1 mg to 10 mg, for example from 1 mg to 9 mg, such as from 1 mg to 8 mg, for example from 1 mg to 7 mg, such as from 1 mg to 6 mg, for example from 1 mg to 5 mg, such as from 1 mg to 4 mg, for example from 1 mg to 3 mg, such as from 1 mg to 2 mg, for example from 2 mg to 10 mg, such as from 2 mg to 9 mg, for example from 2 mg to 8 mg, such as from 2 mg to 7 mg, for example from 2 mg 6 mg , such as from 2 mg to 5 mg, for example from 2 mg to 4 mg, such as from 2 mg to 3 mg, for example from 3 mg to 10 mg, or example from 3 mg to 9 mg, such as from 3 mg to 8 mg, for example from 3 mg to 7 mg, such as from 3 mg to 6 mg, for example from 3 mg to 5 mg, such as from 3 mg to 4 mg, for example from 4 mg to 10 mg, such as from 4 mg to 9 mg, for example from 4 mg to 8 mg, such as from 4 mg to 7 mg, for example from 4 mg to 6 mg, such as from 4 mg to 5 mg, for example from 5 mg to 10 mg, such as from 5 mg to 9 mg, for example from 5 mg to 8 mg, such as from 5 mg to 7 mg, for example from 5 to 6 mg, for example from 6 mg to 10 mg, such as from 6 mg to 9 mg, for example from 6 mg to 8 mg, such as from 6 mg to 7 mg, for example from 7 mg to 10 mg, such as from 7 mg to 9 mg, for example from 7 mg to 8 mg, for example from 8 mg to 10 mg, such as from 8 mg to 9 mg, for example from 9 mg to 10 mg, or from10 mg to 200 mg, for example from 10 mg to 190 mg, such as from 10 mg to 180 mg, for example from 10 mg to 170 mg, such as from 10 mg to 160 mg, for example from 10 mg to 150 mg, such as from 10 mg to 140 mg, for example from 10 mg to 130 mg, such as from 10 mg to 120 mg, for example from 10 mg to 110 mg, such as from 10 mg to 100 mg, for example from 10 mg to 90 mg, such as from 10 mg to 80 mg, for example from 10 mg to70 mg , such as from 10 mg to 60 mg, for example from 10 mg to 50 mg, such as from 10 mg to 40 mg, for example from 10 mg to 30 mg, such as from 10 mg to 20 mg, such as from 20 mg to 200 mg, for example from 20 mg to 190 mg, such as from 20 mg to 180 mg, for example from 20 to 170 mg, such as from 20 to 160 mg, for example from 20 mg to 150 mg, such as from 20 mg to 140 mg, for example from 20 mg to 130 mg, such as from 20 mg to 120 mg, for example from 20 mg to 110 mg, such as from 20 mg to 100 mg, for example from 20 mg to 90 mg, such as from 20 mg to 80 mg, for example from 20 mg to70 mg , such as from 20 mg to 60 mg, for example from 20 mg to 50 mg, such as from 20 mg to 40 mg, for example from 20 mg to 30 mg, such as from 30 mg to 200 mg, for example from 30 mg to 190 mg, such as from 30 mg to 180 mg, for example from 30 to 170 mg, such as from 30 to 160 mg, for example from 30 mg to 150 mg, such as from 30 mg to 140 mg, for example from 30 mg to 130 mg, such as from 30 mg to 120 mg, for example from 30 mg to 110 mg, such as from 30 mg to 100 mg, for example from 30 mg to 90 mg, such as from 30 mg to 80 mg, for example from 30 mg to70 mg , such as from 30 mg to 60 mg, for example from 30 mg to 50 mg, such as from 30 mg to 40 mg, such as from 40 mg to 200 mg, for example from 40 mg to 190 mg, such as from 40 mg to 180 mg, for example from 40 to 170 mg, such as from 40 to 160 mg, for example from 40 mg to 150 mg, such as from 40 mg to 140 mg, for example from 40 mg to 130 mg, such as from 40 mg to 120 mg, for example from 40 mg to 110 mg, such as from 40 mg to 100 mg, for example from 40 mg to 90 mg, such as from 40 mg to 80 mg, for example from 40 mg to70 mg , such as from 40 mg to 60 mg, for example from 40 mg to 50 mg, such as from 50 mg to 200 mg, for example from 50 mg to 190 mg, such as from 50 mg to 180 mg, for example from 50 to 170 mg, such as from 50 to 160 mg, for example from 50 mg to 150 mg, such as from 50 mg to 140 mg, for example from 50 mg to 130 mg, such as from 50 mg to 120 mg, for example from 50 mg to 110 mg, such as from 50 mg to 100 mg, for example from 50 mg to 90 mg, such as from 50 mg to 80 mg, for example from 50 mg to70 mg , such as from 50 mg to 60 mg, such as from 60 mg to 200 mg, for example from 60 mg to 190 mg, such as from 60 mg to 180 mg, for example from 60 to170 mg, such as from 60 to 160 mg, for example from 60 mg to 150 mg, such as from 60 mg to 140 mg, for example from 60 mg to 130 mg, such as from 60 mg to 120 mg, for example from 60 mg to 110 mg, such as from 60 mg to 100 mg, for example from 60 mg to 90 mg, such as from 60 mg to 80 mg, for example from 60 mg to70 mg , such as from 70 mg to 200 mg, for example from 70 mg to 190 mg, such as from 70 mg to 180 mg, for example from 70 to170 mg, such as from 70 to 160 mg, for example from 70 mg to 150 mg, such as from 70 mg to 140 mg, for example from 70 mg to 130 mg, such as from 70 mg to 120 mg, for example from 70 mg to 110 mg, such as from 70 mg to 100 mg, for example from 70 mg to 90 mg, such as from 70 mg to 80 mg, such as from 80 mg to 200 mg, for example from 80 mg to 190 mg, such as from 80 mg to 180 mg, for example from 80 to170 mg, such as from 80 to 160 mg, for example from 80 mg to 150 mg, such as from 80 mg to 140 mg, for example from 80 mg to 130 mg, such as from 80 mg to 120 mg, for example from 80 mg to 110 mg, such as from 80 mg to 100 mg, for example from 80 mg to 90 mg, such as from 90 mg to 200 mg, for example from 90 mg to 190 mg, such as from 90 mg to 180 mg, for example from 90 to 170 mg, such as from 90 to 160 mg, for example from 90 mg to 150 mg, such as from 90 mg to 140 mg, for example from 90 mg to 130 mg, such as from 90 mg to 120 mg, for example from 90 mg to 110 mg, such as from 90 mg to 100 mg, such as from 100 mg to 200 mg, for example from 100 mg to 190 mg, such as from 100 mg to 180 mg, for example from 100 to 170 mg, such as from 100 to 160 mg, for example from 100 mg to 150 mg, such as from 100 mg to 140 mg, for example from 100 mg to 130 mg, such as from 100 mg to 120 mg, for example from 100 mg to 110 mg, such as from 110 mg to 200 mg, for example from 110 mg to 190 mg, such as from 110 mg to 180 mg, for example from 110 to 170 mg, such as from 110 to 160 mg, for example from 110 mg to 150 mg, such as from 110 mg to 140 mg, for example from 110 mg to 130 mg, such as from 110 mg to 120 mg, such as from 120 mg to 200 mg, for example from 120 mg to 190 mg, such as from 120 mg to 180 mg, for example from 120 to170 mg, such as from 120 to 160 mg, for example from 120 mg to 150 mg, such as from 120 mg to 140 mg, for example from 120 mg to 130 mg, such as from 130 mg to 200 mg, for example from 130 mg to 190 mg, such as from 130 mg to 180 mg, for example from 130 to 170 mg, such as from 130 to 160 mg, for example from 130 mg to 150 mg, such as from 130 mg to 140 mg, such as from 140 mg to 200 mg, for example from 140 mg to 190 mg, such as from 140 mg to 180 mg, for example from 140 to 170 mg, such as from 140 to 160 mg, for example from 140 mg to 150 mg, such as from 150 mg to 200 mg, for example from 150 mg to 190 mg, such as from 150 mg to 180 mg, for example from 150 to 170 mg, such as from 150 to 160 mg, such as from 160 mg to 200 mg, for example from 160 mg to 190 mg, such as from 160 mg to 180 mg, for example from 160 to 170 mg, such as from 170 mg to 200 mg, for example from 170 mg to 190 mg, such as from 170 mg to 180 mg, such as from 180 mg to 200 mg, for example from 180 mg to 190 mg, such as from 190 mg to 200 mg,

In one embodiment of the present invention a carrier is comprised within the composition to be used as a contrast agent. Carriers suitable for the preparation of unit dosage forms for peroral administration are well-known in the art.

Peroral compositions also include liquid solutions, emulsions, suspensions, and the like. The carriers suitable for preparation of such compositions are well known in the art. Liquid oral compositions preferably comprise from about 0.001 % to about 5% of the active complex.

Other compositions useful for attaining systemic delivery of the complexes include sublingual and buccal dosage forms. Such compositions typically comprise soluble filler substances and binders, as well as optional glidants, lubricants, sweeteners, colorants, antioxidants and flavoring agents may also be included.

In one embodiment the present invention relates to compositions comprising filler substances and/or binders.

In one embodiment, the composition is administered to a human being.

In another embodiment, the composition is administered to an animal, such as a pet or a laboratory animal.

### Combinations of methods

The biomolecule complexes of the present invention can further be used for PET imaging-based methods, in which the PET imaging method is combined with other imaging methods, such as for example in PET-CT or PET-MRI.

Positron emission tomography - computed tomography (also know as PET-CT) is a medical imaging device which combines in a single gantry system both a Positron Emission Tomography (PET) and an x-ray Computed Tomography, so that images acquired from both devices can be taken sequentially, in the same session from the patient and combined into a single superposed (co-registered) image. Thus, functional imaging obtained by PET, which depicts the spatial distribution of metabolic or biochemical activity in the body can be more precisely aligned or correlated with anatomic imaging obtained by CT scanning. Two- and three-dimensional image reconstruction may be rendered as a function of a common software and control system.

The biomolecule complexes according to the present invention can be used for PET-CT analysis for example, in oncology, surgical planning, radiation therapy and cancer staging.

CT perfusion studies have been useful in evaluating viable tumour tissue, which normally shows increased perfusion. CT perfusion is further beneficial as it can quantify blood volume, blood flow and tumour transit time directly. This is of great help in follow up studies of tumours on different therapeutic regimes.

In one embodiment high resolution CT of a desired organ is obtained with superimposition of PET images on underlying anatomical data, leading to unparalleled imaging acquisition.

PET-CT is well established in diagnosis, staging and follow-up of e.g. colorectal cancer, oesophageal malignancies, lymphomas, lung cancer, melanomas, breast malignancy, head and neck tumours and in characterisation of a pulmonary nodule. In addition, CT-PET has been found invaluable in accurate localization of very small areas of increased traced activity.

In one embodiment, the method according to the present invention does not comprise CT imaging techniques.

In one embodiment the present invention relates to PET-MRI as previously described above.

In one embodiment, the method according to the present invention does not comprise MR imaging techniques.

### References

Asarod,K, Iversen B.M, Hammerstrsm J, and Bostad, L and Jsrstad, S. Clinical outcome inpatientsw ith Wegnersg raulomatosistr eatedw ith plasmapheresis Blood Purification 20:167-173.2002
AUKLAND, K., TENSTAD, O. & WIIG, H. (2001). Distribution spaces for hyaluronan andalbumin in rat tail tendons. Am J Physiol Heart Circ Physiol 281, H1589-1597.
BAKOUSH, O., TENCER, J., TORFFVIT, O., TENSTAD, O., SKOGVALL, I. & RIPPE, B. (2004).Increased glomerular albumin permeability in old spontaneously hypertensive rats. Nephrol Dial Transplant 19, 1724-1731.
Baran D, Tenstad O, Aukland K: Aprotinin uptake in the proximal tubules in the rat kidney. II. Uptake site relative to glomerulus. J Struct Biol 142:409-415, 2003a
Baran D, Tenstad O, Aukland K: Localization of tubular uptake segment of filtered Cystatin C and Aprotinin in the rat kidney. Acta Physiol (Oxf) 186:209-221, 2006
Bivol LM, VagnesO B, IversenB M.The renal vascularr esponseto ANG II injection isreduced in the non-clipped kidney of two-kidney, one clip hypertension.Am J Physiol Renal Physiol. Am J Physiol Renal Physiol. A;289(2):F393-400, 2005
BLETSA, A., BERGGREEN, E., FRISTAD, I., TENSTAD, O. & WIIG, H. (2006). Cytokine signalling in rat pulp interstitial fluid and transcapillary fluid exchange during lipopolysaccharide-induced acute inflammation. J Physiol 573, 225-236.
Chang, Y.S. et al. Preparation of 18F-human serum albumin: A simple and efficient protein labelling method with 18F using hydrazone-formatin method. Bioconjugate Chem. 2005, 16: 1329-33.
Choyke PL, Kobayashi H "Functional magnetic resonance imaging of the kidney using macromolecular contract agents" Abdom Imaging. 2006 Mar-Apr, 31(2): 224-31.
Christiansen RE, Roald AB, Gjerstad C, et al.: Renal hemodynamics in young and old spontaneously hypertensive rats during intrarenal infusion of arginine vasopressin.Kidney Blood Press Res 24:176-184, 2001
Christiansen RE, Roald AB, Tenstad O, et al.: Renal hemodynamics during development of hypertension in young spontaneously hypertensive rats. Kidney Blood Press Res 25:322-328, 2002
Christiansen RE, Tenstad O, Leh S, et al.: Glomerular charge selectivity is impaired in hypertensive nephropathy. Nephrol Dial Transplant 19:1083-1091, 2004
Christiansen, R F, Roald, A, Tenstad O and Iversen B M. Renal hemodynamics in young rats with genetich ypertdnsion.K idney and Blood PressureR eserarch2. 5: 322-328,2002.
ChristiansenR .E, TenstadO, Leh S and IversenB M. Glomerularc harges electivity impaired in hypertensiven ephropathy.N ephrol Dialysis Transplantation: 1 9: I 083-109I, 2004
ERGA, K. S., PEEN, E., TENSTAD, O. & REED, R. K. (2000). Lactoferrin and antilactoferrinantibodies: effects of ironloading of lactoferrin on albumin extravasation in different tissues in rats. Acta Physiol Scand 170, 11-19.
Erik Ilsø Christensen & Henrik Birn, Nature Reviews Molecular Cell Biology 3, 258-268, April 2002
Ersver, E, Bertelsen,L -T, Espenes,L.CB, redholt,T,BAe, SO, Iversen,B M, Bruserud,O,Ulvestad,E , Gjerts-enB, T. Characterizationo f ribosomalP autoantibodiesin relation to cell destruction and autoimmune diseaqe. Scand. Jour. Immunology 60,189-198,2004.
Fraker PJ and Speck JC Jr. Protein and cell membrane iodinations with a sparingly soluble chloroamide, 1,3,4,6-tetrachloro-3a,6a-diphrenylglycoluril. Biochem Biophys Res Commun 80: 849-857, 1978
Gill, H.S. et al. A modular platform for the rapid side-specific radiolabeling of proteins with 18F exemplified by quantitative positron emission tomography of human epidermal growth factor receptor J.Med.Chem. 2009, 52: 5816-25.
GYENGE, C. C., TENSTAD, O. & WIIG, H. (2003). In vivo determination of steric and electrostatic exclusion of albumin in rat skin and skeletal muscle. J Physiol 552, 907-916.
HansenF H, VagnesO B, IversenB M.Enhancedr esponseto argininev asopressin( AVP) in the interlobular artery (ILA) from the spontaneously hypertensive rat (SHR). Am J Physiol RenalPhysiol 288 (6) :F ]oa9 - 5 6,2005 .
Helle, F, Vagnes O and Iversen BM: Angiotensin II indiced calcium- signalling in-twokidney -one clip hypertension Amer J. Physiolol, Renal, 2006
Hodson, C.J. 1986 The pig as a model for studying kidney disease in man. In: Swine in Biomedical Research. Plenum, New York, pp. 1691-1704.
Kobayashi et al: Detection of lymph node involvement in hematologic malignances using micromagnetic resonance lymphangiography with a gadolinium labelled dendrimer nanoparticles" Neoplasia. 2005 Nov, 7(11):984-91)
KVEINE, M., TENSTAD, E., DOSEN, G., FUNDERUD, S. & RIAN, E. (2002). Characterization of the novel human transmembrane protein 9 (TMEM9) that localizes to lysosomes and late endosomes. Biochem Biophys Res Commun 297, 912-917.
Leh S, Vaagnes O, Margolin SB, Iversen BM, Forslund T.Pirfenidone and candesartanameliorate morphological damage in mild chronic anti-GBM nephritis in rats.Nephrol DialTransplant 20(1:)7 1-82, 2005.
LUND, U., RIPPE, A., VENTUROLI, D., TENSTAD, O., GRUBB, A. & RIPPE, B. (2003). Glomerular filtration rate dependence of sieving of albumin and some neutral proteins in rat kidneys. Am J Physiol Renal Physiol 284, F1226-1234.
Manjunath G, Tighiouart H, Coresh J, et al.: Level of kidney function as a risk factor for cardiovascular outcomes in the elderly. Kidney Int 63:1121-1129, 2003a
Manjunath G, Tighiouart H, Ibrahim H, et al.: Level of kidney function as a risk factor for atherosclerotic cardiovascular outcomes in the community. J Am Coll Cardiol 41:47-55, 2003b
Mindt, T.L. et al. A "click chemistry" approach to the efficient synthesis of multiple imagnig probes derived from a single precursor. Biojonjugate Chem. 2009, 20:1940-49.
NEGRINI, D., TENSTAD, O. & WIIG, H. (2003). Interstitial exclusion of albumin in rabbit lung during development of pulmonary oedema. J Physiol 548, 907-917.
NEGRINI, D., TENSTAD, O. & WIIG, H. (2003). Interstitial exclusion of albumin in rabbit lung measured with the continuous infusion method in combination with the wick technique. Microcirculation 10, 153-165.
NEGRINI, D., TENSTAD, O., PASSI, A. & WIIG, H. (2006). Differential degradation of matrix proteoglycans and edema development in rabbit lung. Am J Physiol Lung Cell Mol Physiol 290, L470-477.
Ofstad J, Iversen BM. Glomerular and tubular damage in normotensive and hypertensive rats.Am J Physiol Renal Physiol. ;288(a) :F665- 7 2, 2005.
Roald AB, Aukland K, Tenstad O: Tubular absorption of filtered cystatin-C in the rat kidney. Exp Physiol 89:701-707, 2004a
Roald AB, Tenstad O, Aukland K: The effect of AVP-V receptor stimulation on local GFR in the rat kidney. Acta Physiol Scand 182:197-204, 2004b
Roald AB, Tenstad O, Aukland K: The effect of AVP-V2 receptor stimulation on local GFR in the rat kidney. Acta Physiol Scand 168:351-359, 2000
Roald, A, Ofstad, J and Iversen B.M. Attenuated buffering of renal pressure variation in juxtamedullaryc ortex in the spontaneouslyh ypertensiver at. Am. J.Physiol Renal Physio1282: F506-F511. 2002.
ROSENGREN, B. I., RIPPE, B., TENSTAD, O. & WIIG, H. (2004). Acute peritoneal dialysis in rats results in a marked reduction of interstitial colloid osmotic pressure. J Am Soc Nephrol 15, 3111-3116.
Rytand, D.A. 1938 The number and size of mammalian glomeruli as related to kidney and to body weight, with methods for their enumeration and measurement. Am. J. Anat., 62507-520.
Strommen, K., Stormark ,TA., Iversen, BM. and Matre K. Volume estimation of small phantoms and rat kidneys using three-dimensional ultrasonography and position sensor.30(9), 2004
Svarstad, E and Iversen B.M. Renal artery thombosis and acute renal failure after ACE inhibition. Nephrology Dialysis and Transplation 2004: 5 65.
Svarstad,E , Urheim L and B.M.Iversen:C ritical renal artery stenosism ay causeaspectrum of cardiorenal failure and associated thromboembolic events. Clinical Nephrology63, 487-492, 2005.
Svarstad,E., BostadL .,Kaabae, A,Houge,G., Tsndel,C.,L yngdal,P.T.a nd Iversen,B.M:Focal and segmental glomerular sclerosis (FSGS) in a man and a woman with Fabry'sDisease Clinical nephrology 63: 394-400, 2005
Svarstad,E, IversenB M and Bostad,L . Bedsides tereomicroscopyo f renal biopsiesm ay leadt o a rapidd iagnosiso f Fabry'sd isease. Nephrol.Dial.Transplant.;1 9: 3202 - 3203,2004.
SvarstadE, , Myking O, OfstadJ , and IversenB . M. Effect of light excersiseo n renal hemodynamicsin patientsw ith hjpertension and chronic renal disease. Scan J Urology Nephrology.3 6: 6, 464-473,2002
SvarstadE, Hultstroffi,D.,Jensen,D.,Jenssen,G.,Iversen,B.M. Renal artery thrombosis with acute failure after withdrawal of angiotensin converting enzyme inhibitor: a case report. Nephology Dialysis Transplantation 17:| -2, 2002.
Tenstad O, Roald AB, Grubb A, et al.: Renal handling of radiolabelled human cystatin C in the rat. Scand J Clin Lab Invest 56:409-414, 1996
Tenstad O, Williamson HE, Aukland K: Repeatable measurement of local and zonal GFR in the rat kidney with aprotinin. Acta Physiol Scand 152:21-31, 1994a
Tenstad O, Williamson HE, Clausen G, et al.: Glomerular filtration and tubular absorption of the basic polypeptide aprotinin. Acta Physiol Scand 152:33-50, 1994b
TENSTAD, O., HEYERAAS, K. J., WIIG, H. & AUKLAND, K. (2001). Drainage of plasma proteins from the renal medullary interstitium in rats. J Physiol 536, 533-539.
TORLAKOVIC, E., TENSTAD, E., FUNDERUD, S. & RIAN, E. (2005). CD10+ stromal cells form B-lymphocyte maturation niches in the human bone marrow. J Pathol 205, 311-317.
Treeck B, Aukland K. Effect of L-NAME on glomerular filtration rate in deep and superficial layers of rat kidneys. Am J Physiol. 1997 Mar;272(3 Pt 2):F312-8.
Treeck B, Roald AB, Tenstad O, Aukland K. Effect of exogenous and endogenous angiotensin II on intrarenal distribution of glomerular filtration rate in rats. J Physiol. 2002 Jun 15;541(Pt 3):1049-57.
US 7,048,097 4. US 7,048,907 B2 (Groman) Synthesis, compositions and methods for the measurement of the concentration of stable-isotope labeled compounds in life forms and life form excretory products.
Vagnes O, HansenF H, ChristiansenR E, Gjerstad,C and IversenB M: Age regulationo f the V1a receptori n rats with genetich ypertension. Amer. J. Physiol 286:F997-F1003, 2004.
VagnesO, HansenF H, Feng JJ, IversenB M, ArendshorstW J.Enhance d Ca2+responset oAVP in preglomerular vessels from rats with genetic hypertension during different hydrationStates Am J Physiol R enal Physiol. 88(5):F1023-31, 2005.
Vikse BE, Vollset SE, Tell GS, Refsum H, Iversen BM. (2004). Distribution and determinants of serum creatinine in the general population: the Hordaland Health Study. Scand J Clin LabInvest, 64,1-14,2004.
Vikse, B E, Aasarod K, Bostad L and Iversen BM. Prognostic factors in.biopsy proven benign nephrosclerosisN. ephrology Dialysis and Transplantation:2 003: 18: 517-23
Vikse, B E, Bostad, L, Aasarod,K, Dag Einar Lysebo, D E, and Iversen, BM. Prognostic factor in mesangioprqliferative glomerulonephritis. Nephrology Dialysis and transplantation 18: 17-23,2002.
Vikse, B.E., Irgens,L., Bostad, L. and Iversen B.M.. Adverse perinatal outcome and latekidney diseasein the mother. J. Amer. Soc Nephrology. 2006
Vimtrup, B. 1928 On the number, shape, structure and surface area of the glomeruli in the kidney of man and mammals. Am. J. Anat., 41:123-151.
Wang X, Aukland K, Bostad L, et al.: Autoregulation of total and zonal glomerular filtration rate in spontaneously hypertensive rats with mesangiolysis. Kidney Blood Press Res 20:11-17, 1997a
Wang X, Aukland K, Iversen BM: Acute effects of angiotensin II receptor antagonist on autoregulation of zonal glomerular filtration rate in renovascular hypertensive rats. Kidney Blood Press Res 20:225-232, 1997b
Wang X, Aukland K, Iversen BM: Autoregulation of total and zonal glomerular filtration rate in spontaneously hypertensive rats during antihypertensive therapy. J Cardiovasc Pharmacol 28:833-841, 1996
Wang X, Aukland K, Ofstad J, et al.: Autoregulation of zonal glomerular filtration rate and renal blood flow in spontaneously hypertensive rats. Am J Physiol 269:F515-521,1995
Wängler, C. et al. Simple and convenient radiolabeling of proteins using a prelabeling-approach with thiol-DOTA. Bioorg.Med.Chem. 2009, 19:1926-29.
Wester, H-J. et al. A comparative study of N.C.A. fluorine-18 labeling of proteins via acylation or photochemical conjugation Nucl.Med.Biol. 1996, 23: 365-72.
WIIG, H. & TENSTAD, O. (2001). Interstitial exclusion of positively and negatively charged IgG in rat skin and muscle. Am J Physiol Heart Circ Physiol 280, H1505-1512.
WIIG, H., AUKLAND, K. & TENSTAD, O. (2003). Isolation of interstitial fluid from rat mammary tumors by a centrifugation method. Am J Physiol Heart Circ Physiol 284, H416-424.
WIIG, H., GYENGE, C. C. & TENSTAD, O. (2005). The interstitial distribution of macromolecules in rat tumours is influenced by the negatively charged matrix components. J Physiol 567, 557-567.
WIIG, H., KOLMANNSKOG, O., TENSTAD, O. & BERT, J. L. (2003). Effect of charge on interstitial distribution of albumin in rat dermis in vitro. J Physiol 550, 505-514.
WIIG, H., REED, R. K. & TENSTAD, O. (2000). Interstitial fluid pressure, composition of interstitium, and interstitial exclusion of albumin in hypothyroid rats. Am JPhysiol Heart Circ Physiol 278, H1627-1639.
WIIG, H., TENSTAD, O. & BERT, J. L. (2005). Effect of hydration on interstitial distribution of charged albumin in rat dermis in vitro. J Physiol 569, 631-641.

### Examples

### Example 1: Gd-DTPA-Aprotinin protocol

### Materials:

● DTPA (Diethylenetriaminepentaacetic acid dianhydride, Sigma D-6148, C₁₄H₁₉N₃O₈, molecular weight 357.32).
● aprotinin from bovine lung, Sigma A4529 lyophilized powder, 3-7 TIU/mg solid, molecular weight 6511.44).
● HEPES (Sigma, H7523, 4-(2-Hydroxyethyl)piperazine-1-ethanesulfonic acid, C₈H₁₈N₂O₄S, molecular weight 238.30).
● Citric acid (Sigma C0759, HOC(COOH)(CH₂COOH)₂ molecular weight 192.12).
● Citrate tribasic dehydrate (Sigma S4641, HOC(COONa)(CH₂COONa)₂ · 2H₂O, molecular weight 294.10).
● DMSO (Sigma Dimethyl sulfoxide, D8418, (CH₃)₂SO, molecular weight 78.13).
● Dialysis Membrane (Spectra/Por 3 Tubing: 3.5k MWCO Regenerated Cellulose, Cat.No.: 132720).
● NTA, (Sigma, N0128, Nitrilotriacetic acid disodium salt, C₆H₇NO₆Na₂, molecular weight 235.10
● GdCl₃ (MP Biomedicals Cat.No.: 203712, gadolinium chloride, Cl₃GdH₁₂O₆, molecular weight: 371.7).
● Gadolinium-153 Radionuclide in 0.5N HCl (PerkinElmer NEZ142001MC)

### Day 1: Production of DTPA-aprotinin.

Dissolve 11 mg aprotinin in 250 µl 0,1M HEPES buffer at pH 8.8.

Dissolve 0.48g DTPA in 1 ml DMSO.

Add 25µl of the DMSO/DTPA Solution to the Aprotin solution and dialyse overnight against 1000 ml 0.1M citrate buffer adjusted to pH 6.5 by using 1% Citric acid.

### Day 2: Second dialysis

Change to fresh citrate buffer.

### Day 3: Third dialysis

Change to fresh citrate buffer.

### Day 4: Production of Gd-DTPA-aprotinin.

Make Gd(NTA)₂ by dissolving 260 mg GdCl₃ in 4 ml pH 1.0 HCl. Ad 100µCi Gadolinium-153 if biological screening using gamma detection is desired.

Add 494 mg NTA (Get white precipitate). Add 3M NaOH dropwise until pH is 4.9.

Add 20 µl Gd(NTA)₂ to the dialysed DTPA-aprotinin solution with stirring.

Continue stirring in for 3h and dialyse overnight against 1000 ml 0.1M citrate buffer as described above.

### Day 5: Second dialysis

Change to fresh citrate buffer.

### Day 6: Third dialysis

Change to fresh citrate buffer.

Gd-DTPA-aprotinin is stable in citrate buffer at 4-8°C for at least 3 months.

### Example 2: Gd-DTPA-Lysozyme protocol

### Materials:

● DTPA (Diethylenetriaminepentaacetic acid dianhydride, Sigma D-6148, C14H19N3O8, molecular weight 357.32).
● Lysozyme from chicken egg white, Sigma L6876, lyophilized powder, molecular weight 14.7 kDa.
● HEPES (Sigma, H7523, 4-(2-Hydroxyethyl)piperazine-1-ethanesulfonic acid, C8H18N2O4S, molecular weight 238.30).
● Citric acid (Sigma C0759, HOC(COOH)(CH2COOH)2 molecular weight 192.12).
● Citrate tribasic dehydrate (Sigma S4641, HOC(COONa)(CH2COONa)2 • 2H2O, molecular weight 294.10).
● DMSO (Sigma Dimethyl sulfoxide, D8418, (CH3)2SO, molecular weight 78.13).
● Dialysis Membrane (Spectra/Por 3 Tubing: 3.5k MWCO Regenerated Cellulose, Cat.No.: 132720).
● NTA, (Sigma, N0128, Nitrilotriacetic acid disodium salt, C6H7NO6Na2, molecular weight 235.10
● GdCl3 (MP Biomedicals Cat.No.: 203712, gadolinium chloride, Cl3GdH12O6, molecular weight: 371.7).
● Gadolinium-153 Radionuclide in 0.5N HCl (PerkinElmer NEZ142001MC)

### Day 1: Production of DTPA-Lysozyme

Dissolve 220 mg Lysozyme in 5 ml 0,1M HEPES buffer at pH 8.8.

Dissolve 0.48g DTPA in 1 ml DMSO.

Add 100µl of the DMSO/DTPA solution to the Lysozyme solution and adjust pH to 8.8. Repeat this step five times so that totally 500µl DSMO/DTPA is added. Dialyse overnight against 1000 ml 0.1M citrate buffer adjusted to pH 6.5 by using 1% Citric acid.

### Day 2: Second dialysis

Change to fresh citrate buffer.

### Day 3: Third dialysis

Change to fresh citrate buffer.

### Day 4: Production of Gd-DTPA-Lysozyme

Make Gd(NTA)2 by dissolving 260mg GdC13 in 4 ml pH 1.0 HCl. Ad 100µCi Gadolinium-153 if biological screening using gamma detection is desired.

Add 494 mg NTA (get white precipitate). Add 3M NaOH dropwise until pH is 4.9.

Add 400 µl Gd(NTA)2 to the dialysed DTPA-Lysozyme solution with stirring.

Continue stirring in for 3h and dialyse overnight against 1000 ml 0.1M citrate buffer as described above.

### Day 5: Second dialysis

Change to fresh citrate buffer.

### Day 6: Third dialysis

Change to fresh citrate buffer.

Gd-DTPA-Lysozyme is stable in citrate buffer at 4-8°C for at least 3 months.

### Example 3: Study of glomerular filtration rate in a patient

In one example, provided in order to illustrate and not to limit, a complex comprising aprotinin linked to a PET marker is administered intravenously to a patient and PET imaging performed. Imaging visualises the glomerular filtration rate of said patient. Subsequently, a complex comprising lysozyme linked to a paramagnetic marker is administered intravenously to the same patient and PET imaging performed. The images acquired in the two scans are compared. Deficient uptake of complex comprising a lysozyme derivative (tubular injury inhibits absorption of this complex) in the absence of deficiency of the complex comprising aprotinin (absorbed even by injured tubules) indicates damage (e.g. after acute ischemia) locally to tubuli which has not yet resulted in lowered glomerular filtration rate.

### Example 4: Study of alterations of intrarenal distribution og GFR in a patient.

In another illustrative example aprotinin linked to a PET marker is administered intravenously to a patient suffering from unilateral renal artery stenosis in order to measure the intracortical distribution of glomerular filtration rate in the two kidneys. Unilateral renal artery stenosis is known to result in early perturbations of the nephrons in outer cortical layers of the stenotic kidney. The inner cortical nephrons of the contra lateral kidney exposed to hypertension are on the other hand the first to develop glomerulosclerosis. Thus, the intracortical profile of local glomerular filtration rate (Fig 14) in the two kidneys, being similar in healthy subjects and altered in opposite directions in unilateral renal artery stenosis, would allow very early detection of functional abnormality and help the clinicians to decide when surgical intervention is favourable.

### Example 5: Proof of concept

### Materials

The PET-CT hardware used to obtain the data in the figures described below was a Siemens Biograph 40, TruePoint PET/CT with the following settings for static recordings (Pig1 and 2): CT: KV120, eff.mas: 55; slice: 5,0mm; collimator: 24x1,2mm; pitch: 1,4; PET: Filter Gaussian, FWHM(mm)5, Iterations 4, Subsets 8, Image size 168. For dynamic recordings: CT: KV120, eff.mas: 170; slice: 5,0mm; collimator: 24x1,2mm; pitch: 0,8; PET: Acquisition time: 60 min (Pig3) or 20min (Pig4) in list mode covering both kidneys (one bed, 55 slices of 0.5 mm = 27.5 cm). Reconstruction: FWHM 5mm, zoom: 1; Gaussian-filter. Iterations 4, Subsets 8, Image size 168. CT software was Somaris 5 and PET software was PETSyngo 6.0.1. Exported DICOM images were further processed using OsiriX - DICOM Viewer version 3.6.1 for Mac OS X.

### Making the PET scan probe for monitoring local glomerular filtration rate

For the purpose of establishing proof of concept of the invention, the basic polypeptide aprotinin was chosen due to its high specificity to the target cells (accumulation in renal cortex is only determined by glomerular filtration) and because of its slow degradation within the proximal tubular cells [Tenstad et al 1994a,b]. However, any probe that is freely filtered and quantitatively retained in tubular cells close to the feeding glomerulus for at least 1-2 minutes is feasible provided that it can be labeled by a positron emitting isotope. Thus, for diagnostic purposes, particularly for diagnosing perturbations of glomerular function, an endogenous macromolecule like cystatin C that is more rapidly degraded and cleared from the body [Tenstad et al 1996] may be desired due the less risk of side effects.

Aprotinin (Ap, Sigma-Aldrich, catalog number A1153) was labeled with 124I by using 1,3,4,6-tetrachloro-3α,6 α-diphenylglycouril (Iodo-Gen) (1). Briefly, 0.1 mg Iodo-Gen (T0656,Sigma-Aldrich) dissolved in 0.1 ml chloroform was dispersed in a 1.8-ml Nunc vial (Nunc-Kamstrup, Roskilde, Denmark). A film of the virtually water-insoluble Iodo-Gen was formed in the Nunc vial by allowing the chloroform to evaporate to dryness under nitrogen. Then, 1.5 ml 0.05 M phosphate buffer, pH 7.5, containing 1-2 mg Ap, 20-170 MBq 124I (IBA Molecular), and 15 µl 0.01 M NaI were added, and the iodinating tube was gently agitated for 10 minutes before the reaction was terminated by removing the solution from the Iodo-Gen tube. The stock solution diluted with 3 ml Ringer-Lactate and stored in the dark at 4°C. Low molecular weight radioactivity accounting for 10-25% of the total activity was removed before use by filtration (Millipore - Amicon® Ultra-4 Centrifugal Filter Units) and the desired amount of 124I-Ap (17-133 mBq) was resuspended in a total volume of 4 ml Ringer-Acetate. 124I-Ap was validated by ion exchange and size exclusion chromatography and found to have the same elution pattern as unlabelled Ap.

### The animal model

The farm pig was used since it possesses a kidney that closely resembles the structural and functional features of the human kidney. Kidneys from both humans and pigs are classified as multipapillary with an identical papillary and calyceal organization [Hodson, 1986], and the adult organs have similar weight, size, and number of nephrons [Vimtrup, 1928; Rytand, 1938]. The four pigs (25-27 kg bodyweight) used in the examples described below were housed at the Vivarium, the animal facititiy at Haukeland University Hospital, Bergen, Norway 1-2 weeks prior to the experiments. They were sedated by an intramuscular injection of 500 mg Ketamine, 15 mg Midazolam and 1 mg Atropin and anaesthetized by 4% isoflurane (induction) and Propofol, 4-10 mg per kilogram of body weight per hour intravenously (maintenance) into a cannulated (Braunule, 16-18 gauge) marginal ear vein. A contralateral marginal ear vein was cannulated for infusion of Ringer-Lactate (0.5-1 liter per hour) and injection of CT-contrast (Omnipaq 60ml) and 124I-aprotinin (17-133 Mbq). The pigs were then tracheostomized and ventilated (Siemens Servo 900) until they were euthanized by an intravenous injection of 50 gram Pentobarbital.

All experiments were performed in accordance with recommendations given by the Norwegian State Commission for Laboratory Animals and were approved by the ethical committee at the Department of agriculture of Norway.

### Experimental protocol

Following an initial CT scan, a calibrated dose of 133 Mbq of 124I-aprotinin was injected intravenously into the first pig (pig 1) and several PET scans covering 1-6 beds (1 bed = 27.5 cm), acquisition time ranging from 15 seconds to 3 minutes were performed (Fig 9). In two pigs (pig 2 and pig 3), the lower polar artery, a branch from the left renal artery supplying the inferior 1/3-1/2 of the kidney, was ligated to induce localized renal failure. Similar PET-CT scans as described above for Pig 1 was obtained in Pig 2, but the 124I-Aprotinin dose was reduced from 133 Mbq to 47 Mbq (Fig 10). In pig 3 CT-contrast (60ml Omnipaque, 240 mg/ml) was infused to visualize the vasculature and urinary spaces. 30 Mbq 124I-aprotinin was injected and dynamic list mode PET scans were obtained in a period of 60 minutes (Figs 11-13 and Fig 14, panels C, E, G). In another pig (pig 4) unilateral acute urinary obstruction was induced by ligating the left ureter. The right femoral artery of this animal was cannulated to allow frequent sampling of blood during dynamic PET recordings in a period of 20 minutes following injection of 17 Mbq 124I-aprotinin (Fig 14, Panel A,B,D and F).

### Results

Figure 9 shows the high selectivity of aprotinin as a targeting biomolecule for glomerular filtration rate in the renal kidney cortex. Aprotinin is filtered by the glomeruli and accumulated quantitatively in tubular cells close to the parent glomeruli for 20-30 minutes after i.v. injection. Therefore, linking this biomolecule as in this example by a PET isotope allowed direct visualization and calculation of local glomerular filtration in subpopulations of nephrons throughout the renal cortex. A superior sensitivity and signal to noise ratio is obtained since the complex is accumulated in the target compartment as compared to conventional GFR markers that are excreted in the urine and therefore rapidly removed from the target compartment.

Note that recording after extended period of 60 minutes after i.v. tracer injection results in degradation of the targeting biomolecule in the lysosomes of the tubular cells, release of free marker (124I) to the blood stream and subsequent uptake of free iodine marker in thyroid gland and urinary excretion shown by high local activity in the neck and suprabubic region respectively. The diffuse uptake in liver most likely represents removal of radiolabelled impurities and aggregates by the Kupffer cells.

Figure 10 and 11 illustrate the ability and high sensitivity of the targeting biomolecule to detect local defects in glomerular filtration rate following ligation of a branch of the renal artery. The uptake of 124I-Aprotinin is strictly confined to renal cortex containing filtering nephrons and the marker intensity is directly related to function, i.e. the amount of marker being filtered by the glomeruli at any area of interest in the kidney cortex.

The data in figures 12 to 14 document that the targeting biomolecule permits detailed quantitative information of local GFR using PET. Following i.v. injection there was an initial rapid increase of 124I-aprotinin in the target compartments leveling off about 10 minutes after i.v. injection (Figure 12A-B) reflecting the rapid decay in plasma (Figure 12D). The clearance of 124I-aprotinin from plasma to target compartment equals Q/P where Q = 124I-aprotinin activity in target compartment and P = time integrated plasma activity (area under the plasma activity curve). Q/P is therefore a direct measure of glomerular filtration rate in nephrons within the target compartment.

Note that glomerular filtration rate is underestimated in clearance periods of more than 20-30 minutes duration (Figure 13A) due to intratubular degradation of the biomolecule. The optimal clearance period is therefore of about 1 minute to about 20 minutes duration using aprotinin and less than 5 minutes using complexes like cystatin C that are more rapidly degraded within the tubular cells (Tenstad et al 1996).

Ligation of a branch of the renal artery resulted, as expected, in loss of function in its feeding area with a relatively sharp demarcation zone (Figure 12C and Figure 13B). Interestingly, glomerular filtration seemed best maintained in inner cortex of the ischemic section C (Figure 13) which may relate to structural and functional differences between juxtamedullary nephrons and the more superficially located nephrons. This observation was confirmed by the data in Figure 14 where serial sampling of glomerular filtration rate from superficial to deep kidney cortex revealed perturbations in regional glomerular filtration rate after acute ischemia as well as in acute hydronephrosis. These findings are to the best knowledge of the inventor not known in the art, most likely due to methodological limitations of presently available methods.

Figure 14 also clearly demonstrates the ability of the present technology to quantify total and local glomerular filtration rate in a non-urine producing kidney (Figure 14 A,B,D,F).

### Preferred embodiments

1. A biomolecule complex for use as a contrast agent in positron emission tomography-based imaging methods, said complex comprising a biomolecule and a marker.
2. The complex according to embodiment 1 wherein the biomolecule is coupled to the marker via a linker.
3. The complex according to embodiment 1 wherein the biomolecule linked to the marker forms supracomplexes.
4. The complex according to any of embodiments 1 to 3, wherein the marker is a PET isotope.
5. The complex according to any of embodiments 1 to 4, wherein the PET isotope is selected from the group consisting of isotopes of Aluminium, Barium, Calcium, Cesium, Copper, Dysprosium, Fluorine, Gadolinium, Iodine, Iron oxide, Lithium, Magnesium, Manganese, Oxygen, Platinum, Sodium, Strontium, Tungsten, Technetium and Uranium.
6. The complex according to any of the preceeding embodiments, wherein said complex comprises one or more biomolecules and one or more markers.
7. The complex according to any of embodiments 1 to 3, wherein the marker is a paramagnetic isotope.
8. The complex according to embodiment 7, wherein the paramagnetic isotope has a magnetic moment in the range of from about 6 to about 9.
9. The complex according to embodiment 7, wherein the paramagnetic isotope has a magnetic moment greater than 7.
10. The complex according to any of embodiments 1 to 9, wherein the biomolecule is selected from the group comprising nucleic acids, polypeptides, including glycosylated and otherwise post-translationally modified polypeptides, polysaccharides and lipids.
11. The complex according to embodiment 10, wherein the biomolecule is modified.
12. The complex according to any of embodiments 1 to 11, wherein the biomolecule is a polypeptide.
13. The complex according to embodiment 12, wherein the polypeptide is a globular polypeptide.
14. The complex according to any of embodiments 1 to 13, wherein the complex is anionic.
15. The complex according to any of embodiments 1 to 13, wherein the complex is cationic.
16. The complex according to embodiments 1 to 13, wherein the complex is suitable for imaging of the kidney.
17. The complex according to embodiment 16, wherein the complex is capable of accumulating in the renal cortex.
18. The complex according to embodiments 16 or 17, wherein the biomolecule is capable of binding to Megalin and/or Cubulin.
19. The complex according to any of embodiments 1 to 18, wherein the biomolecule has a molecular weight of from 1 kDa to 50 kDa.
20. The complex according to any of embodiments 1 to 18, wherein the biomolecule has a molecular weight of from 2 kDa to 45 kDa.
21. The complex according to any of embodiments 1 to 18, wherein the biomolecule has a molecular weight of from 5 kDa to 40 kDa.
22. The complex according to any of embodiments 1 to 21, wherein the biomolecule has an isoelectric point of from 7.5 to 11.5.
23. The complex according to any of embodiments 1 to 21, wherein the biomolecule has an isoelectric point of from 8 to 10.5.
24. The complex according to any of embodiments 1 to 23, wherein the biomolecule comprises or consists of aprotinin, or a fragment or a derivative thereof.
25. The complex according to any of embodiments 1 to 23, wherein the biomolecule comprises or consists of chymotrypsinogen A, or a fragment or a derivative thereof.
26. The complex according to any of embodiments 1 to 23 wherein the biomolecule comprises or consists of lysozyme, or a fragment or a derivative thereof.
27. The complex according to any of embodiments 1 to 23 wherein the biomolecule comprises or consists of ovalbumin, or a fragment or a derivative thereof.
28. The complex according to any of embodiments 1 to 23, wherein the biomolecule comprises or consists of a ribonuclease, or a fragment or a derivative thereof.
29. The complex according any of embodiments 1 to 23 wherein the biomolecule comprises or consists of cytochrome c, or a fragment or a derivative thereof.
30. The complex according to any of embodiments 1 to 23, wherein the biomolecule comprises or consists of cystatin c, or a fragment or a derivative thereof.
31. The complex according to any of embodiments 1 to 23, wherein the biomolecule comprises or consists of angiotensin, or a fragment or a derivative thereof.
32. The complex according to any of embodiments 1 to 23, wherein the complex is suitable for use as a marker of proteinuria.
33. The complex according to any of embodiments 1 to 23, wherein the complex is suitable for use as a marker of kidney tubular function.
34. The complex according to any of embodiments 1 to 23, wherein the complex is suitable for use as a marker of glomerular filtration rate (GFR).
35. A method for generating the complex according to any of embodiments 1 to 34, said method comprising the steps of:
   a) providing a biomolecule,
   b) providing a marker,
   c) optionally providing a linker, and
   d) linking the biomolecule and the marker as provided in a) and b), thereby generating a complex.
36. A method for generating the complex according to any of embodiments 1 to 34, said method comprising the steps of:
   a) providing a biomolecule,
   b) providing a marker,
   c) optionally providing a linker, and
   d) linking the biomolecule to the marker, and
   e) selecting the complex.
37. The method according to embodiments 35 or 36 , wherein the marker is a PET isotope.
38. The method according to embodiments 35 or 36, wherein the marker is paramagnetic.
39. The method of embodiments 35 or 36 further comprising the steps of:
   a) dissolving an amount of the biomolecule in a suitable solvent thereby obtaining a solution of the biomolecule,
   b) dissolving an amount of the linker in a suitable solvent thereby obtaining a solution of the linker,
   c) combining the solutions obtained in a) and b) and optionally adjusting the pH of the mixture of solutions,
40. A composition for use as a contrast agent comprising one or more complexes according to any of embodiments 1 to 34, wherein said one or more complexes can be the same or different.
41. The composition according to embodiment 40, further comprising a carrier.
42. The composition according to embodiments 40 or 41, wherein the composition comprises one complex.
43. The composition according to embodiments 40 or 41, wherein the composition comprises at least two different complexes.
44. The composition according to any of embodiments 40 to 43, wherein the biomolecules are Megalin/Cubulin ligands selected from the group consisting of aprotinin, lysozyme, chymotrypsinogen A, ovalbumin, Cystatin C, Cytochrome C or Ribonuclease.
45. The composition according to any of embodiments 40 to 44, wherein the different complexes comprise different markers.
46. The composition according to any of embodiments 40 to 45, wherein at least one of said complexes comprises aprotinin.
47. The composition according to any of embodiments 40 to 46, wherein at least one of said complexes comprises a paramagnetic marker.
48. The composition according to any of embodiments 45 and 47, wherein at least one of said complexes comprise a PET isotope and aprotinin.
49. The composition according to any of embodiments 45 and 48, wherein at least one of said complexes comprise a PET isotope and lysozyme.
50. The composition according to any of embodiments 45 and 49, wherein at least one of said complexes comprise a PET isotope and chymotrypsinogen A.
51. The composition according to any of embodiments 45 and 50, wherein at least one of said complexes comprise a PET isotope and ovalbumin.
52. The composition according to any of embodiments 45 and 51, wherein at least one of said complexes comprise a PET isotope and cystatin C.
53. The composition according to any of embodiments 45 and 52, wherein at least one of said complexes comprise a PET isotope and cytochrome C.
54. The composition according to any of embodiments 40 to 53, wherein at least one of said complexes comprise a PET isotope and ribonuclease.
55. A method for generating an image of a target compartment of an individual, said method comprising the steps of:
   a) administering to the individual a contrast agent comprising the complex according to any of embodiments 1 to 34 and/or the composition according to any of embodiments 40 to 54,
   b) visualising the one or more complexes by employing a positron emission tomography (PET) based method, and
   c) capturing images of said target compartment.
56. The method according to embodiment 55, wherein the visualisation further comprises Computed Tomography (CT), wherein images are acquired sequentially over time during the same scanning session of the individual, and wherein the sequential images are combined into a single superimposed image.
57. The method according to embodiment 55, wherein the visualisation further comprises Magnetic Resonance Imaging (MRI), wherein images are acquired sequentially over time during the same scanning session of the individual, and wherein the sequential images are combined into a single superimposed image.
58. The method of any of embodiments 55 to 57, wherein the target compartment is kidney.
59. The method of any of embodiments 55 to 57, wherein the target compartment is small intestine.
60. The method of any of embodiments 55 to 57, wherein the target compartment is any compartment wherein Megalin and/or Cubulin is localised, such as a target compartment selected from the group consisting of kidney, intestine, epididymis, endometrium, oviduct, lung, thyroid, eye, cerebral ventricles, inner ear, parathyroid, placenta, yolk sac.
61. A method for assessment of a target compartment functionality of an individual, said method comprising the steps of performing the method of any of embodiments 55 to 60, thereby generating one or more images of said compartment, analysing said one or more images, and assessing the functionality of said compartment based on a comparison of the obtained one or more images with a physiological standard image from a healthy normal individual.
62. The method of embodiment 61, wherein the target compartment is kidney.
63. The method of embodiment 62 comprising a further step of calculating the glomerular filtration rate (GFR) of the kidney on the basis of information at least partly obtained from the images.
64. The method of embodiment 63, wherein the calculated GFR corresponds to the total GFR.
65. The method of embodiment 63, wherein the calculated GFR corresponds to a regional GFR.
66. The method of any of embodiments 55 to 65, wherein one or more images of at least two different complexes are assessed, wherein said at least two complexes are administered simultaneously or sequentially in any order.
67. The method of embodiment 61, wherein the target compartment is small intestine.
68. A method for the assessment of target compartment functionality, said method comprising the steps of performing the method of embodiment 61 more than once using different complexes each comprising a marker and a biomolecule, wherein the different complexes comprises different markers and/or different biomolecules, and profiling the target compartment function by performing, for each different complex, a comparison of the obtained one or more images for that complex with a physiological standard image from a healthy normal individual.
69. The method of embodiment 68, wherein the target compartment is kidney.
70. The method of embodiment 68, wherein the target compartment is small intestine.
71. A method for the assessment of kidney function in an individual comprising the steps of:
   a) administering to an individual a complex or a composition comprising a Megalin and/or Cubulin ligand linked to a marker,
   b) visualising the complex or the composition administered in step a) by the method of embodiment 55 and capturing one or more images of the kidney of said individual,
   c) administering to the same individual a complex or a composition comprising a Megalin and/or Cubulin ligand different from the one used in step a) linked to a marker,
   d) visualising the complex or composition administered in step c) by the method of embodiment 55 and capturing one or more images of the kidney of said individual, and
   e) profiling kidney function in said individual based on a comparison of the obtained one or more images for each complex administered with a physiological standard image from a healthy normal individual for said one or more complexes.
72. The method of any of embodiments 55 to 71, wherein the complex or the composition is administered intravenously to said individual.
73. The method of any of embodiments 55 to 71, wherein the complex or the composition is administered perorally to said individual.
74. The method of any of embodiments 55 to 73, wherein said individual is a human being.
75. The method of any of embodiments 55 to 73, wherein said individual is an animal, such as a pet or a laboratory animal.
76. A kit of parts comprising one or more complexes or one or more compositions according to the present invention and instructions for use thereof in a method of the present invention.

## Claims

1. A biomolecule complex for use as a contrast agent in positron emission tomography (PET)-based imaging methods, said complex comprising a biomolecule and a marker, wherein the biomolecule comprises or consists of cystatin c, or a fragment or a derivative thereof and, optionally, wherein the biomolecule is coupled to the marker via linker.

2. The complex according to claim 1, wherein the marker is a PET isotope, in particular, Aluminium, Barium, Calcium, Cesium, Copper, Dysprosium, Fluorine, Gadolinium, Iodine, Iron oxide, Lithium, Magnesium, Manganese, Oxygen, Platinum, Sodium, Strontium, Tungsten, Technetium and Uranium.

3. The complex according to claim 1 or 2, wherein said complex comprises one or more biomolecules and one or more markers.

4. The complex according to any one of claims 1 to 3, wherein one marker is a paramagnetic isotope, preferably, wherein the paramagnetic isotope has a magnetic moment in the range of from 6 to 9, like, wherein the paramagnetic isotope has a magnetic moment greater than 7.

5. The complex according to any one of the preceding claims, wherein the complex is suitable for imaging of the kidney, preferably, wherein the complex is capable of accumulating in the renal cortex.

6. The complex according to any one of the preceding claims wherein the biomolecule has a molecular weight of from 1 kDa to 50 kDa, preferably, wherein the biomolecule has a molecular weight of from 2 kDa to 45 kDa in particular, wherein the biomolecule has a molecular weight of from 5 kDa to 40 kDa.

7. The complex according to any one of the preceding claims, wherein the biomolecule has an isoelectric point of from 7.5 to 11.5, preferably, wherein the biomolecule has an isoelectric point of from 8 to 10.5.

8. The complex according to any one of the preceding claims for use as a marker of proteinuria and/or for use as a marker of kidney tubular function, and/or for use as a marker of glomerular filtration rate (GFR).

9. A composition for use as a contrast agent comprising one or more complexes according to any one of claims 1 to 8, wherein said one or more complexes can be the same or different, optionally, further comprising a carrier.

10. The composition according to claim 9, wherein the different complexes comprise different markers wherein at least one of said complexes comprises a PET isotope and cystatin C.

11. A method for generating an image of a target compartment of an individual, said method comprising the steps of
- visualizing one or more complexes according to any one of claims 1 to 8 and/or the composition according to any one of claims 9 or 10 being administered to the individual as a contrast agent, by employing a positron emission to tomography based method and
- capturing images of said target compartment.

12. The method according to claim 11, wherein the visualisation further comprises Computed Tomography (CT), wherein images are acquired sequentially over the time during the same scanning session of the individual, and wherein the sequential images are combined into a single superimposed image and/or wherein the visualisation further comprises Magnetic Resonance Imaging (MRI), wherein images are acquired sequentially over time during the same scanning session of the individual, and wherein the sequential images are combined into a single superimposed image.

13. A method for assessment of a target compartment functionality of an individual, said method comprising the steps of claims 11 or 12, thereby generating one or more images of said compartment, analysing said one or more images, and assessing the functionality of said compartment based on a comparison of the obtained one or more images with a physiological standard image from a healthy normal individual.

14. A method for the assessment of target compartment functionality according to claim 13, whereby said method comprises the steps of performing the method according to claim 13 more than once using different complexes each comprising a marker and a biomolecule, wherein the different complexes comprises different markers and/or different biomolecules, and profiling the target compartment function by performing, for each different complex, a comparison of the obtained one or more images for that complex with a physiological standard image from a healthy normal individual.

15. The method of any one of claims 12 to 14, wherein the target compartment is a kidney, preferably, said method comprising a further step of calculating the glomerular filtration rate (GFR) of the kidney on the basis of information at least partly obtained from the images, in particular, wherein the calculated GFR corresponds to the total GFR or wherein the calculated GFR corresponds to a regional GFR.

16. A kit of parts comprising one or more complexes or one or more compositions according to any one of claims 1 to 10 and instructions for use thereof in a method of the present invention.
